# EUROPEAN PATENT APPLICATION

(11) **EP 3 257 861 A1**
(43) Date of publication of application: **20.12.2017**
(21) Application number: 16174174.9
(22) Date of filing: 13.06.2016
(51) Int. Cl.: C07K 14/37, C07K 14/39, C12N 1/14

(54) **FUNGAL STRAIN OF THE PHYLUM ASCOMYCOTA**

(71) Applicant: AIT Austrian Institute of Technology GmbH, 1220 Wien (AT)
(72) Inventor: SCHMOLL, Monika, 2100 Korneuburg (AT); STAPPLER, Eva, 1130 Wien (AT)
(74) Representative: Sonn & Partner Patentanwälte

(57) **Abstract**

Disclosed is a fungal strain of the phylum Ascomycota, preferably *Trichoderma, Penicillium, Neurospora* or *Aspergillus,* wherein one or more of the LCS genes are either
(a) overexpressed; or
(b) underexpressed or in which
(c) the function of the encoded protein is altered or abolished by genetic manipulation
in said fungal strain, compared to an unmodified wildtype fungal strain of the same genus cultured under controlled light conditions.

## Description

The present invention relates to fungal strains of the phylum Ascomycota, preferably Trichoderma, *Neurospora* or *Aspergillus.*

Fungi are increasingly used in biotechnological applications to produce plant cell wall degrading enzymes, predominantly cellulases, for the production of biofuels (Kumar *et al*.2008), or heterologous proteins such as industrially used enzymes.

By use of cellulases for the degradation of agricultural waste material, a competition between land use for production of food or for fuel can be avoided and former waste material can be used for sustainable fuels for transportation. Moreover, enzymes are becoming more and more important as efficient catalysts for energy efficient production of industrial chemicals, which often also has the advantage of stereoselectivity and thus abolishes the need for costly and sometimes very inefficient separation steps for isomers. Strain improvement of fungi - especially with respect to the high performance of their cellulase promotors and expression of cellulase genes contributes to economically feasible production of enzymes for diverse applications from bioethanol production to chemical synthesis to production of food additives (such as pectinases for clearing of fruit juice or enzyme mixtures for increased shelf life of bread).

For the application of enzymes produced by fungi in food and feed production, potential secretion of mycotoxins and other secondary metabolites with biological activity is an important issue. Secondary metabolites represent organic substances produced by fungi as by-products of their metabolism, but often also as "chemical warfare" to defend their territory in times of limited nutrients being available, and include toxic, antimycotic, insecticidal, antibiotic substances.

It has been a long desired goal in the art to effectively control the secretion of secondary metabolites in fungi. Secondary metabolite production is desired to be avoided in times of homologous and heterologous protein expression by the fungi, in order to avoid a possible contamination and also in order to focus the expression performance on the expression of the proteins of interest. In times, when a specific secondary metabolite is desired to be extracted, the expression of secondary metabolites can however also be desired.

It is an object of the present invention to provide improved means for biotechnological usage of fungi, especially for providing genetically manipulated fungal strains with improved properties concerning secondary metabolites and processes wherein such improved fungal strains are used, especially on an industrial scale.

A specific object of the present invention is to provide a fungal strain, especially a *T. reesei* strain, that is capable of being cultivated without or with strongly diminished secretion of harmful substances such as Alamethicin, Orsellinic acid, Trichodimerol, Dihydrotrichotetronine, Paracelsin A or Paracelsin B or other problematic secondary metabolites, and to perform cultivation of such fungal strains, especially *T. reesei,* in the absence of such secondary metabolites so as to obtain toxin-free cultivation products and material.

Another specific object of the invention is to provide a fungal strain, especially a *T. reesei* strain, that is capable of producing increased amounts of cellulase or other enzymes or providing a fungal strain in which production of heterologous proteins is possible without the interference of other cellulolytic enzymes and toxic metabolites.

Therefore, the present invention provides fungal strains of the phylum Ascomycota, preferably *Trichoderma, Neurospora, Penicillium* or *Aspergillus,* wherein one or more of the lcs genes or is either
(a) overexpressed; or
(b) underexpressed or in which
(c) the function of the encoded protein is altered
or abolished by genetic manipulation in said fungal strain, compared to an unmodified wildtype fungal strain of the same genus cultured under controlled light conditions, wherein the lcs genes are defined by the group of genes encoding LCS1, LCS2, LCS3, LCS4, LCS5, LCS6, LCS7, LCS8, LCS9, and LCS10.

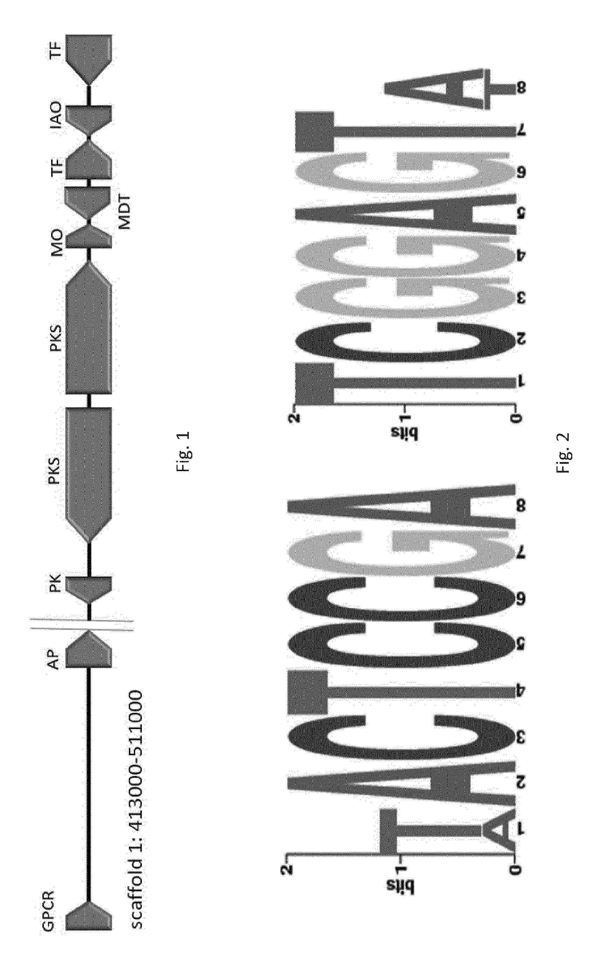

The production of secondary metabolites and of cellulolytic enzymes in the fungal strains according to the present invention is controllable through genetic manipulation of genes (Table 1, Figure 1) residing in vicinity in the genome and being regulated by light upon growth on cellulose (lcs genes for light and cellulose regulated factor associated with secondary metabolism) on scaffold 1: 413000-513000 (JGI genome database, http://genome.jgi.doe.gov/Trire2/Trire2.home.html, 31 May 2016), particularly the expression of the G-protein coupled receptor LCS1 and the transcription factor protein LCS8.

**Table 1: Designations and genomic localization of genes of interest according to the published genome sequence at the Joint Genome Institute JGI in May 2016 (http://genome.jgi.doe.gov/Trire2/Trire2.home.html) lcsx: light and cellulose regulated factor associated with secondary metabolism**

| | | | | | | |
|---|---|---|---|---|---|---|
| lcs1 | TRIRE2_53238 | 1 | 413309 | 413384 | G-protein coupled receptor | EGR52173 |
| lcs2 | TRIRE2_73604 | 1 | 431275 | 431838 | putative histidine acid phosphatase-like protein | EGR52681 |
| lcs3 | TRIRE2_53776 | 1 | 478516 | 479862 | putative kinase | EGR52688 |
| lcs4 | TRIRE2_73618 | 1 | 482236 | 483096 | candidate polyketide synthase | EGR52690 |
| lcs5 | TRIRE2_73621 | 1 | 491045 | 491683 | candidate polyketide synthase | EGR52182 |
| lcs6 | THIRE2_73623 | 1 | 499906 | 500205 | putative monooxygenase | EGR52183 |
| lcs7 | TRIRE2_43701 | 1 | 501820 | 502664 | putative MFS transporter | EGR52691 |
| lcs8 | TRIRE2_102497 | 1 | 504139 | 504304 | transcription factor | EGR52184 |
| lcs9 | TRIRE2_73631 | 1 | 506653 | 506941 | putative iso amyl alcohol oxidase | EGR52692 |
| lcs10 | TRIRE2_102499 | 1 | 510413 | 510424 | transcription factor | EGR52693 |

In general, functions in regulation of cellulase gene expression and/or secondary metabolite production were observed for all LCS genes analyzed and in most cases their function differs between growth in light and darkness on cellulose.

### LCS1

Although deletion of lcs1 did not have a major influence on biomass production upon growth in the presence of cellulose (25-35% decrease) in light or darkness, transcription and expression of cellulases strongly decreased in these mutants (more than 10 fold) in darkness, but not in light. Hence, LCS1 has a dark-specific positive effect on cellulase production.

With respect to regulation of secondary metabolism, deletion of lcs1 strongly decreases production of all secondary metabolites detected in the cultures in the wild-type (Trichodimerol, Dihydrotrichotetronine, Alamethicin, Orsellinic acid and Paracelsin A and B) in darkness. Except for Trichodimerol which was decreased by more than 60 %, abundance of all other metabolites was decreased even by 97 % or below detection limits. It is therefore evident that also other not yet described secondary metabolites of T. reesei will be considerable decreased in an LCS1 mutant or totally abolished in darkness.

In light Orsellinic acid was not detected, neither in the wild-type nor in any mutant analyzed.

For Trichodimerol and Dihydrotrichotetronine, decreased production was observed in light (by 25-40 %), but Alamethicin was even increased (by 75%) in the mutant strain.

In summary, these data obtained in the course of the present invention show that LCS1 is a darkness specific positive regulator of secondary metabolite production and enzyme expression.

### LCS2

LCS2 positively influences growth on cellulose in light (40 % decrease of biomass in the mutant), but has a negative effect on cellulase production under these conditions. In the deletion mutants specific cellulase activity increased by roughly 40 %. In darkness however, no influence on biomass formation was detected, but specific cellulase activity decreased by more than 40 % in the mutants and transcript abundance even by more than 70 %. These data show that LCS 2 is a light dependent regulator of cellulase gene expression.

Lack of LCS2 causes decreased abundance of Trichodimerol and Dihydrotrichotetronine (by 80-90%) in darkness, but only has small effects on the other secondary metabolites under this condition. In light absence of LCS2 in contrast increases levels of Trichodimerol, Dihydrotrichotetronine and Alamethicin 1.5-2.3fold.

In summary, these data obtained in the course of the present invention show that LCS2 is a light dependent regulator of cellulase and secondary metabolite production.

### LCS3

LCS3 is a negative regulator of cbh1 transcription (4fold increase in mutant) in darkness and lack of LCS3 causes considerably decreased biomass formation on cellulose and correspondingly lower cellulase activity in the supernatant.

LCS3 is required for high level production of Dihydrotrichotetronine, Alamethicin and Paracelsin A and B, with 60-90% decrease in the mutant strains.

In summary, these data obtained in the course of the present invention show that LCS3 is a regulator of cellulase and secondary metabolite production.

### LCS4

For deletion of LCS4 only small changes in cellulase transcription and activity were observed in darkness and light, albeit in light a decrease in biomass formation by roughly 30 % occurred upon growth on cellulose. Therefore, there is currently no basis for postulating a function of this polyketide synthetase in cellulase regulation.

Upon deletion of LCS4, production of Trichodimerol and Dihydrotrichotetronine are abolished in darkness, while no major changes in abundance were observed for the other secondary metabolites in darkness.

In light, abundance of Trichodimerol is decreased by more than 30 %, and Dihydrotrichotetronine is abolished, while abundance of Alamethicin and Paracelsin B are even 4fold increased.

Consequently, LCS4 is essential for production of Dihydrotrichotetronine, but not Trichodimerol (which is still produced in light in the mutant). Additionally, the altered secondary metabolite composition caused by deletion of LCS4 obviously results in increased production of other metabolites.

### LCS5

Deletion of LCS5 also caused only small changes in cellulase transcription and activity as well as biomass formation in light. In darkness, however, an increase by more than 50 % was observed. Hence a feedback of the altered secondary metabolite production as caused by deletion of LCS5 on enzyme expression in darkness cannot be excluded.

LCS5 is essential for production of Trichodimerol and Dihydrotrichotetronine in light and darkness. Alamethicin, Orsellinic acid, Paracelsin A and B are somewhat increased in an LCS5 mutant in darkness (20-60%). In light Paracelsin B is decreased by 70 % and Alamethicin by 30 % in contrast to darkness in the mutant.

These data obtained in the course of the present invention show that LCS 5 is involved in biosynthesis of Trichodimerol and Dihydrotrichotetronine and causes altered regulation of other secondary metabolites in light and darkness.

### LCS6

LCS6 was found to be a darkness specific, strongly positive regulator of cellulase transcription and activity specifically in darkness with more than 10fold decreased values in the mutants. In light, only small changes were observed.

LCS6 is essential for production of Trichodimerol and Dihydrotrichotetronine in light and darkness. Additionally, Alamethicin, Orsellinic acid, Paracelsin A and B are strongly decreased or abolished in darkness in the LCS5 mutant as well. In light, production of Alamethicin is roughly 2.4fold increased.

These data obtained in the course of the present invention show that LCS 6 is plays a role in biosynthesis of Trichodimerol and Dihydrotrichotetronine as well as Orsellinic acid and causes altered regulation of other secondary metabolites in light and darkness.

### LCS7

Deletion of LCS7 caused somewhat decreased cellulase activity and transcription values by approximately 20 %, which we do not consider sufficient to assign a function of this transporter in cellulase regulation.

Deletion of LCS7 only caused small effects on secondary metabolite production in darkness with only 10-20 % differences. However, in light, Trichodimerol and Dihydrotrichotetronine were strongly overproduced in this mutant (4-10fold). Consequently, the function of LCS7 negatively influences production of specific secondary metabolites.

### LCS8

Deletion of LCS8 causes considerably decreased biomass formation upon growth in darkness on cellulose (decrease by more than 80 %), but a roughly 2.5fold increase in cellulase transcription and activity. In light only small changes in secreted cellulase activity (decrease by roughly 20 %), but not cbh1 transcript levels were detected. This shows that LCS8 is a darkness specific cellulase regulating transcription factor.

LCS8 is essential for induction of Orsellinic acid production in darkness and is a positive regulator of Trichodimerol, Dihydrotrichotetronine, Paracelsin A and B in darkness. In light deletion of LCS8 causes decrease of Trichodimerol production by 80 % and small increases of Alamethicin and Paracelsin (20-60 %).

In summary, these data obtained in the course of the present invention show that LCS8 is a light dependent regulator of cellulase and secondary metabolite production.

### LCS9

Although cbh1 transcript levels do not change in darkness on cellulose, secreted cellulase activity and biomass formed under these conditions are significantly decreased in the LCS9 mutant with cellulase activity of only about 50 % of the wild-type. In contrast, this mutant strain grows almost 1.5fold better than the wild-type on cellulose in light and also cbh1 transcription in more than 2fold elevated. Nevertheless, secreted cellulase is still 30 % lower than in wild-type.

Hence, LCS9 is involved in light dependent regulation of cellulase gene expression.

LCS9 is essential for production of Trichodimerol and Orsellinic acid in darkness and its deletion causes decreased production of Dihydrotrichotetronine, Alamethicin, Paracelsin A and B (by 30 -60 %). In the mutants, Trichodimerol is decreased in light as well (by 80 %), but Dihydrotrichotetronine is increased by 60 %.

In summary, these data obtained in the course of the present invention show that LCS9 is involved in light dependent regulation of cellulase and secondary metabolite production.

### LCS10

lcs10 is a regulatory target of LCS8 in darkness and not transcribed in light. Therefore LCS10 has a role in mediating the function of LCS8 in cellulase gene expression and secondary metabolite regulation. The genomic position in close vicinity to LCS1-9 of which many have functions in regulation of cellulase gene expression and/or secondary metabolite production strongly indicates a similar function for LCS10 as well. This assumption is further supported by the conserved genomic position in P. *chrysogenum, A. niger* and *Fusarium fujikuroi* (see below).

Thereby the present invention overcomes the limitation of uncontrollable secondary metabolite production by the fungi which exist in the prior art with respect to several metabolites and/or provides increased amounts of cellulase or other enzymes so as to provide a fungal strain in which production of heterologous proteins is possible without the interference of other cellulolytic enzymes and toxic metabolites. From the aforementioned disclosure of the effects of LCS1 to LCS10, it is clear as to how to establish fungal strains manipulated with respect to the expression of these lcs genes under culturing conditions to be applied (darkness/light).

Accordingly, the present invention provides a fungal strain of the phylum Ascomycota, preferably *Trichoderma, Neurospora* or *Aspergillus,* wherein one or more of the LCS genes individually, or in combination is either
(a) overexpressed, especially in the case of LCS1 and LCS6 to improve cellulase gene expression, and LCS1, LCS2, LCS4, LCS5, LCS6, LCS8 and LCS9 to improve Trichodimerol and/or Dihydrotrichotetronin production in the dark, or
(b) *underexpressed,* especially in the case of LCS8 to improve cellulase gene expression and of LCS1, LCS3, LCS6, LCS8, and LCS9 to prevent Paracelsin and/or Alamethicine production in the dark and LCS7 to improve Trichodimerol and Dihydrotrichotetronin production in light,
in said fungal strain, compared to an unmodified wildtype fungal strain of the same genus.

Since most effects are specifically occurring in darkness, it is preferred to perform culturing the present fungal strains in the dark, especially under industrial production conditions (which usually do not apply light). Accordingly, culturing the fungal strains according to the present invention and/or expressing the heterologous protein(s) in these fungal strains are preferably performed in darkness.

Moreover, the present invention provides a fungal strain of the phylum Ascomycota, preferably *Trichoderma, Neurospora* or *Aspergillus,* wherein one of the LCS genes is absent in said fungal strain, compared to an unmodified wildtype fungal strain of the same genus.

Literature search revealed that a deletion mutant of lcs8 had been previously created in *T. reesei* in the background of a *pyr4*-negative derivative of QM9414 (TU-6) lacking the *tku70* (Guangtao *et al.,* 2009) gene as part of a demonstration study for high throughput gene deletion using the non-homologous end joining defect and sexual crossing (Schuster *et al.,* 2012). However, in this study no functional analysis whatsoever was performed and the deletion mutant did not show any growth phenotype. As far as the present invention relates to fungal strains as such, wherein the expression of transcription factor protein LCS8 is absent, it does specifically exclude the *T. reesei* knock-out mutant Δtr_102497 as disclosed in Schuster et al. (Biotechnology for Biofuels, Volume 5(1), 2012, 1-10).

Accordingly, the present invention preferably provides a fungal strain of the phylum Ascomycota, especially *Trichoderma, Neurospora* or *Aspergillus,* wherein one of the LCS genes is de-activated or absent in said fungal strain, compared to an unmodified wildtype fungal strain of the same genus, with the proviso that said fungal strain is not the *T. reesei* knock-out mutant Δtr_102497 as disclosed in Schuster et al. (Biotechnology for Biofuels, Volume 5(1), 2012, 1-10). The Δtr_102497 mutant was obtained by replacing the Zn2Cys6 transcription factor in the *Δtku70 T. reesei* strain with the orotidine-5'-phosphate decarboxylase gene of *T. reesei* (*pyr4,* TR_74020) by using a transformation cassette constructed using the primers 5F_tr_102497 (5'-GTAACGCCAGGGTTTTCCCAGTCACGACGGTAAAGTTCGGACCTTGTAG-3'), 5R_tr_102497 (5'-CGACGATATCAGCTTCCATATTCCGACTAACAGACGAAAGATAAGAAGC-3'), 3F_tr_102497 (5'-AGAAAAGCACAAAGAAGAGGCTCCAACTACTGCGGTTTACATTTCACTC-3') and 3R_tr_102497 (5'-GCGGATAACAATTTCACACAGGAAACAGCTTCTTTCAGGGAGTTCAAGC-3') as described in (Schuster *et al.* 2012).

Ascomycota is a phylum of the kingdom of fungi, which forms together with the basidiomycota the subkingdom dikarya. *Trichoderma, Neurospora* and *Aspergillus* are genera of Ascomycota.

*Trichoderma* is a genus of fungi, which is widely used for industrial production of proteins, especially cellulolytic and hemicellulolytic enzymes. *Trichoderma* is prevalent in a variety of soils and many species of this genus can be characterised as plant symbiotes. The filamentous fungus *Trichoderma reesei* (*T. reesei*), which is the anamorph of *Hypocrea jecorina* has become a model organism for plant cell wall degradation due to its high capacity for cellulose degradation, which is also industrially exploited. Traditionally, this species was also used as a model for studying light responses (Schmoll *et al.,* 2010). Cellulases as well as numerous glycoside hydrolases are regulated in dependence of the light status in *T. reesei.*

*Neurospora* is widely used as a model organism in the field of genetics, as it is very easy to culture. *Neurospora crassa* (*N. crassa*) is an example of a well-known model organism in biotechnology. Due to the versatile tools developed for *Neurospora,* including a close to complete knockout library, this fungus has recently also become a model system for plant cell wall degradation (Znameroski and Glass, 2013). Photoreceptors were shown to regulate cellulase gene expression in *Neurospora crassa* (Schmoll *et al.,* 2012). Although *N. crassa* was long thought not to produce any toxins, genome sequencing revealed that the genomic inventory of this fungus comprises more genes potentially involved in secondary metabolism than expected (Galagan et al., 2003). Nevertheless, secondary metabolism in *N. crassa* is currently hardly studied in detail.

*Aspergillus* is a genus of fungi consisting of a large variety of species. Some species of *Aspergillus* are important commercially for microbiological fermentations or production of enzymes and chemicals such as lysozyme or citric acid, also by heterologous protein production. Additionally Aspergilli are known as producers of one of the most harmful natural chemicals, aflatoxin and many other toxins, but also of pharmaceutically relevant metabolites (Knuf and Nielsen 2012). An important model organism, predominantly for investigation of secondary metabolism and also used in basic genetic research is *Aspergillus nidulans* (*A. nidulans*).

According to the present invention overexpression of a protein is defined as the protein being present in amounts higher than in a comparable standard condition, which is further defined in the respective embodiment of the invention. According to the present invention, overexpression of a protein is defined as more than 130%, more than 140%, more than 150%, preferably more than 160% of the protein level obtained in a standard condition, wherein the level obtained in the standard condition represents 100%. The standard condition is defined as an unmodified wildtype fungal strain of the same genus, which is cultured under commonly used conditions (liquid shake flask culture in a mainly dark rotary shaker). An overexpression of a protein can for instance result from an upregulation of its gene expression, an increased translation of its mRNA or an increased stability of the protein, for example owing to a decrease in protein degradation.

According to the present invention underexpression of a protein is defined as the protein being present in amounts lower than in a comparable standard condition, which is further defined in the respective embodiment, wherein protein levels are however still present in a detectable amount. According to the present invention, underexpression of a protein is defined as less than 70%, less than 60%, less than 50%, preferably less than 40% of the protein level obtained in the standard condition, wherein the level obtained in the standard condition represents 100%. The standard condition is defined as an unmodified wildtype fungal strain of the same genus, which is cultured under standard conditions, usually under controlled light conditions i.e. constant darkness corresponding to an industrially used steel fermentor or constant light. An underexpression of a protein can for instance result from a downregulation of its gene expression, a decreased translation of its mRNA or a decreased stability of the protein, for example owing to an increase in protein degradation.

If a protein cannot be detected with analytical methods of the art, the protein is defined as being absent, according to the present invention. According to the invention, the absence of a protein, can result for instance from removal or silencing of the corresponding gene itself, e.g. by gene knock-out, gene silencing or gene replacement of the respective gene. Additionally, abolishment of gene expression can be achieved by removal of a promotor motif to which transcription factors essential for induction of transcription of the target gene bind.

According to the invention, genetic manipulation is defined as the direct manipulation of an organism's genome using methods of biotechnology. According to the present invention, such genetic manipulation methods can be used to obtain overexpression, underexpression, de-activation or absence of a gene of interest and thereby of its respective protein. Non-exclusive examples for such methods are molecular cloning, introduction of DNA via vectors, gene targeting, gene silencing, nuclease-based methods, transformation, genome editing, gene knock-out, transcription enhancement or repression etc.; also application of the Crispr/cas technology is possible (Liu et al., 2015).

Using genetic manipulation techniques, one can for instance introduce new DNA sequences into the genome of an organism, edit or remove existing sequences. Organisms where the genome has been changed through genetic manipulation are termed genetically modified organisms.

Specifically, in one embodiment of the invention, underexpression or overexpression of proteins can be achieved by linking the desired gene operably to a recombinant promoter by means of genetic manipulation techniques, which is capable to induce the decreased or increased expression of the protein, respectively, compared to the unmodified wildtype promoter. The conditions under which the protein is underexpressed or overexpressed, respectively, depend on the gene promoter used. If the promoter is an inducible promoter, respective conditions or substances have to be applied in order to switch on or to regulate the promoter activity. The expression of a promoter being "operably linked" to a protein encoding DNA sequence means that the promoter directly influences the expression of the protein encoding DNA sequence. In another preferred embodiment of the present invention the fungal strain comprises an expression vector carrying the gene to be overexpressed operably linked to a promoter.

Suitable and therefore preferred promoters used for upregulation of gene expression are according to the invention: *cbh1, gpdl, tefl, hfb2* as well as other promotors enhancing the level of gene transcription (for an overview see Schmoll and Dattenböck (eds) 2016 Gene expression systems in fungi, particularly Paloheimo et al., 2016 chapter 2 in this book).

According to the present invention underexpression of a protein of interest is preferably achieved for instance by genetic manipulation of the fungal strains to synthesise antisense RNA to the mRNA of the corresponding gene of interest. The synthesis of antisense RNA may be coupled to an inducible promoter, such as the *xylP* promoter in Penicillium (Zadra *et al.,* 2000) or the *gpd* promotor in (Schuster et al., 2011). Additionally, underexpression can be achieved by promotor exchange for a lowly expressed promotor under specifically defined conditions. Additionally, decreased gene expression can be achieved by removal of a promotor motif to which transcription factors essential for induction of transcription of the target gene bind.

Deletion of target genes, i.e. gene knock-out to obtain recombinant strains can be performed using different marker cassettes including but not limited to the hygromycin B marker cassette, the *amdS* marker cassette, benomyl resistence, the phosphinothricin resistance marker, the nptII marker cassette or the kanamycin resistance cassette (for an overview see van den Berg and Maruthachalam 2015 (eds) Genetic transformation systems in fungi).

Also a knock-out can be performed in an auxotrophic fungal strain, to which the ability to grow on normal growth medium, sufficient for prototrophs, is conferred by the addition of the marker restoring its prototrophy, e.g. introduction of orotidine-5'-phosphate decarboxylase in an uracil auxotrophic strain.

According to the invention, transcription factor proteins are defined as proteins binding specific DNA sequences and thereby controlling the transcription of those specific genes to mRNA. Transcription factor proteins can either lead to a repression of transcription or an activation of transcription by promoting or blocking the recruitment of RNA polymerases to the DNA.

According to the invention, secondary metabolites are defined as compounds which are not directly essential for growth or survival of the organism, yet these secondary metabolites have important roles in signalling, development and interaction with other organisms (Mukherjee et al., 2012). With regard to fungi, secondary metabolites are preferentially produced upon growth under unfavourable conditions, after the active growth phase, if nutrients in the environment become limiting or if environmental conditions such as humidity, temperature, UV irradiation or pH threaten the functionality of the fungal strain (Yu & Keller, 2005). It was shown that production of secondary metabolites as well as the light dependence of this process is strongly dependent on the carbon source. Even the concentration of the carbon source in the cultivation medium can switch the preference for secondary metabolite production from light to darkness in *Aspergillus nidulans.* This process is regulated by VeA and its associated proteins including the photoreceptors LreA and LreB as well as the phytochrome FphA ((Atoui *et al.,* 2010) and references cited therein).

In fungi, biosynthesis of secondary metabolites is organized via regulation of specific gene clusters (Keller *et al.,* 2005). A gene cluster is defined as a set of two or more genes, which encode for the same or similar products. Many of the gene clusters implicated in the synthesis of secondary metabolites in fungi, which became obvious during analysis of fungal genomes, appear to be silent under common laboratory conditions. Therefore, recent research efforts concentrate on elucidation of regulation of secondary metabolite clusters and investigation of environmental signals initiating activation of such silent clusters and "cryptic" pathways connected to them (Brakhage & Schroeckh, 2011). In this respect, especially overexpression of a putative regulator of a predicted but silent cluster proved effective (Bergmann *et al.,* 2007). These efforts also indicate a regulatory crosstalk between different secondary metabolite pathways, due to activation of more than one cluster upon overexpression of a regulator (Gacek & Strauss, 2012).

Often secondary metabolites are used by fungi for defence of their territory. They range from potential antibiotics to mycotoxins as well as volatile organic compounds. In *T. reesei* secondary metabolites have been shown to have a function in chemical communication upon onset of sexual development and they are subject to regulation by VEL1 (Bazafkan et al., 2015). For the biotechnological workhorse *Trichoderma reesei,* so far only the trichothecene toxin trichodermin has been described, which is much less toxic than most other metabolites of this group of toxins, though (Blumenthal, 2004) as well as paracelsin (Bruckner *et al.,* 1984). However, the genome of *T. reesei* contains 11 polyketide synthases (Baker *et al.,* 2012) and several non-ribosomal peptide synthetases (Martinez *et al.,* 2008), which are important for the production of secondary metabolites in many fungi. Although this number is small compared to other fungi (Baker *et al.,* 2012), their presence indicates a considerable potential for production of secondary metabolites. Interestingly, analysis of the genome also revealed that plant cell wall degrading enzymes of *T. reesei* are often found in clusters along with genes involved in secondary metabolism (Martinez *et al.,* 2008). Consequently, it is reasonable to assume that *T. reesei* evolved a mechanism for balancing the operation of primary and secondary metabolism during its life cycle. In general, according to the invention, non-exclusive examples of secondary metabolites produced by fungi are peptaibols, polyketides, siderophores, terpenoids/steroids, pyrones and non-ribosomal peptides. Non-exclusive examples are substances like trichodermin, trichodimerol, dihydrotrichotetronin, paracelsin A, paracelsin B, alamethicin, brevinamid F, rugulusovine, orsellinic acid, gliotoxin or gliovirin.

### Alamethicin

Alamethicin is an amphiphil peptide antibiotic bacteriostatic, fungicidal, cytostatic and hemolytic functions, although also resistence has been reported (summarized in (Kredics *et al.,* 2013)). As a monovalent cation ionophore, it can initiate a fluctuating, voltage dependent ion flux in lipid membranes and forms pores by aggregation.

Interestingly, so far all *Trichoderma* species producing alamethicin were found to belong the the so-called "Brevicompactum clade" of the genus (Kredics *et al.,* 2013), which does not include *Trichoderma reesei.*

Alamethicin also has broad effects on plants from alteration of metabolite production and emission of volatile compounds to rapid death in case of *Arabidopsis thaliana,* which is associated with a dramatic loss of intact RNA and the inhibition of protein synthesis (Rippa *et al.,* 2007). Interestingly, added *Trichoderma* cellulase induced cellular resistence to alamethicin in plants, which appeared to be correlated with changes in the plasma membrane lipid composition (Aidemark *et al.,* 2010).

In mammals, alamethicin shows oral toxicity in high concentrations, cytolytic activity towards specific tumor cells, peritoneal mast cells and lymphocytes. Moreover, hemolytic activity was observed towards human erythrocytes (Leitgeb *et al.,* 2007). Alamethicin can be used to permeabilize membranes of different mammalian cell types. In recent years, alamethicin was used as a reference compound in analysis of biological activity of newly described peptaibols towards mammalian cells and in studies aimed at investigation of mitochondrial function.

It is specifically desirable to provide cultivation methods for fungal strains and specifically modified strains, especially for *T. reesei,* wherein no Alamethicin is produced so that resulting material is absolutely Alamethicin-free. This is specifically needed for cultivation methods that should lead to products that are to be applied to humans (or animals). With Alamethicin-free cultivation methods, the risk for Alamethicin-contamination can be completely excluded.

### Paracelsine

Paracelsins are toxic peptibols found in many *Trichoderma* spp (Solfrizzo *et al.,* 1994), including *T. reesei* (Bruckner et *al.,* 1984).

### Rugulosuvine

Rugulosuvines are bioactive diketopiperazine alkaloides isolated from Penicillium spp (Kozlovskii *et al.,* 2001).

### Orsellinic acid

Orsellinic acid is produced as secondary metabolite by *Aspergillus nidulans* and the cluster responsible for its production has been characterized (Sanchez *et al.,* 2010). This cluster contains a polyketide synthetase which also has a homologue in *T. reesei,* that was shown to be involved in conidial pigmentation, formation of teleomorph structures and antagonistic abilities of *T. reesei* against other fungi (Atanasova *et al.,* 2013), but is not related to the LCS genes.
Orsellinic acid as a chemical is considered harmful, toxic on inhalation and irritating to the eyes.

### Brevianamid F

Brevianamides represent indole alkaloides produced by *Penicillium* and *Aspergillus* species. They cause an inflammatory response in lungs and were shown to have insecticidal, but no bactericidal or fungicidal activity.

### Trichodimerol and Dihydrotrichotetronine

These two metabolites belong to the group of Bisorbicillinoids (Siddiquee 2014 Biology and Biotechnology of Trichoderma 139-175). For Trichodimerol an application in cancer treatment has been suggested (Serwe et al., 2009). Trichodimerol and Dihydrotrichotetronine are reported to be derived from the same precursor molecule (Shirota et al., 1997).

Growth in constant light according to the present invention is defined as growth on Mandels Adreotti Minimal Medium with microcystalline cellulose (1% w/v) as carbon source for 72 hours in constant light (1800 lux) in shake flask cultures. Growth in constant darkness according to the present invention is defined as growth on Mandels Adreotti Minimal Medium with microcystalline cellulose (1% w/v) as sole carbon source for 72 hours in constant darkness in shake flask cultures.

Since the expression of a variety of proteins in fungi is modified in dependence of light (e.g. cellulases, glycoside hydrolases, etc.), crosstalk of gene regulation in response to light and nutrient signal transduction with plant cell wall degradation as output pathway has gained increased attention in recent years (Schmoll *et al.,* 2010).

Changing light conditions, caused by the rotation of earth resulting in day and night or growth on the surface or within a substrate, result in considerably altered physiological processes in fungi (Schmoll, 2011). Harmful UV-radiation, altered temperature, oxygen abundance and levels of reactive oxygen species or humidity necessitate adaptive measures to adjust to day and night (Rodriguez-Romero *et al.,* 2010). Virtually every metabolic process of fungi is regulated by light to some extent (Tisch & Schmoll, 2010) and the influence of light on the growth rate of fungi is dependent on the quality of the substrate they are growing on (Charlile, 1965). In recent years, considerable progress was made in understanding the interplay between light response, nutrient signaling and sexual development in *T. reesei.* Light dependent processes in *T. reesei* include development, cellulase gene expression and expression of glycoside hydrolases in general, carbon utilization and sulphur metabolism. Regulation of one or more of these processes in response to light in *T. reesei* is achieved by the photoreceptors BLR1, BLR2 and by ENV1, the heterotrimeric G-protein pathway, the cAMP pathway and presumably by the sulphur controller LIM1. These studies indicate a strong regulatory interrelationship between nutrient signaling and light response, in which ENV1 plays a major role in connecting the two signaling cascades (Schmoll, 2013, Schmoll et al., 2014).

In one embodiment, this invention provides fungi, which express decreased amounts of so called secondary metabolites. Often the production of secondary metabolites is unfavourable to culture conditions or the industrial production of substances by the fungi, so that it is a long desired goal to generate fungal strains, where the production of said secondary metabolites is decreased when compared to a wild type fungal strain.

In another embodiment, this invention provides fungi, which express decreased amounts of secondary metabolites and cellulases. Often for the production of a heterologous protein a background of production of native enzymes in the fungus is interfering with analysis, downstream processing or product development and hence it is not desired. "Decreased amounts" mean significantly reduced amounts of specific secondary metabolites which are directly or indirectly affected by over- or underexpression by one or more of the lcs genes according to the present invention. For example, specific secondary metabolites are reduced by 50% or more (%w/w), preferably by 80% or more (%w/w), more preferred by 90% or more (%w/w), especially by 95% or more (%w/w) or 99 % or more (% w/v) or to levels lower than the detection limit of current methods.

The present invention solves this problem by providing a fungal strain of the phylum Ascomycota, preferably *Trichoderma, Neurospora* or *Aspergillus,* wherein one or more of the LCS genes is de-activated or absent, for example by genetic manipulation of said fungal strain, preferably by knock-out of its corresponding gene, in said fungal strain when compared to an unmodified wildtype fungal strain of the same genus cultured in controlled light conditions (constant light or constant darkness), with the proviso that said fungal strain is not the *T. reesei* knock-out mutant Δtr_102497 as disclosed in Schuster et al. (Biotechnology for Biofuels, Volume 5(1), 2012, 1-10). In fungal strains, especially *T. reesei,* where one of the LCS genes expression is completely silenced, i.e. wherein one or more LCS encoded proteins are absent because it is not expressable by the strains, also production of Trichodimerol, Dihydrotrichotetronin, Alamethicin, Paracelsin and Orsellinic acid or other yet to be determined secondary metabolites is diminished or does not take place. Such strains (e.g. *T. reesei lcsl* or *lcs8* knock-out strains) can be cultivated in a safeguarded Alamethicin-free manner with considerably decreased abundance of potentially harmful secondary metabolites. Thus the present invention provides a method for cultivating a fungal strain under toxin reduced conditions. The resulting strain culture and the products derived therefrom show a significantly decreased level of harmful secondary metabolites. Accordingly the present invention also relates to a method for cultivating a fungal strain, especially *T. reesei,* wherein strongly decreased amounts of Trichodimerol, Dihydrotrichotetronine, Alamethicin, Orsellinic acid, Paracelsin A or Paracelsin B are produced or in which these compounds are not produced at all. Furthermore the present invention relates to the use of this method for the production of toxin-reduced cell products, preferably proteins and more preferably heterologous proteins.

Toxin-reduced according to the present invention means that the culture conditions contain maximally 30%, preferably 20% or less, more preferably 10% or less and most preferably 5% or less of the toxin concentration obtained in the culture of unmodified wildtype fungal strains cultured under standard conditions.

Accordingly, the present invention provides cell cultures and cell culturing products which contain - compared to the fungal strain cultured under standard conditions without the lcs modification according to the present invention maximally 30%, preferably 20% or less, more preferably 10% or less and most preferably 5% or less of Trichodimerol; and/or maximally 30%, preferably 20% or less, more preferably 10% or less and most preferably 5% or less of Dihydrotrichotetronine; and/or maximally 30%, preferably 20% or less, more preferably 10% or less and most preferably 5% or less of Alamethicin; and/or maximally 30%, preferably 20% or less, more preferably 10% or less and most preferably 5% or less of Orsellinic acid; and/or maximally 30%, preferably 20% or less, more preferably 10% or less and most preferably 5% or less of Paracelsin A; and/or maximally 30%, preferably 20% or less, more preferably 10% or less and most preferably 5% or less of Paracelsin B.

Also, the present invention relates to the deletion of one or more of the LCS genes in order to achieve a cellulase free background for expression of heterologous proteins.

Moreover, the present invention relates to the use of increased levels of lcs genes for production of potentially beneficial compounds such as Trichodimerol and Dihydrotrichotetronine, for which an application in cancer treatment has been suggested or in order to increase cellulase gene expression.

According to the present invention a strain product is defined as organic molecules produced by the strain and include for example proteins, lipids, carbohydrates, RNA, DNA, etc.

In another aspect, the present invention provides a fungal strain of the phylum Ascomycota, preferably *Trichoderma, Neurospora* or *Aspergillus,* wherein one of the lcs genes is underexpressed in said fungal strain by genetic manipulation, preferably by a downregulation of the corresponding gene, of said genetic strain, when compared to an unmodified wildtype fungal strain of the same genus cultured in a normal light-darkness cycle or under otherwise suitable conditions. The absence or reduction in expression of several lcs genes leads to downregulation of many secondary metabolites, which are under their control. In another embodiment the present invention provides a fungal strain, wherein one of more of the lcs genes are underexpressed in said fungal strain, compared to an unmodified wildtype fungal strain of the same genus cultured in constant light or constant darkness.

In another embodiment, the present invention provides a fungal strain, wherein one or more of the lcs genes are underexpressed in said fungal strain due to alteration or removal of the common promotor motif detected in several of the lcs promotors (Figure 2). Promotors of the lcs genes were investigated for putative common novel DNA binding motifs using MEME and SCOPE. One common motif was found in several lcs promotors (lcs4, lcs5, lcs6, lcs7, lcs8, lcs9) and is designated LCS motif (Figure 2).

In another embodiment, this invention provides a fungal strain of the phylum Ascomycota, preferably *Trichoderma, Neurospora* or *Aspergillus,* wherein one or more of the lcs genes are overexpressed in said fungal strain, compared to an unmodified wildtype fungal strain of the same genus cultured under controlled light conditions. Increased amounts of the respective lcs gene(s) lead to increased levels of secondary metabolites and/or cellulases, which are under their control as outlined above. In one embodiment of the invention the fungal strain can be used to produce said secondary metabolites, which can be for example used in a pharmaceutical, cosmetic or insecticidal formulation. Several secondary metabolites, such as alamethicin or paracelsin A exhibit antibiotic effects (Fringeli & Fringeli, 1979). Other secondary metabolites such as brevianamide have been demonstrated to exhibit insecticidal activity (Paterson *et al.,* 1990).

To this end, the present invention provides a fungal strain of the phylum Ascomycota, preferably *Trichoderma, Neurospora* or *Aspergillus,* in which one or more lcs gene(s) are overexpressed in said fungal strain by genetic manipulation of said fungal strain, when compared to an unmodified wildtype fungal strain of the same genus cultured under standard conditions. Said overexpression of an lcs gene preferentially results from a genetic manipulation and specifically up-regulation of the corresponding gene(s).

Since deletion of specific lcs genes (for example lcs1, lcs2 and lcs6) leads to considerably decreased cellulase activity (as disclosed in the example section), overexpression these genes is expected to lead to increased cellulase activity.

Therefore, the present invention also provides a fungal strain producing increased levels of cellulases.

Accordingly, the present invention provides cell cultures and cell culturing products which contain - compared to the fungal strain cultured under standard conditions without the lcs modification according to the present invention - more than 50%, preferably 70% or more, more preferably 100% or more and most preferably 200 % or more of produced cellulases or heterologous protein produced using a cellulase promotor enhanced by the function of the modified LCS genes.

According to the present invention, cellulases are defined as cellulolytic enzymes, which are known to be co-regulated with hemicellulolytic enzymes, which can be used to break down cellulose and hemicellulose, which are components of the plant cell wall. Cellulose and hemicellulose represent the largest reservoir of renewable carbon sources on earth, which could be used for the production of biofuels and other biorefinery products, thereby replacing products derived from fossile carbon components (Kumar *et al.,* 2008). The application of plant cell wall degrading enzymes for production of second generation biofuels reflects a market worth of an estimated 4 billion EUR. Cellulases produced by the fungus *Trichoderma reesei,* which is used as a production host by the worldwide leading companies in this field, represent a significant share of this enzyme production. The enzymatic break-down of cellulose and hemicellulose is preferred to chemical processes leading to the hydrolysis of said compounds, since these chemical processes result in the formation of inhibitory by-products and are thus less environmentally compatible.

In this aspect of the invention relates to a fungal strain, wherein one or more lcs genes are overexpressed in said fungal strain compared to an unmodified wildtype fungal strain of the same genus cultured under controlled light conditions, and wherein said fungal strain is used for the production of cellulases.

The fungal strains of the invention can be used to increase the production of cellulolytic and hemicellulolytic enzymes compared to their wildtype strains cultured under controlled light conditions. The present invention relates to the use of fungal strain, wherein one or more lcs genes are overexpressed in said fungal strain compared to an unmodified fungal wildtype strain cultured under controlled light conditions, for production of cellulases.

According to another aspect, the present invention relates to the use of a fungal strain, wherein one or more lcs genes are underexpressed in said fungal strain compared to an unmodified wildtype strain cultured under controlled light conditions, for production of heterologous proteins.

According to preferred embodiments of the present invention, the fungal strain according to the present invention may be cultured either in continuous light or in continuous darkness (e.g. without affecting the fungal culture to natural day/night cycles).

In another preferred embodiment, the present invention relates to the use of a fungal strain, one or more lcs genes are absent in said fungal strain compared to an unmodified wildtype strain cultured under controlled light conditions, with the proviso that said fungal strain is not the *T. reesei* knock-out mutant Δtr_102497 as disclosed in Schuster et al. (Biotechnology for Biofuels, Volume 5(1), 2012, 1-10), for the production of heterologous proteins. By the decrease of secondary metabolite production via decrease of expression or absence of *lcs8* in the fungal strain of the invention, the production of heterologous proteins will be enhanced in said fungal strain compared to its wildtype strain cultured under controlled light conditions.

According to the present invention, a heterologous protein is defined as a protein, which is not expressed by a specific wildtype strain of a particular species, but rather derived from a different strain type or from a different species altogether. Generally the production of heterologous proteins in a specific strain type is achieved by introducing its corresponding gene into the DNA of said strain. Several biotechnological methods to introduce a gene into a host strain are known to the skilled artisan, for example via viral vectors, plasmids, expression vectors, liposomes, integration into the DNA etc. Said heterologous proteins can be operably linked to any constitutive or inducible promoter, but according to the invention is preferably operably linked to any of the promoters described above or a cellulase promoter of the fungal strain.

In another embodiment, the present invention relates to the use of LCS1 and its homologues in other human and plant pathogenic fungi is a drug target and for development of novel drugs for fighting fungal diseases. LCS1 represents a G-protein coupled receptor, which if deleted and hence also if switched off chemically, downregulates toxin production. Consequently, if this toxin production can be decreased by treatment of a fungal disease with a specific chemical blocking LCS1 function, harmful effects on the patient could be alleviated or in case of plant pathogens, high toxin values in the crops could be prevented.

More than half of the therapeutic agents currently on the market have GPCRs as targets. These also include a considerable number of the top 100 best-selling drugs. Consequently, assays to screen for GPCR ligands are major subjects of current research (Zhang et al., 2012). GPCRs have a long history of use as drug targets and consequently a large toolset for discovery of agonists and antagonists to activate or inactivate the function of GPCRs is available. Nowadays high throughput screening is the method of choice to identify effective ligands for manipulation of the activity of GPCRs (Jacoby et al., 2006). Consequently, antagonistic ligands can be applied to inhibit the function of the GPCR and hence achieve the same effect as genetic manipulation of the GPCR sequence (such as deletion) would have. Therefore, the GPCR LCS1 can also be a target for drug development in case of human pathogenic fungi or for specific fungicide development in case of plant pathogenic fungi.

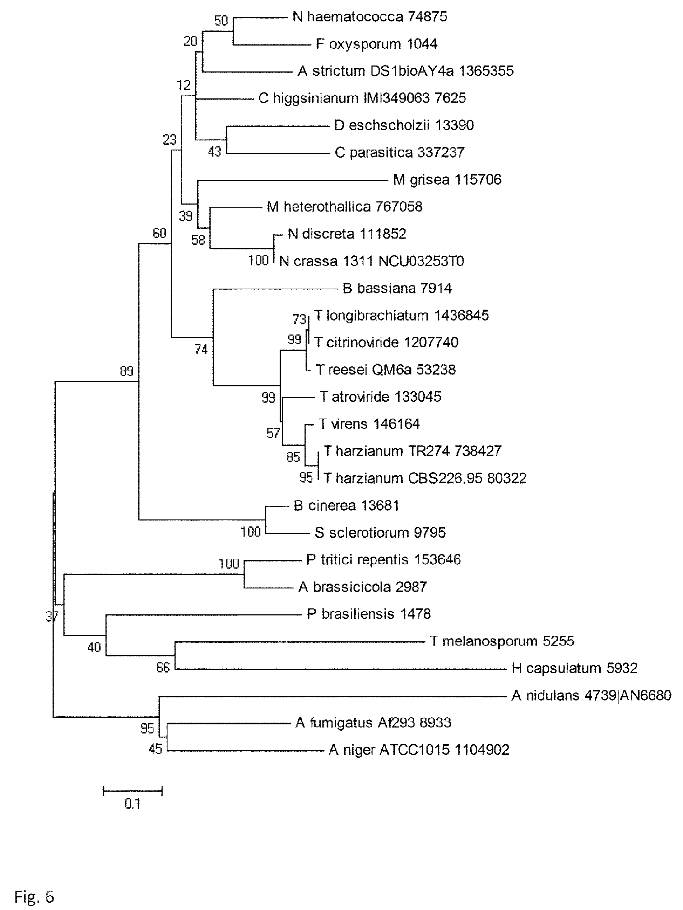

LCS1 belongs to GPCR class VII (secretin-like, related to rat growth hormone releasing factor) and is conserved in all sequenced *Trichoderma* species as well as numerous plant pathogens such as *Fusarium* spp., *Botrytis cinerea, Sclerotinia sclerotiorum* etc. and also human pathogens like *Histoplasma capsulatum, Paracoccidioides brasiliensis* and *Aspergillus fumigatus* (Figure 6).

The present invention is further illustrated by the following examples and the figures, yet without being restricted thereto.
Fig 1 shows distribution of the lcs genes in the genome of *T. reesei* on scaffold 1 (the scheme is drawn to scale) in the JGI genome database.
Fig 2 shows the LCS motif in forward and reverse orientation as determined by MEME and SCOPE.
Fig 3 shows the phylogenetic analysis of genes with sequence similarity to lcs8. Phylogenetic relationships of 74 protein sequences were analysed by MEGA4 using the minimum evolution method. The bootstrap consensus tree inferred from 500 replicates represents the evolutionary history of the proteins analyzed. Sequences were taken from AspGD or JGI, protein IDs or designations are given in the tree. Abbreviations of the species are summarized in Figure 4.
Fig 4 gives the abbreviations used in phylogenetic analysis as given in Figure 3.
Figure 5 shows a TMHMM analysis (online server for analysis: http://www.cbs.dtu.dk/services/TMHMM/) of transmembrane domains revealing intracellular (blue) extracellular (pink) and transmembrane (red) areas of LCS1.
Figure 6. Phylogenetic analysis of LCS1 homologues in ascomycetes including human and plant pathogenic fungi. Sequences were retrieved from the Joint Genome Institute (JGI) databases at http://genome.jgi.doe.gov/programs/fungi/index.jsf on May 23rd 2016. Species names are provided along with protein IDs as listed in JGI databases for *Nectria haematococca, Fusarium oxysporum, Acremonium strictum, Colletotrichum higgsinianum, Daldinia eschscholzii, Cryphonectria parasitica, Magnaporthe grisea, Myceliophtora heterothallica, Neurospora discreta, Neurospora crassa, Beauveria bassiana, Trichoderma longibrachiatum, Trichoderma citrinoviride, Trichoderma reesei, Trichoderma atroviride, Trichoderma virens, Trichoderma harzianum, Botrytis cinerea, Sclerotinia sclerotiorum, Pyrenophora tritici repentis, Alternaria brassicicola, Paracoccidioides brasiliensis, Tuber melanosporum, Histoplasma capsulatum, Asperbillus nidulans, Aspergillus fumigatus* and *Aspergillus niger.* Phylogenetic analysis of protein sequences was performed with MEGA4 after sequence alignment using ClustalX.

### Examples

### Example 1 - Discovery of a light-regulated group of genes potentially involved in secondary metabolite production in Trichoderma reesei

The wildtype fungal strain of *Trichoderma reesei* QM9414 was grown on Mandels Adreotti Minimal Medium with microcystalline cellulose (1% w/v) as sole carbon source for 72 hours in constant light (1800 lux) or constant darkness in shake flask cultures.

It was previously revealed that 2.8% of the genes of *T. reesei* are regulated in response to light upon growth on cellulose (Tisch et al., 2011b). Investigation of the distribution of these genes within the genome revealed non-random distribution of light regulated genes upon growth on cellulose, including one group of these genes on scaffold 1 (Table 1). Interestingly, transcript levels of genes within this group are repressed by BLR1 (photoreceptor-encoding gene) and BLR2 (photoreceptor-encoding gene) in light and up-regulated by ENV1 and GNB1 (Tisch and Schmoll 2013, Tisch et al., 2011b). The genes in this group are called lcs genes (light and cellulose regulated factor associated with secondary metabolism) and include two polyketide synthases, two transcription factors, a kinase, a G-protein coupled receptor, a monooxygenase as well as a putative multidrug transporter (Figure 1, Table 1).

Screening of the promotors of the LCS genes using the online software tools MEME and SCOPE revealed the motif 5' GTCGBNGTA 3' as common to most LCS genes (Figure 2).

Additionally, CRE1-binding sites (5' SYGGRG 3') as well as GATA-binding sites (Scazzocchio, 2000), which might be targeted by the photoreceptors BLR1 or BLR2 comprising GATA-type zinc finger domains (Castellanos *et al.,* 2010) as well as other important GATA factors, are present in the promotors of this gene group (Table 2).

**Table 2. Known DNA motifs found in the promotors of the lcs genes.**

| Gene | SYGGRG CRE1 | LRE (GATNC-CGATN) Less than 100 bp apart | HGATAR | WCC binding motif of Smith 2010 GATCGA |
|---|---|---|---|---|
| 53238 | 7 | 1 | 6 | 1 |
| 73604 | 4 | 2 | 2 | 13 with mismatch |
| 53776 | 3 | no | 1 | 7 with mismatch |
| 73618 | 4 | unclear | 3 | 1 |
| 73621 | 3 | no | 3 | 1 |
| 43701 | 1 | 1 | 2 | 12 with mismatch |
| 102497 | 3 | no | 4 | 1 |
| 73631 | 2 | no | 8 | 1 |
| 73623 | 4 | no | 5 | 5 with mismatch |

The polyketide synthases LCS4 and LCS5 belong to the group of non-reducing fungal PKS clade III and the reducing clade I of the lovastatin/citrinine type, respectively. Both PKSs appear to be specific to *T. reesei* as no orthologues were found in *T. atroviride* or *T. virens* (Baker *et al.,* 2012) although there are orthologous genes in A. *nidulans* and *N. crassa.* Since lovastatin is known to be produced by an iterative type I polyketide synthase and a partner enoylreductase (Campbell & Vederas, 2010) - representing the two types of enzymes - it is reasonable to assume that *T. reesei* might be able to produce a chemical of considerable commercial value related to lovastatin.

### Example 2 - Proteins related to the transcription factor LCS8 in other fungi

In order to evaluate whether LCS8 might have functional orthologues in other fungi phylogenetic analysis with proteins from numerous other fungi was performed, which showed sequence similarity. It was found that LCS8 clusters most closely with proteins from *Aspergillus fumigatus, Aspergillus nidulans* and *Penicillium* spp., including *Penicillium chrysogenum.* While the LCS8 orthologue of *Trichoderma longibrachiatum* clusters with LCS8, those of *Trichoderma atroviride* and *Trichoderma virens* are found in a sister clade that also comprises proteins of *Oidiodendron majus, Anthostoma avocetta, Chaetomium globosum* and *Thielavia terrestris.* The multiple *A. fumigatus* sequences appearing in these two clades indicate an increased relevance of the function of the LCS8 homologue for this fungus. Therefore, it is evident that LCS8 is an interesting target for drug development to fight *A. fumigatus* mykoses.

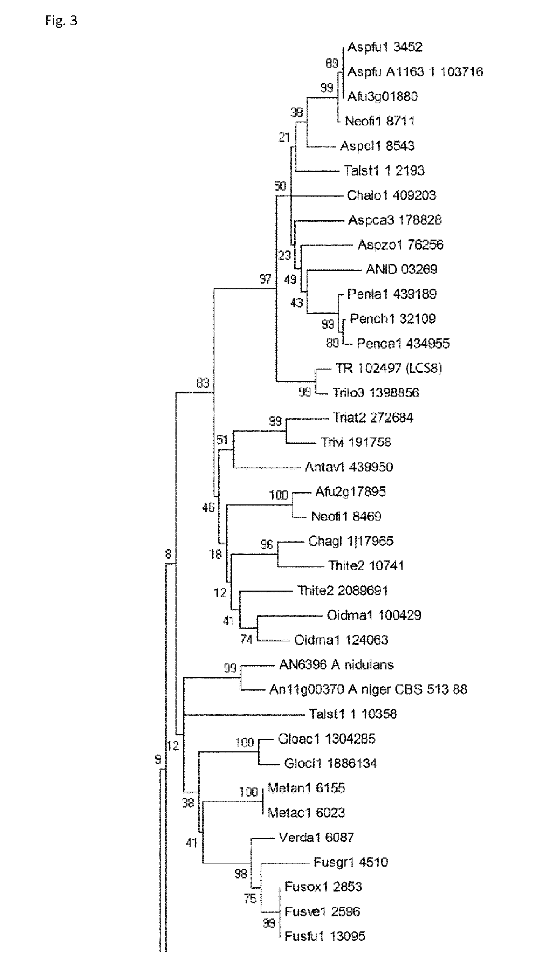

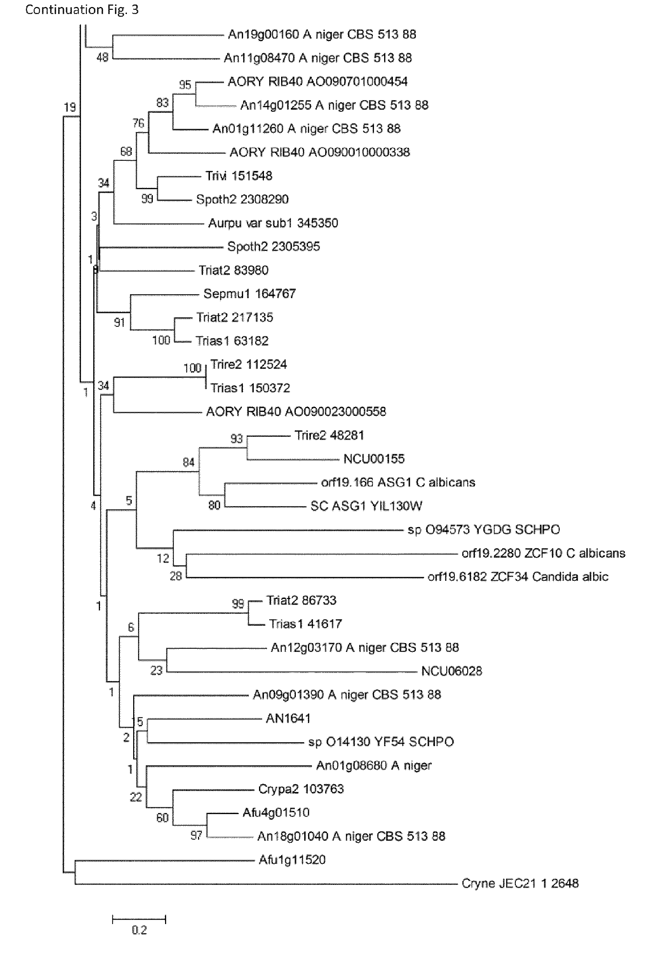

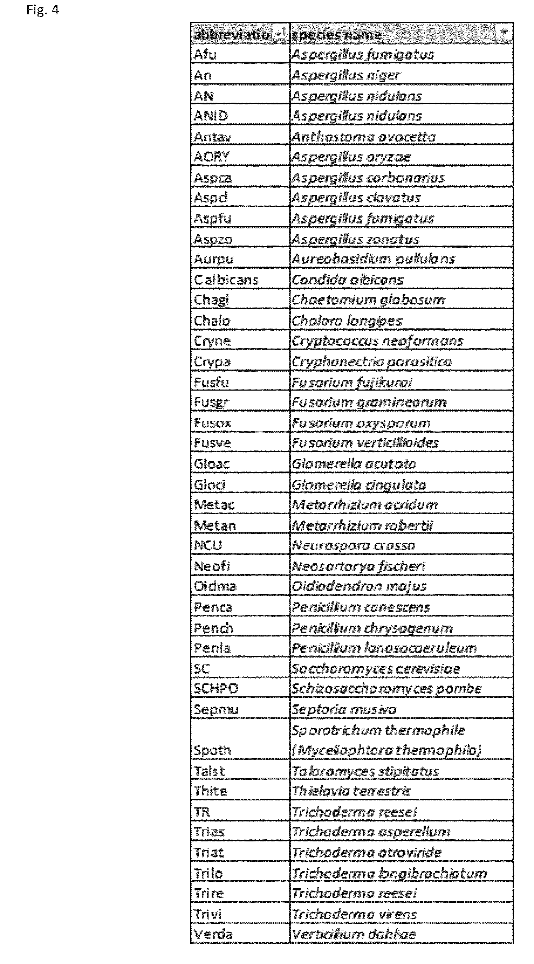

However, as the *Aspergillus nidulans* homologue only appears once in these two clades and related proteins of *Trichoderma* spp also are found in separate clades, the proteins in these two clades are regarded as homologues of LCS8 (Figure 3). Figure 4 gives the abbreviations used in Figure 3.

Interestingly, the proteins most similar to LCS8 from *Fusarium* spp. already cluster with another *A*. *nidulans* protein and are hence unlikely to be true homologues of LCS8 and also *A. niger* proteins appear to be more distantly related.

### Example 3 - Mutations in LCS8 in other strains of T. reesei

Published data on known conventional mutants of *T. reesei* were screened for information on potential alterations in LCS8/*lcs8*. Of the cellulase high producer strain RutC30, the complete genome sequence is online available (http://genome.jgi-psf.org/TrireRUTC30 1/TrireRUTC30 1.home.html). Comparison of the QM6a (wild-type) sequence with that published for RUTC30 revealed that the DNA sequence is 100 % similar between the two strains and hence no mutation has occurred. However, the automatically created protein models of the two strains appear to be different, which is an artifact from computer aided annotation. For the early improved mutant strain QM9414 the genome sequence is not publicly available, but a published CGH analysis of this strains did not reveal any mutation in or around *lcs8* (Vitikainen *et al.,* 2010).

### Example 4 - Investigation of the function the lcs genes in T. reesei in regulation of secondary metabolism and cellulase gene expression

Deletion of *lcs8* was performed using streamlined high-throughput procedure for *T.reesei* (Schuster et al., 2012) in the QM6a *tmus 53* background (Steiger et al., 2011) or the background of *ku80* deletion that abolishes Non Homologous Endjoining with the primers listed in Table 3.

**Table 3. Oligonucleotides used as primers for construction of deletion vectors or for RT-qPCR of genes.**

| Name | Sequence: 5'-XXXXXX-3' | Purpose |
|---|---|---|
| pdel_43701_5F | GTAACGCCAGGGTTTTCCCAGTCACGACGGTAGAAGGCATCGTAGGTCC | Amplification of 43701 gene 5' flanking region for deletion |
| pdel_43701_5R | ATCCACTTAACGTTACTGAAATCTCCAACGTGGTTATACTCCGAAGTCC | |
| pdel_43701_3F | CTCCTTCAATATCATCTTCTGTCTCCGACGCCCTCTCTCATACATCAGC | Amplification of 43701 gene 3' flanking region for deletion |
| pdel_43701_3R | GCGGATAACAATTTCACACAGGAAACAGCGTGTTCAGCTCTTGAAGTGG | |
| pdel_53238_5F | GTAACGCCAGGGTTTTCCCAGTCACGACGCATTCTACAGAGGCACTAGG | Amplification of 53238 gene 5' flanking region for deletion |
| pdel_53238_5R | ATCCACTTAACGTTACTGAAATCTCCAACGAGATGTACGAGTGATGAGC | |
| pdel_53238_3F | CTCCTTCAATATCATCTTCTGTCTCCGACCTATCCATCTGCCATAGACC | Amplification of 53238 gene 3' flanking region for deletion |
| pdel_53238_3R | GCGGATAACAATTTCACACAGGAAACAGCCTCCATGCCTAATACCTACC | |
| pdel_53776_5F | GTAACGCCAGGGTTTTCCCAGTCACGACGACACTCACCTTCTCATCTCC | Amplification of 53776 gene 5' flanking region for deletion |
| pdel_53776_5R | ATCCACTTAACGTTACTGAAATCTCCAACGACTCCTCCACTTACATTCC | |
| pdel_53776_3F | CTCCTTCAATATCATCTTCTGTCTCCGACCTATAGTGCTAGACCCCAGC | Amplification of 53776 gene 3' flanking region for deletion |
| pdel_53776_3R | GCGGATAACAATTTCACACAGGAAACAGCATCTAGCCGTGATACTCTGG | |
| pdel_73604_5F | GTAACGCCAGGGTTTTCCCAGTCACGACGAGTACCCGACTAATGACTGG | Amplification of 73604 gene 5' flanking region for deletion |
| pdel_73604_5R | ATCCACTTAACGTTACTGAAATCTCCAACCTCTATTCGTCCCTGCTACC | |
| pdel_73604_3F | CTCCTTCAATATCATCTTCTGTCTCCGACTCGTGAGAGAGAGAGAGAGG | Amplification of 73604 gene 3' flanking region for deletion |
| pdel_73604_3R | GCGGATAACAATTTCACACAGGAAACAGCTAGCACCAAGAGTAAGCTCC | |
| pdel_73618_5F | GTAACGCCAGGGTTTTCCCAGTCACGACGATCTGGCTTGGATACTCACC | Amplification of 73618 gene 5' flanking region for deletion |
| pdel_73618_5R | ATCCACTTAACGTTACTGAAATCTCCAACGATCAGCAGATCCTGTAAGC | |
| pdel_73618_3F | CTCCTTCAATATCATCTTCTGTCTCCGACCACACACTCACATCTTCAGC | Amplification of 73618 gene 3' flanking region for deletion |
| pdel_73618_3R | GCGGATAACAATTTCACACAGGAAACAGCGCTGTACTATCCGTAGAACC | |
| pdel_73621_5F | GTAACGCCAGGGTTTTCCCAGTCACGACGGTCGAAGAAGTGAGAGATGC | Amplification of 73621 gene 5' flanking region for deletion |
| pdel_73621_5R | ATCCACTTAACGTTACTGAAATCTCCAACGAGTTGAGATACTCCGATGC | |
| pdel_73621_3F | CTCCTTCAATATCATCTTCTGTCTCCGACGTAGTCATTTGCTGGTGTGG | Amplification of 73621 gene 3' flanking region for deletion |
| pdel_73621_3R | GCGGATAACAATTTCACACAGGAAACAGCGTCTTCACTCACCAACATCC | |
| pdel_73623_5F | GTAACGCCAGGGTTTTCCCAGTCACGACGCTCTTCTCTATCAGCAGTGG | Amplification of 73623 gene 5' |
| pdel_73623_5R | ATCCACTTAACGTTACTGAAATCTCCAACCACACCAGCAAATGACTACC | |
| | | flanking region for deletion |
| pdel_73623_3F | CTCCTTCAATATCATCTTCTGTCTCCGACGCGTAGCATTACTCCATAGC | Amplification of 73623 gene 3' flanking region for deletion |
| pdel_73623_3R | GCGGATAACAATTTCACACAGGAAACAGCTACGGTCGCTCACTATTAGG | |
| pdel_73631_5F | GTAACGCCAGGGTTTTCCCAGTCACGACGACCTGAAAGGTAAGGTGACG | Amplification of 73631 gene 5' flanking region for deletion |
| pdel_73631_5R | ATCCACTTAACGTTACTGAAATCTCCAACAGCTGCTATTGAACCAGAGG | |
| pdel_73631_3F | CTCCTTCAATATCATCTTCTGTCTCCGACTCCGTTTACACTGAGACAGC | Amplification of 73631 gene 3' flanking region for deletion |
| pdel_73631_3R | GCGGATAACAATTTCACACAGGAAACAGCCATACGTCCGAAGAGTCACC | |
| 102497_5F | GTAACGCCAGGGTTTTCCCAGTCACGACGGACGCAGTAGATTGTCATGG | Amplification of 102497 gene 5' flanking region for deletion |
| 102497_5R | ATCCACTTAACGTTACTGAAATCTCCAACAGCTACTCATTGGTCAGAGC | |
| 102497_3F | CTCCTTCAATATCATCTTCTGTCTCCGACCTGGTTAAGGTAAGCAGAGC | Amplification of 102497 gene 3' flanking region for deletion |
| 102497_3R | GCGGATAACAATTTCACACAGGAAACAGCGGCCTTACCATTGTGTCTGG | |
| RT_43701_F | GTCAGCACCATTTGGCTTCGGC | qPCR primers. Also Used for inside ORF amplification to check deleted |
| RT_43701_R | TCCCTGACAACTGCGCCATAGC | |
| | | mutants |
| RT_53238_F | AGCGTCATCACCATGTCTCCGC | qPCR primers. Also Used for inside ORF amplification to check deleted mutants |
| RT_53238_R | CGCTTCACTGACGACGAGGGAG | |
| RT_53776_F | GCGCACTGGAGTATCTGCACGA | qPCR primers. Also Used for inside ORF amplification to check deleted mutants |
| RT_53776_R | CGGTAGCCCTGTCAGCATCTCG | |
| RT_73604_F | AAGACCTCCCAGCCGACCGATA | qPCR primers. Also Used for inside ORF amplification to check deleted mutants |
| RT_73604_R | AACGTCTCGTACTCGCACCAGC | |
| RT_73618_F | ATGACGAGGATAGCAAGGCGGC | qPCR primers. Also Used for inside ORF amplification to check deleted mutants |
| RT_73618_R | AATGGACAACTCTGCTCCCGCC | |
| RT_73621_F | GCAACCTCGTCGATTTGGCTGC | qPCR primers. Also Used for inside ORF amplification to check deleted mutants |
| RT_73621_R | AAGTGTCTCGAGAAGGACGCGC | |
| RT_73623_F | GACGAGGATGACGTGAAGCGCT | qPCR primers. Also Used for inside ORF amplification to check deleted mutants |
| RT_73623_R | GCCAAGACCAGCGAGTCTTCCA | |
| RT_73631_F | AACGGCTCCGAAATCACTGCGA | qPCR primers. Also Used for inside ORF amplification to check deleted mutants |
| RT_73631_R | CCCCGGCATCAGATATCGCAGG | |

Briefly: For deletion of *lcs8* in the QM6a Δmus53 strain, the 5' flanking sequence was PCR amplified using primers 5F_tr_102497_ and 5R _tr_102497_ to obtain a 1148bp fragment. The 3' flanking sequence was PCR amplified using primers 3F_tr_102497_and 3R_tr_102497_to obtain a 1115bp PCR product. Plasmid pRS46 was digested with EcoRI and XhoI. The hygromycin-resistance cassette hph was PCR amplified using primers hphNCU_F and hphNCU_R to obtain a 1447bp fragment. PCR products (flanking region and selectable marker) and the digested vector were assembled in yeast by homologous recombination (Schuster et al., 2012) to obtain the plasmid pDELTR_102497_hph. The deletion cassette was PCR amplified from pDELTR_102497_hph and was used for the transformation of protoplasts of QM6a Δmus53. Transformants were selected on plates containing 100µg/mL hygromycin B. Stable transformants were obtained after two rounds of single spore isolation. Oligonucleotides used for construction of deletion vector and screening.

All other deletion strains were prepared accordingly using the primers listed in Table 3.

Analysis of the metabolome of wild-type and mutant strains under different conditions in light and darkness was performed using various chromatography and mass spectrometry approaches. With this technique around 2000 metabolites, especially such of fungi can be routinely detected.

Due to the importance of *T. reesei* for production of plant cell wall degrading enzymes, the influence of of the lcs genes on cellulase production was investigated. Several independent mutant strains per gene were grown on Mandels Andreotti minimal medium with 1 % (w/v) microcrystalline cellulose as carbon source in constant light and constant darkness and analyzed for cellulase activity (Megazyme Azo-CM Cellulose Kit). The respective parental strain (QM6aΔku80 or QM6aΔmus53) was used as a control. At least three independent biological replicates were used in this analysis. Therefore cellulase production was analyzed in the deletion mutants using standard techniques.

In summary, functions in regulation of cellulase gene expression and/or secondary metabolite production were observed for all genes analyzed and in most cases their function differs between growth in light and darkness on cellulose.

### LCS1

Although deletion of lcs1 did not have a major influence on biomass production upon growth in the presence of cellulose (25-35% decrease) in light or darkness, transcription and expression of cellulases strongly decreased in these mutants (more than 10 fold) in darkness, but not in light. Hence, LCS1 has a dark-specific positive effect on cellulase production.

With respect to regulation of secondary metabolism, deletion of lcs1 strongly decreases production of all secondary metabolites detected in the cultures in the wild-type (Trichodimerol, Dihydrotrichotetronine, Alamethicin, Orsellinic acid and Paracelsin A and B) in darkness. Except for Trichodimerol which was decreased by more than 60 %, abundance of all other metabolites was decreased even by 97 % or below detection limits. It is therefore evident that also other not yet described secondary metabolites of *T. reesei* will be considerable decreased in an LCS1 mutant or totally abolished in darkness.

In light Orsellinic acid was not detected, neither in the wild-type nor in any mutant analyzed.

For Trichodimerol and Dihydrotrichotetronine, decreased production was observed in light (by 25-40 %), but Alamethicin was even increased (by 75%) in the mutant strain.

In summary, these data obtained in the course of the present invention show that LCS1 is a darkness specific positive regulator of secondary metabolite production and enzyme expression.

### LCS2

LCS2 positively influences growth on cellulose in light (40 % decrease of biomass in the mutant), but has a negative effect on cellulase production under these conditions. In the deletion mutants specific cellulase activity increased by roughly 40 %. In darkness however, no influence on biomass formation was detected, but specific cellulase activity decreased by more than 40 % in the mutants and transcript abundance even by more than 70 %. These data show that LCS 2 is a light dependent regulator of cellulase gene expression.

Lack of LCS2 causes decreased abundance of Trichodimerol and Dihydrotrichotetronine (by 80-90%) in darkness, but only has small effects on the other secondary metabolites under this condition. In light absence of LCS2 in contrast increases levels of Trichodimerol, Dihydrotrichotetronine and Alamethicin 1.5-2.3fold.

In summary, these data obtained in the course of the present invention show that LCS2 is a light dependent regulator of cellulase and secondary metabolite production.

### LCS3

LCS3 is a negative regulator of cbh1 transcription (4fold increase in mutant) in darkness and lack of LCS3 causes considerably decreased biomass formation on cellulose and correspondingly lower cellulase activity in the supernatant.

LCS3 is required for high level production of Dihydrotrichotetronine, Alamethicin and Paracelsin A and B, with 60-90% decrease in the mutant strains.

In summary, these data obtained in the course of the present invention show that LCS3 is a regulator of cellulase and secondary metabolite production.

### LCS4

For deletion of LCS4 only small changes in cellulase transcription and activity were observed in darkness and light, albeit in light a decrease in biomass formation by roughly 30 % occurred upon growth on cellulose. Therefore, there is currently no basis for a function of this polyketide synthetase in cellulase regulation.

Upon deletion of LCS4, production of Trichodimerol and Dihydrotrichotetronine are abolished in darkness, while no major changes in abundance were observed for the other secondary metabolites in darkness.

In light, abundance of Trichodimerol is decreased by more than 30 %, and Dihydrotrichotetronine is abolished, while abundance of Alamethicin and Paracelsin B are even 4fold increased.

Consequently, LCS4 is essential for production of Dihydrotrichotetronine, but not Trichodimerol (which is still produced in light in the mutant). Additionally, the altered secondary metabolite composition caused by deletion of LCS4 obviously results in increased production of other metabolites.

### LCS5

Deletion of LCS5 also caused only small changes in cellulase transcription and activity as well as biomass formation in light. In darkness, however, an increase by more than 50 % was observed. Hence a feedback of the altered secondary metabolite production as caused by deletion of LCS5 on enzyme expression in darkness cannot be excluded.

LCS5 is essential for production of Trichodimerol and Dihydrotrichotetronine in light and darkness. Alamethicin, Orsellinic acid, Paracelsin A and B are somewhat increased in an LCS5 mutant in darkness (20-60%). In light Paracelsin B is decreased by 70 % and Alamethicin by 30 % in contrast to darkness in the mutant.

These data obtained in the course of the present invention show that LCS 5 is involved in biosynthesis of Trichodimerol and Dihydrotrichotetronine and causes altered regulation of other secondary metabolites in light and darkness.

### LCS6

LCS6 was found to be a darkness specific, strongly positive regulator of cellulase transcription and activity specifically in darkness with more than 10fold decreased values in the mutants. In light, only small changes were observed.

LCS6 is essential for production of Trichodimerol and Dihydrotrichotetronine in light and darkness. Additionally, Alamethicin, Orsellinic acid, Paracelsin A and B are strongly decreased or abolished in darkness in the LCS5 mutant as well. In light, production of Alamethicin is roughly 2.4fold increased.

These data obtained in the course of the present invention show that LCS 6 is plays a role in biosynthesis of Trichodimerol and Dihydrotrichotetronine as well as Orsellinic acid and causes altered regulation of other secondary metabolites in light and darkness.

### LCS7

Deletion of LCS7 caused somewhat decreased cellulase activity and transcription values by approximately 20 %, which we do not consider sufficient to assign a function of this transporter in cellulase regulation.

Deletion of LCS7 only caused small effects on secondary metabolite production in darkness with only 10-20 % differences. However, in light, Trichodimerol and Dihydrotrichotetronine were strongly overproduced in this mutant (4-10fold). Consequently, the function of LCS7 negatively influences production of specific secondary metabolites.

### LCS8

Deletion of LCS8 causes considerably decreased biomass formation upon growth in darkness on cellulose (decrease by more than 80 %), but a roughly 2.5fold increase in cellulase transcription and activity. In light only small changes in secreted cellulase activity (decrease by roughly 20 %), but not cbh1 transcript levels were detected. This shows that LCS8 is a darkness specific cellulase regulating transcription factor.

LCS8 is essential for induction of Orsellinic acid production in darkness and is a positive regulator of Trichodimerol, Dihydrotrichotetronine, Paracelsin A and B in darkness. In light deletion of LCS8 causes decrease of Trichodimerol production by 80 % and small increases of Alamethicin and Paracelsin (20-60 %).

In summary, these data obtained in the course of the present invention show that LCS8 is a light dependent regulator of cellulase and secondary metabolite production.

### LCS9

Although cbh1 transcript levels do not change in darkness on cellulose, secreted cellulase activity and biomass formed under these conditions are significantly decreased in the LCS9 mutant with cellulase activity of only about 50 % of the wild-type. In contrast, this mutant strain grows almost 1.5fold better than the wild-type on cellulose in light and also cbh1 transcription in more than 2fold elevated. Nevertheless, secreted cellulase is still 30 % lower than in wild-type.

Hence, LCS9 is involved in light dependent regulation of cellulase gene expression.

LCS9 is essential for production of Trichodimerol and Orsellinic acid in darkness and its deletion causes decreased production of Dihydrotrichotetronine, Alamethicin, Paracelsin A and B (by 30 -60 %). In the mutants, Trichodimerol is decreased in light as well (by 80 %), but Dihydrotrichotetronine is increased by 60 %.

In summary, these data obtained in the course of the present invention show that LCS9 is involved in light dependent regulation of cellulase and secondary metabolite production.

### LCS10

lcs10 is a regulatory target of LCS8 in darkness and not transcribed in light. Therefore LCS10 has a role in mediating the function of LCS8 in cellulase gene expression and secondary metabolite regulation. The genomic position in close vicinity to LCS1-9 of which many have functions in regulation of cellulase gene expression and/or secondary metabolite production strongly indicates a similar function for LCS10 as well. This assumption is further supported by the conserved genomic position in P. chrysogenum, A. niger and Fusarium fujikuroi (see below).

### Example 5 - Conservation of the G-protein coupled receptors in other fungi

Investigation of the function of the G-protein coupled receptor (GPCR) encoded by lcs1, LCS1, showed that it positively regulates production of secondary metabolites as well as cellulase expression.

Consequently, its deletion can be applied to prevent production of unwanted secondary metabolites in fermentations. In contrast, constitutive activation of this GPCR or its homologues is expected to enable induction of otherwise silent secondary metabolite gene clusters.

Since deletion of LCS1 strongly decreases cellulase gene expression, a corresponding mutation is useful to express a desired heterologous protein in a low cellulase background.

However, the function of LCS1 also opens up an interesting perspective for fighting pathogenic fungi:
More than half of the therapeutic agents currently on the market have GPCRs as targets. These also include a considerable number of the top 100 best-selling drugs. Consequently, assays to screen for GPCR ligands are major subjects of current research (Zhang et al., 2012). GPCRs have a long history of use as drug targets and consequently a large toolset for discovery of agonists and antagonists to activate or inactivate the function of GPCRs is available. Nowadays high throughput screening is the method of choice to identify effective ligands for manipulation of the activity of GPCRs (Jacoby et al., 2006).

Once a compound binding to the GPCR is found, the question remains whether this compound represents an agonist or antagonist.

Additionally, GPCRs are known to activate not only one, but often multiple signaling pathways, as is also the case for LCS1, which targets both secondary metabolism and enzyme expression.

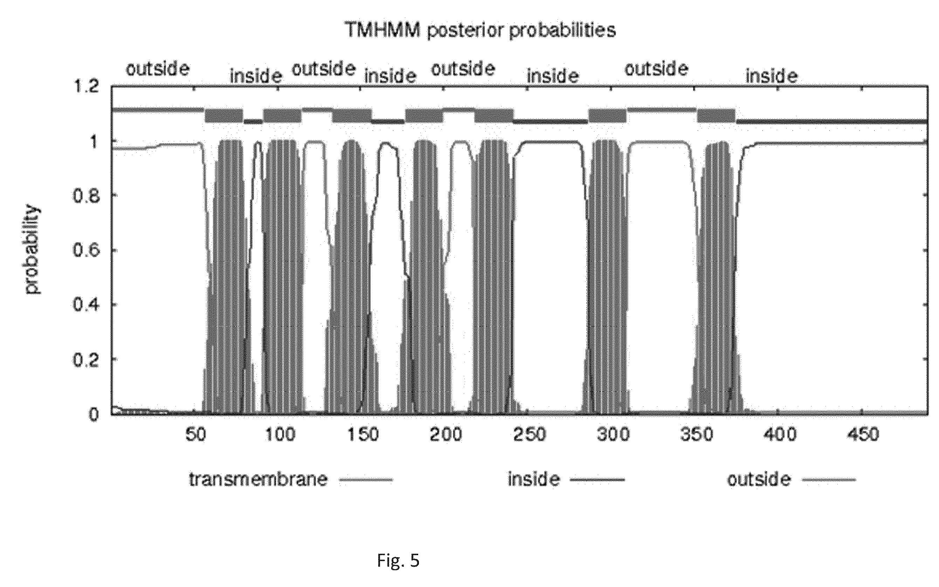

The areas of the GPCR-protein which are binding agonistic or antagonistic ligands are predominantly found in the extracellular region of the GPCR and can be specifically targeted by extracellularly present compounds (Wichard et al., 2011). Intra-and extracellular regions as predicted by TMHMM are shown in Figure 5.

Consequently, antagonistic ligands can be applied to inhibit the function of the GPCR and hence achieve the same effect as genetic manipulation of the GPCR sequence (such as deletion) would have. Therefore, the GPCR LCS1 can also be a target for drug development in case of human pathogenic fungi or for specific fungicide development in case of plant pathogenic fungi.

LCS1 belongs to GPCR class VII (secretin-like, related to rat growth hormone releasing factor) and is conserved in all sequenced *Trichoderma* species as well as numerous plant pathogens such as *Fusarium spp., Botrytis cinerea, Sclerotinia sclerotiorum* etc. and also human pathogens like *Histoplasma capsulatum, Paracoccidioides brasiliensis* and *Aspergillus fumigatus* (Figure 6) .

### Example 6 - Conservation of genomic localization of the lcs genes in other ascomycetes.

As shown in Table 1, the localization of the lcs genes spans a genomic area of roughly 100 000 bp in *T. reesei.* We analyzed genomic localization of the homologues of the lcs genes in other fungi and found that conservation of this locus is limited.

Even though *Fusarium* spp. are relatively closely related to *Trichoderma,* conservation of the localization of lcs genes in *F. oxysporum lycopersici* f. sp. 4287 and in F. *graminearum* is limited to *lcsl* and *lcs2.* In *F. fujikuroi lcs4, lcs5, lcs 6* and *lcs10* are found with relatively small distance on the same scaffold. A similar situation was detected for *Aspergillus niger NRRL3* and *A. niger* CBS513.88, but not in A. niger ATCC1015, in which *lcs10* is on a different scaffold. In *Aspergillus nidulans* and *Cryphonectria parasitica* only *lcs5* and *lcs6* are close to each other and in *A. fumigatus* only *lcs7* and *lcs8* are in vicinity in the genome. Hence, in general the localization of the lcs genes in close proximity in the genome is not highly conserved outside of the genus *Trichoderma.*

However, we found that in *Penicillium chrysogenum* localization of a considerable part of the *lcs* genes is indeed conserved with *lcs4, lcs5, lcs6, lcs7, lcs8* and *lcs10* in close proximity. This degree of conservation despite the phylogenetic distance of *Trichoderma* and *Penicillium* suggests a specific functional relevance of this group of genes.

Interestingly, a comparable gene locus appears to exist in *Aspergillus nidulans* FGSC A4, with AN1034 and AN1036 as PKS genes, AN1033 as a monooxygenase and AN1029 as transcription factor. This cluster is responsible for the synthesis of asper-furanone (Chiang et al., 2009). However, the best homologues of the LCS genes in *T. reesei* are AN7903, AN6791, AN7902 and AN3385. Consequently, the cluster corresponding to the LCS genes described here is not characterized in *Aspergillus nidulans.*

In *Aspergillus niger* ATCC1015 the two PKS genes and the monooxygenase (*lcs4, lcs5* and *lcs6*) have homologues with the protein IDs 1148627, 1080089 and 1114655, but the two transcription factors and the other lcs genes are not present in this locus. Zabala et al., (2012) characterized the cluster in *A*. *niger* and termed the characterized genes aza genes. The homologous genes to *T. reesei* are *azaA*, *azaB* and *azaH*, but the transcription factor in the *A. niger* cluster described by Zabala et al., is different. Moreover, this cluster is described to be silent and activated by a transcription factor related to but not homologous of *lcs10.* Hence also the regulation can be expected to be different.

Additionally, a regulation of the abundance of Alamethicin, Paracelsin A or B, Orsellinic acid, Trichodimerol, Dihydrotrichotetronine or cellulase gene expression is not reported.

The present invention therefore discloses the following preferred embodiments:
1. A fungal strain of the phylum Ascomycota, preferably *Trichoderma, Neurospora, Penicillium* or *Aspergillus,* wherein one or more of the lcs genes or is either
   (a) overexpressed; or
   (b) underexpressed or in which
   (c) the function of the encoded protein is altered or abolished by genetic manipulation
   in said fungal strain, compared to an unmodified wildtype fungal strain of the same genus cultured under controlled light conditions.
2. A fungal strain of the phylum Ascomycota, preferably *Trichoderma, Neurospora, Penicillium* or *Aspergillus,* wherein one or more of the lcs genes is absent in said fungal strain, compared to an unmodified wildtype fungal strain of the same genus cultured in a normal light-darkness cycle, with the proviso that said fungal strain is not the *T. reesei* knock-out mutant Δtr_102497 as disclosed in Schuster et al. (Biotechnology for Biofuels, Volume 5(1), 2012, 1-10).
3. A fungal strain according to embodiment 1 or 2, wherein said fungal strain belongs to the species *Trichoderma reesei.*
4. A fungal strain according to embodiment 1 (b), wherein said fungal strain is cultured either in continuous light or in continuous darkness.
5. A fungal strain according to embodiment 1 (b) or 4, wherein the gene encoding an LCS protein is downregulated in said fungal strain by genetic manipulation of said fungal strain, wherein said genetic manipulation preferably leads to expression of Antisense RNA to and LCS gene mRNA.
6. A fungal strain according to embodiment 1 (b) or 4, wherein the gene encoding an LCS protein is downregulated or upregulated by removal or manipulation of the LCS DNA motif 5'T/A ACTCCGA 3' .
7. A fungal strain according to embodiment 2, wherein the gene encoding an LCS protein is knocked out in said fungal strain by genetic manipulation of said fungal strain.
8. A fungal strain according to embodiment 1 (a), wherein the gene encoding an LCS protein is upregulated in said fungal strain by genetic manipulation of said fungal strain, wherein said genetic manipulation is preferably achieved by promoters selected from the group consisting of *tefl, cbh1, gpd1, hfb2* and other strong promotors routinely used for heterologous expression in *T. reesei* being operably linked to the gene encoding an LCS protein.
9. Use of a fungal strain according to embodiments 1(b), 2, 4, 5, 6 and/or 8 for production of heterologous proteins.
10. Use of a fungal strain according to embodiments 1(a), 6 and/or 9 for the production of secondary metabolites.
11. Use of a fungal strain according to embodiments 1(a), 6 and/or 9 for the production of cellulases or modulation of their production levels.
13. Method for cultivating a fungal strain under toxin reduced conditions or for avoiding contamination of fungal products with further metabolites as regulated by LCS proteins.
14. Method according to embodiment 13, wherein said fungal strain is a fungal strain according to embodiment 2 or 6.
15. Method according to embodiment 13 or 14, wherein said fungal strain is further defined as belonging to the species of *Trichoderma reesei.*
16. Use of the method according to embodiments 13, 14 or 15 for the production of Alamethicin-free strain products.
17. Use according to embodiment 16, wherein the strain product is defined as being a protein.
18. Use according to embodiment 17, wherein the protein is further defined as being a heterologous protein.
19. Use according to embodiment 18, wherein the heterologous protein is further defined as being a protein of human origin.
20. Use of an LCS gene product, especially LCS1 as a target for drug development in plant pathogenic fungi such as *Fusarium* spp., *Botrytis, Sclerotinia, Pyrenophora* etc and in human pathogenic fungi such as *Paracoccidioides brasiliensis, Histoplasma capsulatum* or *Aspergillus fumigatus* etc.
21. A fungal strain of the phylum Ascomycota, preferably *Trichoderma, Neurospora* or *Aspergillus,* wherein one or more of the LCS genes individually, or in combination is either
   (a) overexpressed, especially in the case of LCS1 and LCS6 to improve cellulase gene expression, and LCS1, LCS2, LCS4, LCS5, LCS6, LCS8 and LCS9 to improve Trichodimerol and/or Dihydrotrichotetronin production in the dark, or
   (b) *underexpressed,* especially in the case of LCS8 to improve cellulase gene expression and of LCS1, LCS3, LCS6, LCS8, and LCS9 to prevent Paracelsin and/or Alamethicine production in the dark and LCS7 to improve Trichodimerol and Dihydrotrichotetronin production in light,
   in said fungal strain, compared to an unmodified wildtype fungal strain of the same genus.

### Sequences

### Protein sequences encoded by lcs genes

>lcs1_TRIRE2_53238
MANITGINLCPAPFYDASLFGDSGGYISGRLCQTIAGSVQCCLPCPMTDWVYPDSFTTIGTVANWIATAS
VICCVFLLASWAALPVDKTNRHYLSICFTIGILIMNLGFVIPLAAEPDQCFDAITPNDMHTLSVCGASGA
MLILGGWCGVMWAFLRSLSLHLQICWQVLVGRNFMIFAQAAGWGIPAIGIILALVFSGVSFRFGATCHIN
HKNSLADLWIPLLIFASITVVTTFVTFGYCIKVYLASLNDNSASTEGSNLPTYTNSVITMSPRQAYRRVR
RVIALQWRGIAIVLIIIADVIFFSVVFVFQDNIVQSIADDPKVAQNWIVCLIGAQGDKTKCLNEAGSLVV
SEATVSAVLVLLSLNGIWILFLLGRWTMVTGWIDLVKSPFNSRKKEFVSVDARYQSQKDTRSYEMLSRDT
SAAVTPLDPVKSSAGPQAPDYFGDTARYHAPARSYSNPRPPHSSLPEWDAQKTYAAPQGRDMNPLGMNRI

>lcs2_TRIRE2_73604
MPASQPISKPARGDTSDYRPVHQDDRQDDSQDGTEEEGVVSPAYLPPRRSRADRRYRTTILVLVVALVIL
LATNLYLSLPYLIYGSGPGPGSCPPPSKVPQYFQTSPELWAGPTATGRAAFMAQTRTFDPTATFVPNEPL
QTAIPVEGMKPGNDTIFKMMGYLSPYFPSPGFGVDEYPLPSGAEIVQVQMLSRHGARYPTSGVGVTKFAQ
RIANASDKFYPKGPLSFLKDWEYNLGSEILVPKGREELYNSGVLHSYMYGRLYNPKSKIIVRTTTQDRML
KSAENWMAGFFGLEWTNNATIEVIIESHGFNNSLAGALNCPNSNSKHSADEAKEKWINTYLKDAVPRFQA
MTEGFEWTVDDVYNAQTLCPYETVAYGYSMFCDLFTYEEWQGFGYSIDVWFSGGNAFHSPVGRATGIGYQ
QEVLARLKNHTLGYSGSQINVTLDNNTETFPLNQSLYFDFSHDTNIISVLTAFGLRQFAEDLPADRYPGA
HNFTVSHLTPFGARLDIEIIKTPKPLSPNRDGYLRGEEQKYVHFILNQRTIPLGLSFPECDASRKDGWCE
YETFVKIQEGMSAKASYDYACFGDYPKEPYGVITDGVPK

>lcs3_TRIRE2_53776
MAKRERSPTGYGPQPEEAIPSPSRPGILTVVLHEGTGLSIPGSYKEDQRPDQQGLSKRNTPYAVLDYDRS
QQQADSDEGTAEDPVWVSDIVPWRLDEVTGEYRDANWKFDVCRPAELAIHLYLRDPHASPCVRSQDACIG
VARIPIDVAQASASEETPSQWVDVEGGTGRLRVSIKYDDTKDKTLEAADFIEQSKIQKGNSGYTARVIKK
DTRQRYTTRTIPAVRRPQNADHPFIAPLTLVFQSQEGLHLLSPTMCGGYLFQHLQNQRVFSLKRARIYAA
EILCALEYLHESRGIYSWLKPRNVLLDSLGHVVLCGSGLYDPGVEDDGRGGYGLPEYPAPEILRDQNRSG
AADWWTLGIFIYEMLTGLPPFFHENADEIRRQILSSDSIQFPDDLPPDARDVIAKLLDRKPDFRLGARGG
ASEVKEHPFFADIDCGKLKQREYTPSFKP

>lcs4_TRIRE2_73618
MGSLGPDSQSRWASEPIAIIGMSSKFAGDATNTDKLWQMLAEGRSGWTEFPASRFRSEGVYHPNNERLNT
THVKGAHFLQEDVGLFDAAFFSYSSETASSLDPQYRLQLESAYEALENAGIPLTQIAGSNTSVFTGVFVH
DYRDALLRDADNLPRLMATGTGVPMMSNRISHFFDLRGASMTIETACSSGMVATHQGIQSLRTGEADMSI
VGGANLTLNPDMFKALGSAGFLSADGKSYAFDSRASGYGRGEGVATIVMKRLSDALAAGDPIRAVIRSSL
LNQDGKTETITTPSLEAQIDLIRQCYARAGLDPRDTQYFEAHGTGTQAGDTVEARAIATVFSHNQDPLLI
GSIKTNIGHTEAASGLASIIKTALALEKGVIPASINFEKPNPKLSLEDWHLKLVRQLQEWPAASTRRASI
NNFGYGGANAHIILEDGASWTPSPVEGTSSKEPIDTGSRVLVLSGKDEQACRTMISNLAEYLQRVASAED
EPSRLLDSLAYTLGQRRTRFPWVAAHPVPVTEGIGAVVNTLQSPKFKPYRSSRRPRIGMVFTGQGAQWWA
MGRELRDAYPVYKASLDEADAYIRQFGADWSLVEELSRDAASSRINESGLSTPICVAVQISLVRLLESWG
VVPAAVTSHSSGEIAAAYTVGALSYKDAMAYAYHRAVLAADTSLRGPVKGGMLAIGLGREDTEAYLKRLT
TGGKAMVACVNSPSSTTVSGDLSAVQELEELANADGVFARLLKVETAWHSHHMTAIANVYVEALDNIKRK
NSRNESSIAYSSPVTGGRVANIEEVARPEHWVKSLVQPVQFVDAFTDMVLGNPNGSANVDVVVEVGPHTA
LGGPIQQILALPAFKGLQIPYYGCLVRKADAKDTMQALAANLLQQGYPLDMDAVNFPHGRGSRVRVLTGL
PSYPWNHQVKHWVEPRFNRALRERSVPPHHLLGSLVEGTNLEEPTWRHTLRISESPWTRDHAIQSNVVYP
AAGYICLAIEATKQLHALNHTKAGAKEVSGYRLRDVDFLQALMIPDTSDGIEIQTSLRPVNDKDVAIQGW
KHFEVWTVTGDNRWTQHAKGLISIEFEASAQVYEPKLGEFTIKGYKRQIPPAQLFANLKALGIGHGPVFQ
NMSHIVQSGFDRRSVVLTTVPDTSVPNDLPREHVLHPVTLDSFITSPYSAVPGAAGRETAAKVPRSVKSF
WVSSNISHSPEHVFKAHSHIIRDDKHGMEADVIVANDGVDDNNVLLEMKGFSYQSLGRSVSLQHTEPWES
QLCSSIHWRPDISIKLPATVSLVKQELSSNSNSAEAGGVEISSLCLYFIQKALASLSDSDFTNGSHYSKY
YAWMKSAAQQAAITDIDEEHIDQIAKAQADGEMIRLLGKQLVSILRGQSTPTEIMEQDKNLLSRFYSETP
RAKRTSSQLSGLLRHLVHKNPRARILEIGASTGGITGSALGVLDTATSGGPHASLYHYTDLSDRNFDAAR
EGFAAWSDILAFDVLDIERDPADQGFTVGSYDVVIASHAFSSTSSAIAGVLENVRSLLKPGGTLLFTEDF
KPSIDVQFVKGLFPSWWSSERFSEQHVEPSPLLSVPLWDRSLRHAGFTGIDIELRDSDDVDASVSATIMS
TLPPHPAGQSDIDAGKVVIVTSEQAGIPPSEWLKALQHSIASYSKAVNGAEGKVLPSVQSIESAAATAAW
YADKICIFIGEINEPILYNLDAASLEGIKAMSTGCKGLLWVTRGGAVDCERPEVSLATGFVRTLRNEYVG
RKFITLDLSPKGSLWQESGHEAIAQVLQNAFGQSLPGHSSGPDKGPVELEYAERDGVILIPRVYHDVAKD
NAITPKTLESEEDTQGITTVEPFYQQHRPLCFLPELLVFGDDASAAVYRDTLPPRLVEIMPRAYGAGLNP
ADRTITGQECSGIITRVGIEASKHGYSVGDRVVCLLQQSSFTSRAVVDWTSVVQMPSRLSFQQAASLPAA
FLVAYFSLVETARLKTSQSVLIHNAAGSIGQAAIMVAKHIGATVFTTVASPKQRDLLTREHGIPSHQIFD
SNNASFGTAVTAATNGRGVDVVLNSLTGPLLQTSFNLVAPLGHFIEVGKYDSLANSNLEMLPFTRGVSFS
AVDVPSLLQHRGSDVHRCLEEVMRLFELEALAPVSPVIEHNIGDIAQVSRLIQTEEETGKRVLSVAQDEM
VSVLPHTAPAATLSPDASYLIVGGNGGLGQAVAHWMVSRGAKNLVLLSRSAGQSPKMAVLAEELRDAGCH
RVLPVSCDVAKEDDLARAMDTCAREGLPPIRGVVHAAFVLHDSFVENMTLDDYKYTIQSKVSGAWNLHNQ
FNLPGDLDFFVLFSSINGILGYASQAAYSAAGAYEDALAHWRVKHKGLPAVSIDLSLVDGVGYVAEASAA
EAMRKSLIKAGRRVINEEQVLASLELAIVSPYDPQFILGGINSGPGPHWDVDGDLGRDMRLLALKYRQPA
AADGHDDEDSKAANGGDSLSAKIASASSRDEAISVVGSAVAAMLADMFLVSVEEVDLNDSPSQQGIDSLV
AVEVRNMLFSQAGAELSIFNIMQSPSLAQLVANVVDRSTFAKFAKSS

>lcs5_TRIRE_73621
MAASSTRTLLMFGPGAMSLNETYFASILSFISTDSASQWALSAVRDIESHWPSLCEAIPKLQHTSGVSNA
QKLAEWLRTGTLAPGSTIASLPNAILGPLVVIAQLVEYLRHVDSLSESGLRGGEGFQVPSAPDAETVGCC
LGTFSALVVSSSSSWAQFCHNASAVVRIVFVLGALSDAQDATDASGPSVSLIAFWRGGQSLSDLKKALEK
FPEAYISVLYDENRVTVTTSTRTVAALKNHLQTVGITTNETEFHGRFHAAQLYQTELEAFLAYCRRFPTF
QLPDSSCIVVPTRVNSENVVTSQESLLEISCRAFLVSQFDWIKTFRAAVSSTLQNRASRIIEFGPERCVP
PTLLRRLNSQVTHFDFEESIKRAKASLSHDQELPAGVAENDIAVIGMACKVAGADDVNEYWELLLQGKSQ

HRELIPNDRFVMETPFRPFEACDDKKKWFCNFICDHDAFDYKFFKKSPREALHMDPQQRLMLQAAYQAVA
QSCYYNANLNVHCPTKVCCYICVVANDYENNISHTTPIAFSATCALRSYIACKVSHFFCWTCPAMTVDTA
CSASTVALDLACKAILSCECSAALVCCTNFFSTPMFFQNLAACSFLSTTCQCKPFDAKADCYCRCEAVCT
VFLKKLSQAIADCDQVLCVISATAINQNRNETPIFVPNSPSLTNVFRTAIEKSCLDAKDISVVEAHCTCT
PVCDPVEYDSIRQVFCCAVRACQDALQVCSVKCLVCHTECASCVVALVKILLMLQRCQIPPQASFETINP
SIKYSPSDNLEITKTPLPWNQEFKAALINNYCAACSNASVIIKQCPAQLLRRLPPVVDSETEALLSKVCA
DDAQKAPFFISCLDEKAILAYAQKLRQFIYSHSNLDIQDLAFSVNRQSNWSLCRCAVFSACSIAELDEKL
ASIETFPVPSSQPPVILCFCCQVSTFVCLDYQLFAKSAILRRHLDQCDAACKSICACSIYPRIFQSDPID
DPSVLQPLLFSLQYSCAMSWIDCCIVPAKLVCHSFCELTALCISCVVSLQDCLKLVYCRSKIIKESWCAE
RCAMLAVEADLEELESLLTTVNSSLQECRATIACFNCPRSFTVACSSAAIDAVQQAISNTQPVLKHKRLN
VTNAFHSVLVERLKPDLEALCRQLTFALPQIPLERATRSREDHCLSPSYVANHMREPVYFHHAVERIAKE
YPEAIWLEACSNSTITTMASKALCLPKASTFQPVNVTNNSKASSQLSDVTMNLWKACLRITFWPHSRAQT
YEYKHIILPPYQFEKHRQWLEFKPPQALQVVVQTDSSTDARNCTTEPQPVCLYTLLDRCQDKYRFRVNTA
ACQFVDAMSDHAICKAQTLPAMFCVDLAIEALLSIHPELCDTSRFDPQIYNVVNQEYIHDSSRALFVLFE
RLCQDENSWAFELTSKCKDCAESLHMSCQLHFQASDDARSRLEFSRLDRFITHDRCLQVLESSCRSDEVI
QCQTIYSVLSSSDVNYCQRLRCLQRLVCRSNESACRLVRRRSCKLFVDFALCEVFTQVCSIWANCMAQHQ
RNTRNKCIYMATCLEQWSKSPKVLQKFNQCLYDNDPEIEWHVLAQHKRNTSDDSFTTDIFVFDAASCDLE
EVILCIKYAPVSLDQLFSCSAIATATTPVANCYVPLTTPFVPVPTTTKQAAVPQPQHVAKKAAPRAAPKR
DIKEELWLRLRPVLADISCLEPEEIQPTDALADICIDSLMCMEMAREVETTFNCTVEQSELLSIVDVPCI
LKFLQSTLCDEDVHDSSETMSTVSSDCNVHSPPTSCSEMASPNLKVSYCSAYCSSDLPISAVLEAFCESS
AKTDQFLKTYCCACYLDCVSQKQTRLCLVLTSAAFKQLCCDLEAAKPCEVLQPVPFVERHRRFHQYLYSM
LEETRIINIDCDVITRTAIPLPSQSADAILQDLMRRHADNCSSHQLAYNVCSRMADVLSCKADCPQLIFC
DAKNRELVANFYCELPFNKLYFELMADFLTRLANKLKLSSSRSCTPTLKILEMCACTCCTTKVLLPILAK
LCIPLEYTFTDLSPSLVAQAKRRFKEYPFMKFAVHDIEQPPSDPELVCSQHVVIASNAVHATHSLRDSAR
NIHKFLRPDCFLMLLEMMRTLHWVDVVWCTLECWWLFDDCRTHAIVNEQRWEKEILASCFKHVRWTDCKL
PEVHVQRVIIALACDCDCDVSDIPALTSPALKDEEDHCSKLDCEERKRVANAYVESTIRDFAIPSYTCPI
LSTAPCACACVLVTCATCSLCSHLVAHIACLPSVDTIYCLNRPRPCKKCQEDQSRDPLSRLTEVLASKSI
QLSEAEISKLRVIETDLPLPQFCLDEIQYEQLLNNVTHIVHSAFPVNCLRSLKQNEPQFTLMRNLVDLAA
CVSARRPTEFKFTFQFISSLSAVCMYPKVHCEKRVPEQQWDVDSALPNCYCEAKVICERVLLETLCQHPD
RFRAMTVRLCQLSCSMKTCYWNHMEMLSFLFKSAQTLRALPDVDCDVSWLHLEDASASLADLLLRDAPTC
HAVYHLDNPRPRDWKDVIPVLADALDIPSSHIVPFEEWLRRVRAYPCEDPWDNPSAKAMDFFEHKFKHMS
CCCVTMATDHALEHSETLRAVQPVSDELVRKYIQAWKDSCFLR

>lcs6_TRIRE2_73623
MEPNNHHDCLNCQKADNPLEVAIVCCCLTCLALALCLLRRNVNFTIYERAASFCELCVCIHFTPNAERAM
KALDPRILQSYVDVATHAECCFLSFVDCLHDDDLLFQLRMCECYKAARRCDIVSQLVKHIPQERVQLLKW
LQSVEEDAECRAVLTFRDCSTAKADVVVCCDCIRSQVRSAMFCSCPSAPRAQYAHQLAFRCLVPMAKVAA
TLCPEKTSRAICYLCPCCFVLSVPLACINMMHLEVFVMDPLPWSDDTTCSKPDKDKDEDDVKRYVLPSTR
AEAEKAFTEFNPTVRSLISLLPEELCKWAIFDMLDSPAPSYALCRMCLACDAAHASTPNQCCCACACMED
SLVLAEILAALADRAKSCARVCSWEISECLKIYSDARYERAQWLVQSSRRVAQLFTRKKCACQCCEEEEP
ISREILERSHQLWDYDVDAAVEEALGKLRAKVLEKH

>lcs7_TRIRE2_43701
MSKEASQDSRSITPVEAVEPLEVVDAEKNVTTSPYNGSGTVEDPFIVEFQQDDKSNPMNWGQFRKWFLTS
IVTFSVFAVTFTSSAYSVSAEEIMTEFDISSTLFITGVSVFVLGFAIGPAVWGPLVTPHDERSNANRASL
QSTRSELYGRQMPWIASHTAMVAFMAGSAGSPNIATLIVLRFLAGTFGGSPLVNSGGAIADLFPPAQRGL
AMTIYCVAPFLGPILGPIVGGFATEYIGWRWVQGMCTIFIGVIGIIGVIFVPETYGPVLLQRKANALSKA
DGKVYISVLQKNQGKKQPSEVFGRALIRPWVLLFREPIVLIASLYMAIIYGTVYMFLGAMPIVYNELRGW
SPGFGGLAFLGMMVGIIIGLGYAIWDNNGRYMKLDPSKRTAESRLPPAIAGAVALPIGMFAFAWTNYPSI
HWAVSIVLSAPFGFGVVLVILPIVNYLIDSYTVYAASVLAAAAVFRSIMGAVFPLFTSQMYHNLGIHWAT
SIPAFLTLVCMPFPFFMYRYGAVVREKCKYAAEAAQIMKKMQGR*

>lcs8_TRIRE2_102497
MSARQDEDQRLQAQTQAQAQVQAQAPLSLYRERLKIANACQSCRASKVKCDGGRPVCARCQKRGRACSYS
QHDAASPRGRGRQRAKAPTRQPRPIRSRASVELPVTAPTPVTAQASPLIAQDYSLQTPSATQTPSTTGFS
GSSDLEHVHEDRDESRAFYAAHGRFAGEVSSTIDKMAGLSPDTTCSLVPFVDAPLFGDVGEPPRNVVLDF
ASDLPRAYADRLLAIYWHHVHPVEPVLDQQQFCRTYDAFYSGSGTPLHVDRDIWLSTLNIVFALAVQIQE
SIPMQKRDDEANRYFQRAWALLRPEAILWKPGSLELVQCLLLMNRYLHCTNNQQKTSMAATLAIRIAQNM
VCHTSEESPSSDADKDLRHKVWASCVALERPALLTGHGSDFHAWELELHEIGTHIQLAQVQSKNSMATKL
GLPRLYQQDEYHAIAVQLDGCLNKWEKSLPDDWRLQNMHMIHDRRARAERYLLHFRLLHSRIYLHRPMLA
RLYAIKSHAPTAAAASDPSTISDRLLQECARMCLEAAQKLTSLIAEIHDPNEPIGILPWWYRVYYLHIAG
IHFLAAMFASDLFTPSVERAWYQVLAALRAHEHLSLYVQQCARTFETLAARILNARCLSVNGNGIMALDD
GAPGLFLDDMFQDVNFDLDEFLFSVDDTGRRTNY

>lcs9_TRIRE2_73631
MYAPPFVRAFGIAVLAVLPSFSSPATAASLKSSGSSSSCRCFPGDACWPSPADWKAFNQSVGGRLIATVP
LGSVCHGTTYDAARCADVKAAWPYADTHTDSSSSVLAPFFANQSCDPFLPRETPCVIGTYVQYAVNVSSV
ADIQKTLAFSQKKNLRLVVRNTGHDYFGKSTGAGGLGLWMHNLKTYDIHDYKSAAYTGKAVTMGAGIQAG
ESAATAFKQGLTIVSGICPTVGLAGGYTQGGGLGPLTTRYGLGADQVLEWHAVLANGSEITATPTKNSDL
YWALTGGGGGTYAVVYSMTVKAHANEKTTGANLTFPNAGSEDVFFQGVQAFHDIIPAISDAGGTAVWTVL
SKALSVGPVTGPNMTKATMDSIFQPVLQKLDALNITYSYSSGEFSSFYESNAAYDPPVVSNGLQIGGRLV
KRSDFTGNPDGFIQAIRGIADQGGLVTGASYQLSSSLQHPPNSVNPELRKSLISFQIGVPWINTDWATDL
HNQDLITNSFVPALAALLPSGGSAYLNQADFREPGWQQVFYGENYEKLLELKDVYDPNGVFWGRTTVGSE
RWAETEDKRLCRVS

>lcs10_TRIRE2_102499
MGSSATATTTGESTRQQPGLACEECRRRKARCDRVRPKCGICADSGRNCVVLDKRSQRGPKKGQLKDLRS
RLMLLEQRLVGQNDAPSLPQERDSLGCPTPSEKVSPEGDLVSSRASTDIGLDTCMGSTAGAFALFGSTSG
SDKMPPLTPDLSTASTANSMAACICKGWQTEPAMFSTSPFPQIPLTPQSTTAPSRMPSISLAAADPIMPD
VRVHPFAPMVHKRRYYAWASDPNASPARTALRSAMRTIASAMSPQFCDIGHVMYASTRRMLETQDACPET
GLPWMTRLKPPHEQRKMHHERIQAWLLLAYYDVLRKSEHQAFITARRAFRLLRLSGLCEMDIDAYSGRQG

DVATCTPPAETTWNMQMCSSENGADEAVLQQDWISVEERRRTVWSAFLLDRLSTMVNDQPTMLMEEAVTK
PHACTFSWYFSADLPISNQFYTRLPMSEAEFQSGTQEPVSQMGFLIEATDGIKASNSIQPLPPFAHCVVV
ANLFARCMTHCKMAMQSPPMSAPEAHDFWIRHQWLASAAANACESTETRCDPMLVFTRILAYSASLSLCS
TANATSWQTLDHHLMAMACKPAAHQAASEVVRIIKTAPRIAFFKMHPFFPNAIALVTSFLNADVPYLPST
RGGNAMDAIQERQDAVNELLAALRRSSQVNNLAAELLCKLELDIGQAASDGSIYG

### Promotor sequences used for analysis to detect common sequence motifs in the lcs genes

>lcs1_promotor_TRIRE2_53238
ACATCCCAGAAAAGGGTGGAGAGGACCCATTGGAATCTCCCCTTGTGCGAGGCCCTAGCAAACCAAACAAGCCTTGCTTTGGCCCCTGTGACATCTC
ATCACCTTGTGGATGCCCTCGTACCCTCAGTCCAGGGTCGAGGGCTCCAGTTGGCCAGAGTGCAAGCACTCGTCTGCTCGCAACCTCAGAAGCTCCG
GTGCGGACGACGAAGACCCCCCCCCTGTCCAGCTTTCCGAGCCCATGGAGCTCCACAAAACTCACGACCCTACCAGGAACACCAGACCCCGGCACTC
TGCACCTGCAGGGGTCTGCCCGTCCTCTTGGTCGACTGAACGTTGTATTCGAGACTAGTTTCCTGGCCTGGCAGCTGCTGCTCGACAGAGTCCCTCT
GCCTATCGAGCCAGGAAGTACTTTATGACCCGCATGCCCTCTCTTCATGCCTGGATGCCGTCGTCTCCGTTAAGATCTTGGGTTTGGGCGAAACTCG
GGTTCCTTCCGTATTCTTATCTTCATACCTCTTTGTTCTTCGTGTCTTCCTTTTTCCATCATTGGATAATAACGCTCTCTTCTACTTCGCTGGATAA
CACCGCACACGACCGTATACCTTTCTTTGCCTCACTATGCTGTGATACTACCACGACTGCCTGCTACCTATCAAAACACGCCTGCCGCAAGGTCTGA
GGATTCATCACATCAATCGATTGTAATACGCCGCCTTCGGATGGCTCGAACCACTTGATATACTGGGTCATATGATAACGCATCTGTTCGACTGTGC
ACGAGACATCACAGCGCTCCGAGTACCACGATTCACCAGTGACATTGACGGCCCCTCTTCTTGATTTCGCATTTCGACGATCGATTGTTCAGCTCAT
CACTCGTACATCTCGGGGCGCACCGGGAAAAGCGACTATCGACGAGCCTCCCCAGGAGGGATACTTATTTCTCGACGACACCCTCTATCTTTCCAAG
GCTACCTTTGCGGAACATCCCTTTCCCACC

>lcs2_promotor_TRIRE2_73604
CCCAGAAAAGGAAGCCATCGTCGTCGAGCTGCTGCGGTCCATCAAGGTTCATACCTACTTAGGTACCTACATGCATGCCGAGAAATCCATTCTGGGA
TTGAAACCCCCCCTTGTTAGTGGTACAGTGTCGCTACTGTGGACCCGAAACGGCGGGTTGCTTCCCCGTCAGACTTGCTTAGCGGGCTACAGCGTCC
ACTAAGAACTCCCTTTCTTCAGGGTGCTTCCGCTGAGAAACCGGAGGACGAGGACCCGAGAAATACAGTGTCATATATCCAAGAACCCACTGTACGT
CCCTTCTTCCCTGCCAGGGGCACAAGTACCATCTCCATACAGCTCCAGGGGACGAGATACATCGCCCCCCTTGCTTATGCGTGGGGCCTTTTGTCTC
AGTTAATGGGCAGCAGCTTGTCTTCAGCTCGCAGCTCCATCCCTACCCTTCTGCCATCTTCCCAACCTCATCTCTCATCAGCAACATCACGGGCATA
TCATTCATCGCCTCGGGGGATCCAGGCATCAGCCGCTTTTGCCCTGGCAGCGAATCGGACCCGGCAGAGGCCACACGCTGCGTTTGGGGTGGGCGCC
TGATAGAAGGAGGCGGGTCGCATCATTGATCGCATCGCGTCGCCATCGCCATCGAATCGAGGCGCATCTTGCATCACAGCACATCGTATCGCATCGC
ATCGCATCTCACCGTTCCCGCCAGACTAGGTAGCTTGAGGCAAGGGGCTGGGGTAGCAGGGACGAATAGAGACGCGATACGAGAGCATCCAATTGCC
ACTACCAGTAAGTACACAGGCCCCATCATGGACGGGCATCAGCCGCAGCGTCACGGGCTGAGACGCGCGTCTTGTGATCCCTTTCATCTTGTCTCCT
TCTCCTTCTGCCTCTTCCAGCCTGCTGGTGTCTTGGCCTCCGTGCTAATTGATTGGCTCGCCAGGCAGAAGCTGTCGAACGCACACCGGCCACGGAA
CTGGCCCTATCCGAGACTCGACATCCATCTTAGTAGTAGTATCAATCGCC

>lcs3_promotor_TRIRE2_53776
CACGCTGATCTATGTAGCGGTCGTTGCGGGGACTGAGACGAACGGCAAGTTTTTGCAGCATAATCAGGTTGACCCGTAAGTTCATGTGATGCTTATT
GTTGAGGTTCTACGGATAGTACAGCTCAACTGAGTTTGACAGGGGTGCCGAGTTTCTCAACACTGCGGAAGGAGAAGACTTTATTAGCAAGCTCTGG
AAAGAGTCGGTTCAAGACGTTGCTGCTACTGATCCGGCTCTTGGCTCTTATGCTTGACAGGAGGATGCGGTGAGGAGGAGGCATGGTAACGAGAATA
TTATGCCTTAATCAGGTACAGTGTCATCAAGTATTGGTCTGAGAAGATGTTTGATCCCGAGCGACTCTGACCTACTATGATTGCAGTTTGCGTCTAT
GCCACATGTCGCTCATCATGTGCTCAACATTCAATGAGCCATGTTGCAATTGGTATCTTGCAATTATGTGAACGCTTAGTCTCGCATCAACCTATAC
TTTTGCTTTCCCGTGTTATATGGAGAGGAGGCCTCCCGAGTGCTTCTCAGTTTCAGTAAGTGAAGTTGAAGTCTATGGGTATCATTCGTGTGCTCAC
TCTCATGGGCATTCTCAAAAGCTTGCTTACAATGGGACGGTATCAAGTTGGAACACGGAAGCAAGGCGCAAGAGTCGCTTCTCGCTTGGAATGTAAG
TGGAGGAGTCGTCCAGCTATCATATGCGAAGTATCGGAATAACAATCGTTCAGGCCTAACCAGGCCAAGCAATATCACTTACTGCATCCATCGTAGT
CTATTAATCTGTGAGCCCTGTGAGCGTTGCATTTTCATTTACAGACAAGCATTCTCAGATCCTTCCACGGCGGCGGTTCAGCCTGGCGCAACACCTT
CAACTTGGCCCTCACTATTTAGGCAGTTTCCCCGACCCCGCATATAACAGCAACGCATACCTATATATAATATGGACTCTCTCACACCTTTCAAATT
TGATCATGACACCAAAGCAATCTCTCAGCA

>lcs4_promotor_TRIRE2_73618
CGCAGCCACTCTGCAAGCTTCTGCGCGTTGCTGACGCCTGAAGTGTGCTGCAACTTGGGAATTGCTTCACACAGGGATGGCCAATGACTCTCGATGT
CTCTGACGGCGCTCAGCGCCCATTGGCTCGCGGAGTCCGTGCTGATAAAGGACAAGATGCTGGCGAAGTAGGTCTCGTTCAGGGACATGGCCCCAGG
CCCGAACATGAGCAGAGTGCGTGTACTTGAGGCCGCCATGACTGTCTGCGCAAGCTGGCGAACGAGGTTGATGCGCTTCTGCTGTTGAAGTTGCCCT
GGAATCCCCCTTGTGTGGTAATGTTGAGTACCACAAGCCTTCAAGGTGAACGAAACGTGATGCCTTCAAGATGGTAGATGCATGCAGTCACCAAGAC
ACAGAATGTCGTGGACTGCACGCATGCTTTTAGCATCTTTGCGAGATTCGCCTCTCATCCTTCGTCCCGCCAGAAGCTGACGATGCTGACAGACAGA
TTGCCCGCCGCCGTCATTCAAACGTTTGCCTGATACTGCTGACATGGAGTTTCGAGTTTCTGCCCTCAGTGACGATCGCCAACATTGAACTCCGGAG
TTGAGATACTCCGATGCGGATCGCACTTGACGACTCCTTCGGACTCTGCCAAGGCGGACTTTTTCTGCTTCTTTTTCTCTCTCTTTTAATTGCTTAC
TGCCGAATTTCGCCGCTCTCCGACCGCGTCCGTCAGGAATTTTGTTTCTTCAAACGAATCCTGACGATAGTATGTCCGTATCACTCGCAAGCCGCTT
ACAGGATCTGCTGATCGAGTTATAGAGAAGGTTCGTCATGGCAAGACGCGTCTATGAAGCTGACGGCCCTCAAGCTGGGCTTCGAAGCAGTCGCATA
TGTAAAATACCTTGACCGGCTTCGTTTTCCCAGGCCTAAACTTGTCTCCGTCCCCGAACAAGTATTTCCAGACGCAAACACTCAGCCCCCCTTCAACC
TTCTTTATCGACCACCCTCAACGACGGAAG

>lcs5_promotor_TRIRE2_73621
GATTTCCAGCAGGCTCTACCTACAGTATTCAGCCGCTCGTTGTTCGGATGGTAGACTCCTTCCGATCTGAATCTAGAGGCTGGGAACTCTGTCCACC
CGCTTCTCCCCTCAGCCAGCATCTGCCAGAGCTTGTCCGTGTTGGTTGCATCTCCAGCAAACTTGGAGCTCATGCCAATGATGGCAATAGGCTCACT
CGCCCAGCGAGACTGGCTATCGGGGCCGAGACTTCCCATCTTCCGTCGTTGAGGGTGGTCGATAAAGAAGGTTGAAGGGGGCTGAGTGTTTGCGTCT
GGAAATACTTGTTCGGGGACGGAGACAAGTTTAGGCCTGGGAAAACGAAGCCGGTCAAGGTATTTTACATATGCGACTGCTTCGAAGCCCAGCTTGA
GGGCCGTCAGCTTCATAGACGCGTCTTGCCATGACGAACCTTCTCTATAACTCGATCAGCAGATCCTGTAAGCGGCTTGCGAGTGATACGGACATAC
TATCGTCAGGATTCGTTTGAAGAAACAAAATTCCTGACGGACGCGGTCGGAGAGCGGCGAAATTCGGCAGTAAGCAATTAAAAGAGAGAGAAAAAGA
AGCAGAAAAAGTCCGCCTTGGCAGAGTCCGAAGGAGTCGTCAAGTGCGATCCGCATCGGAGTATCTCAACTCCGGAGTTCAATGTTGGCGATCGTCA
CTGAGGGCAGAAACTCGAAACTCCATGTCAGCAGTATCAGGCAAACGTTTGAATGACGGCGGCGGGCAATCTGTCTGTCAGCATCGTCAGCTTCTGG
CGGGACGAAGGATGAGAGGCGAATCTCGCAAAGATGCTAAAAGCATGCGTGCAGTCCACGACATTCTGTGTCTTGGTGACTGCATGCATCTACCATC
TTGAAGGCATCACGTTTCGTTCACCTTGAAGGCTTGTGGTACTCAACATTACCACACAAGGGGGATTCCAGGGCAACTTCAACAGCAGAAGCGCATC
AACCTCGTTCGCCAGCTTGCGCAGACAGTC

>lcs6_promotor_TRIRE2_73623
CGGGTGCGAGCCTACCCTGGGGAGGACCCGTGGGATAACCCCTCGGCCAAGGCAATGGACTTTTTCGAACACAAGTTCAAGCACATGTCTTGCGGCG
TAACCCCTCGGCCAAGGCAATGGACTTTTTCGAACACAAGTTCAAGCACATGTCTTGCGGCG-
GAGTGACAATGGCGACGGATCATGCGCTGGAGCATTCGGAGACTTTGAGGGCTGTACAGCCTGTGAGTGATGAGCTGGTGCGCAAGTATATCCAGGC
GTGGAAGGATAGCGGCTTTTTGCGATGAGTCTGTATTGAAGGGTTATGTCTACTATATTGTTCAAATTGAATATCTTATCTTCTATACTCTACTTCT
TCAGGTGAAATTGACAACTATTCAATCATGAATATGAGTTACCAAGTGCTTTGTACATACGACATGAGCTATCAGGAAAGCGTCAAGTACCTAGGTA
TGCTTCAAACTTGTATACCTACCTAGGTAATATGAGCTTGCAATAATTCAAAAGTTTCTTTTTTAATTGCATTTGGAAGGTAGATTTACGCAAGTCT

TGTTATTCCAAGAACTTTAGGGTAGTCATTTGCTGGTGTGGTATTCTT[?]ATCGAGCTCTTTCTTTTACGTCTTCCCTCATGAATTTAGGATTAACCG
AAGAGTCTCATGCTTGTTGCGCTTACAATGATAATAGTCTGAGCAAATCGTTGCATTACAACCCTTACTGTGAAATCTCTGAGGAGCCATGGCGTTT
GTTTCATAGTTGAAGTCAGCAGTCGTACGACAACGTATGACAAGTTCG[?]GAAGGCAAATCATTTAGTTGTCAAGTCCTCGAGAAACTCCGTACTCCG
GCTTACAGCGTAATGGCCACCGATAATTACTCCGACAAACAGGATGACACACAGCATACAGAATCCTACAGTTGCCAAACTCATGGCAGGCAACAAC
AAACACGAAAGTCTCAAAGCGTATTATGCCTCATTTATAAGCTGTAAA[?]GCATGTATGTATACTAGACGTAGCACAAGCCTGATGCCAGATCCTTTC
TCTCACCTTTGTCAGATCAACCTGCACGTTTCTCACCCACTCTCTCGGGCAATAGTCCTACAGCCAGTAACAAG

>lcs7_promotor_TRIRE2_43701
TGCAATGAGTAGTCTTGCGCGATCAGGGGCGAAGCCTGGGCCGTGACGGGGGTTGGAGCGGTGACTGGGAGTTCGACAGAGGCACGACTGCGGATAG
GTCTAGGCTGGCGGGTTGGTGCTTTTGCCCTTTGTCTGCCGCGACCTC[?]TGGTGACGCTGCATCGTGTTGGCTGTAGCTGCACGCCCGGCCTCTTTT
CTGGCATCTCGCACAGACTGTACGCAGAACATGTGTAAGTGACTTTCTATTGTAGGACTTGGATGGATGGGAGATGGCTATAGACAACAAGAGCGAC
AACAAGGATTATAGACAGTTCCATTACCTGGGCGACCACCATCGCACTTCACCTTGCTCGCCCGGCAGGACTGGCAGGCGTTGGCAATCTTGAGTCG
CTCCCTGTACAGCGACAAGGGCGCCTGAGCCTGCACCTGAGCCTGGGCCTGAGTCTGAGCCTGAAGCCGTTGATCCTCGTCTTGACGGGCGGACATG
ACGCTTTACGTGAGCTGAGTCCAGGGCTGTCGTCGGCAAGTGATTCGGCAGCCGGCAGGCACAGACGAAAGATAAGAAGCGGCTCGTTCACCAGATC
CGAAGGCGGAATGCAAGAATGTCCGATGATGTCGGGGAAGAGAAGGCGATGCCTCGTCTATCTGGGAGGCGAGGGAAGAAGGGAAAGCAACTCCCGC
CTCGGCACAGCTACTCATTGGTCAGAGCGGACTTCGGAGTATAACCACACGGCTGACAAACCTGCATCGACCGTGATGGCGTTCTTTTGGTCCAATC
ATGGCTGAATGCGACGTTGGTTGTGTCGGCCCCTGCAGCCAATGAGAAGCTGGTCCGATCCTGGGGACGAGGGAGAGGCAAGCATATCGTATAAGTC
CAAGCTCAAGCTTGGCGGCAGGTACCGGTTGATGTGGTCGCTGTATATAAGTAAAAGAACTCGATTCCGCTTCGGAGTTTTCTCTGTCACCCTTCAT
GGACGAAGAGCTATTGAGCCGCATCTTTGCTTCGTTGTCCAGCGTGAA[?]TCTCCTACA

>lcs8_promotor_TRIRE2_102497
AAATATCGGAACTTACTATATGGGAAAGATTCATTAGCTTTGTGCATCCTTGGAGACATTGGAGTACTATAGACAACTTACCAGAGGACCCCAAACA
GCAGGGCCAATTGCAAAGCCCAACACAAAGACAGAGACACCGGTGATGAAGAGAGTGCTGCTGATGTCAAACTCGGTCATGATCTCTTCAGCCGACA
CGGAATACGCCGAAGACGTAAACGTGACAGCAAACACGGAAAAGGTGACGATGGATGTGAGGAACCATTTCCTGAACTGGCCCCAGTTCATGGGGTT
GCTCTTGTCGTCCTGCTGGAACTCGACGATAAAGGGATCTTCCACCGTGCCGGAGCCATTGTATGGGGAGGTCGTAACATTCTTCTCGGCGTCGACA
ACCTCGAGGGGCTCGACAGCCTCGACCGGCGTTATCGATCGGGAGTCTTGGGAGGCTTCTTTCGACATTGTAGGAGAATTCACGCTGGACAACGAAG
CAAAGATGCGGCTCAATAGCTCTTCGTCCATGAAGGGTGACAGAGAAAACTCCGAAGCGGAATCGAGTTCTTTTACTTATATACAGCGACCACATCA
ACCGGTACCTGCCGCCAAGCTTGAGCTTGGACTTATACGATATGCTTGCCTCTCCCTCGTCCCCAGGATCGGACCAGCTTCTCATTGGCTGCAGGGG
CCGACACAACCAACGTCGCATTCAGCCATGATTGGACCAAAAGAACGCCATCACGGTCGATGCAGGTTTGTCAGCCGTGTGGTTATACTCCGAAGTC
CGCTCTGACCAATGAGTAGCTGTGCCGAGGCGGGAGTTGCTTTCCCTTCTTCCCTCGCCTCCCAGATAGACGAGGCATCGCCTTCTCTTCCCCGACA
TCATCGGACATTCTTGCATTCCGCCTTCGGATCTGGTGAACGAGCCGC[?]TCTTATCTTTCGTCTGTGCC[?]TGCCGGCTGCCGAATCACTTGCCGACGA
CAGCCCTGGACTCAGCTCACGTAAAGCGTC

>lcs9_promotor_TRIRE2_73631
CTCAATCAGTTAGTCGACTTGGCAAGCAAATGCTTGGTGCAACCCTTTCGGCTCCCGCTGGGAGAACTAACCCCCATGTACTTGGACTATCAA
AAGGGACCGGCTGCAGGAATCTCTTGCCGCGCCTCGTAAGATCGGACCAGTGGCTGAAACTTCCAATGGCAGAGAACGACCTATCGGAGGGGT
CAAATCGTTTTGGCTCCCTTAGTAGACGAAGGAATGCATAACAATGGCCATGGCGTCAAACGGTGAGATCGGGGGTTGTCGGACTAAACATGC
CTCGGGCACCCTGGTCGGCTCGGTCCGATGCTTGTAGCAGATCCCTTCCCGCTTTATCCTCCGTATCCCTTCCCTACCTATCTCACAGTGGAA
CATAGTCTAATAAGATAAAGCCCGGGACAGAGCTCATGTGATACTCTTGACTCGCAGTTCCACGTGGTTTATGCAACTCTATTTAGACCTTTA
TAAAGCTGCGTGCTGCCCGAATCAATATTGAACCAGGCAGCACTTGAGACTCGATATCACGCCGCTGTTGGGGGTACAGTAGCATTGACAATA
CCTGGGTAGGCTTCGGTTGGCAAAGGGTCAACCCTGGAGCTGAGCAATCCTTCGATCCTTCGACTAGAAATCTGCCCATAGCTGAAGACATCC
GGTTCCCCTATTGGGCAATCCGACAGCCGAGACTCCTTCAATCAAGATGGCCTCATTGTTCGCTAGTCGCTCTTTATTGACAGCCGTAAGATG
GCTCGTCATTACGCCGACGGAACTTTTCCCGAAGCTGACACCACATACCAGAATCTACACAGCAAAAAACTCCGTACTCCGACAGAACCTCAT
CACCCCACGCCTCATTCGACCTCTGGTTCAATAGCAGCTTTTCTTTTTTCTTATCGATGTATGATAGTTTATATAAAAATGTGGAAAAAACAC
ACTTGCGTAGCGATAATGCAAAGACCAGCGATAACCTTGAAACGACGGGCCATTTGACGTTCTAGACGCC

>lcs10_promotor_TRIRE2_102499
GTACGGCTTA CCCCAAGCGA ATGAAGGACG AGCGACGGCA AGGGCCAGGC TCACAGAAGC AGCAAACGGT CGGCGTACGT
CCGACAGAGC GGCTGTGGTG TAGTTTTGTT TCACGACGCC GCTGATCCGC CCTGACGACA TACGTACATC AATAGTGTCC
AAGAACAGGA GTGAAGGTGC ATCCACAGTG CATGTAGAGT GCATACCAAG TTGACTACAG ACATGTTGGA TGGTGGCACA
GCTCTAGGCT GCTTAGGTAG CCGGCCGATG TGTAAGGTAG CGAGAGTGAA CTCGCGCCTC ATAAAGAGCA TCTCCAGGGG
GGGGGGGGGC TAATGACGGA TAGCTCAGTT TGCAACAAGT TTGA[?]ACAAG GCGAAAGAGA AGGACCAGAG CGTCGATTGG
ACAGTAGTGA TATGCAGGAT CGTGGACTCG ATAAGGGCCA CCCACCCATG TACTCACGCA AGCAATATGC AAGAGTCGTC
CTATTCAGCC GGGCTTATTG CTCGGCACAC ACACCTGCAG GATCGGCACG CGAGGCTCAT AAAGGCTCAA CGGCGTTAAG
ATGGTTGAAA GACGCCTGCA ACTGGTCCAA GGGTCTGAAG GCTCCATGGC CGCATGTGAT TATGCAAGCC AGAGCCCTCC
GAAGTCTGTT GCCCAGGTAC GCTGTATAGG AATAGCAGGT ATCTGTGCGG GTTTGGAGCC TCGTATGTTA CGAGTATACT
CCCCACAACA TAAGGATTCT CACATCTGGA CCTTGTCTAT GCATGGCTTG AGTGGTCCCG CTAGCAGAAA AGCCTGGAAG
TAGGATGAGA CTGCTTGCTA GGCTACAAGG GGGGAGGCAA TTCGGATGTT ATCATATAAC AGGTCTTCCG CTACCAAACC
ATCAAGCCAT CTGTGTTGGC GAGACTTCCA TCTGATCCTG ATAGACATCG CCATACATCT TGCCTCATTA TCCTACCGTT
TCAACAGGCT AGGTGTGGTA TCGTAAGGTA GCCTGTCATC

### Literature

Aidemark, M., et al., (2010) BMC Plant Biol 10: 274.
Atanasova, L., et al., (2013) Eukaryot Cell 12: 1499-1508.
Atoui, A., et al., (2010) Fungal Genet Biol 47: 962-972.
Baker, S.E., et al., (2012) Microbiology 158: 147-154.
Bazafkan, H., et al., (2015) Mol Microbiol 96: 1103-1118.
Bergmann, S., et al., (2007) Nat Chem Biol 3: 213-217.
Blumenthal, C.Z., (2004) Regul Toxicol Pharmacol 39: 214-228.
Brakhage, A.A. & V. Schroeckh, (2011) Fungal Genet Biol 48: 15-22.
Bruckner, H., et al., (1984) Experientia 40: 1189-1197.
Campbell, C.D. & J.C. Vederas, (2010) Biopolymers 93: 755-763.
Castellanos, F., et al., (2010) Fungal Genet Biol 47: 468-476.
Charlile, M.J., (1965) Ann Rev Plant Physiol 16: 175 - 201.
Fringeli, U.P. & M. Fringeli, (1979) Proc Natl Acad Sci U S A 76: 3852-3856.
Gacek, A. & J. Strauss, (2012) Appl Microbiol Biotechnol 95: 1389-1404.
Galagan, J.E., et al., (2003) Nature 422: 859-868.
Guangtao, Z., et al., (2009) J Biotechnol 139: 146-151.
Jacoby, E., et al., (2006) ChemMedChem 1: 761-782.
Keller, N.P., et al., (2005) Nat Rev Microbiol 3: 937-947.
Knuf, C. & J. Nielsen, (2012) Biotechnol J 7: 1147-1155.
Kozlovskii, A.G., et al., (2001) Prikl Biokhim Mikrobiol 37: 292-296.
Kredics, L., et al., (2013) Chem Biodivers 10: 744-771.
Kumar, R., et al., (2008) J Ind Microbiol Biotechnol 35: 377-391.
Leitgeb, B., et al., (2007) Chem Biodivers 4: 1027-1051.
Liu, R. et al., (2015), Cell Discovery 1:15007
Martinez, D., et al., (2008) Nat Biotechnol 26: 553-560.
Mukherjee, P.K., et al., (2012) Microbiology 158: 35-45.
Paloheimo, M., et al., (2016) Production of industrial enzymes in Trichoderma reesei. In: Gene Expression Systems in Fungi: Advancements and Applications. M. Schmoll & C. Dattenböck (eds). Heidelberg: Springer International, pp. 23-58.
Paterson, R., et al., (1990) Mycological research 94: 538-542. Rippa, S., et al., (2007) Chem Biodivers 4: 1360-1373.
Rodriguez-Romero, J., et al., (2010) Annu Rev Microbiol 64: 585-610.
Sanchez, J.F., et al., (2010) Mol Biosyst 6: 587-593. Scazzocchio, C., (2000) Curr Opin Microbiol 3: 126-131.
Schmoll, M., (2011) Adv Appl Microbiol 76: 27-78.
Schmoll, M., (2013) Sexual development in Trichoderma - scrutinizing the aspired phenomenon. In: Trichoderma: Biology and Applications. P.K. Mukherjee, B.A. Horwitz, U.S. Singh, M. Mukherjee & M. Schmoll (eds). UK: CAB International, pp. 67-86.
Schmoll, M., et al., (2016) Gene Expression Systems in Fungi: Advancements and Applications (ISBN 978-3-319-27949-7), p. 499. Springer International, Heidelberg.
Schmoll, M., et al., (2010) Fungal Genet Biol 47: 909-916.
Schmoll, M., et al., (2014) Genomics analysis of biocontrol species and industrial enzyme producers from the genus Trichoderma. In: Fungal Genomics. M. Nowrousian (ed). Heidelberg, Germany: Springer, pp. 233-265.
Schmoll, M., et al., (2012) BMC Genomics 13: 127.
Schuster, A., et al., (2012) Biotechnol Biofuels 5: 1.
Schuster, A., et al., (2011) Appl Environ Microbiol 77: 4553-4563.
Serwe, A., et al., (2009) Invest New Drugs 27: 491-502.
Shirota, O., et al., (1997) J Chem Soc Perkin Trans 1: 2961-2964.
Siddiquee, S., (2014) Recent advancements on the role and analysis of volatile compounds (VOCs) from Trichoderma. In: Biotechnology and Biology of Trichoderma. V.K. Gupta, M. Schmoll, A. Herrera-Estrella, R.S. Upadhyay, I. Druzhinina & M.G. Tuohy (eds). Waltham, MA, USA: Elsevier, pp. 139-175.
Solfrizzo, M., et al., (1994) Nat Toxins 2: 360-365.
Steiger, M.G., et al., (2011) Appl Environ Microbiol 77: 114-121.
Tisch, D., et al., (2011) BMC Genomics 12: 613.
Tisch, D. & M. Schmoll, (2010) Appl Microbiol Biotechnol 85: 1259-1277.
Tisch, D. & M. Schmoll, (2013) BMC Genomics 14: 657.
van den Berg, M.A. & K. Maruthachalam, (2015). In. Heidelberg: Springer International, pp. 318.
Vitikainen, M., et al., (2010) BMC Genomics 11: 441.
Wichard, J.D., et al., (2011) PLoS One 6: e16811.
Yu, J.H. & N. Keller, (2005) Annu Rev Phytopathol 43: 437-458.
Zabala, A.O., et al., (2012) Chem Biol 19: 1049-1059.
Zadra, I., et al., (2000) Appl Environ Microbiol 66: 4810-4816.
Zhang, R. & X. Xie, (2012) Acta Pharmacol Sin 33: 372-384.
Znameroski, E.A. & N.L. Glass, (2013) Biotechnol Biofuels 6: 6.

## Claims

1. A fungal strain of the phylum Ascomycota, preferably *Trichoderma, Neurospora, Penicillium* or *Aspergillus,* wherein one or more of the lcs genes or is either
(a) overexpressed; or
(b) underexpressed or in which
(c) the function of the encoded protein is altered or abolished by genetic manipulation
in said fungal strain, compared to an unmodified wildtype fungal strain of the same genus cultured under controlled light conditions.

2. A fungal strain of the phylum Ascomycota, preferably *Trichoderma, Neurospora, Penicillium* or *Aspergillus,* wherein one or more of the lcs genes is de-activated or absent in said fungal strain, compared to an unmodified wildtype fungal strain of the same genus cultured in a normal light-darkness cycle, with the proviso that said fungal strain is not the *T. reesei* knock-out mutant Δtr_102497 as disclosed in Schuster *et al.* (Biotechnology for Biofuels, Volume 5(1), 2012, 1-10).

3. A fungal strain according to claim 1 or 2, wherein said fungal strain belongs to the species *Trichoderma reesei.*

4. A fungal strain according to claim 2, wherein the gene encoding an LCS protein is knocked out in said fungal strain by genetic manipulation of said fungal strain.

5. A fungal strain according to claim 1 (a), wherein the gene encoding an LCS protein is upregulated in said fungal strain by genetic manipulation of said fungal strain, wherein said genetic manipulation is preferably achieved by promoters selected from the group consisting of *tef1, cbh1, gpd1, hfb2* and other strong promotors routinely used for heterologous expression in *T. reesei* being operably linked to the gene encoding an LCS protein.

6. Use of a fungal strain according to claims 1(b), 2and/or 5 for production of heterologous proteins.

7. Use of a fungal strain according to claim 1(a)for the production of secondary metabolites.

8. Use of a fungal strain according to claim 1(a) for the production of cellulases or modulation of their production levels.

9. Method for cultivating a fungal strain under toxin reduced conditions or for avoiding contamination of fungal products with further metabolites as regulated by LCS proteins.

10. Method according to claim 9, wherein said fungal strain is further defined as belonging to the species of *Trichoderma reesei.*

11. Use of the method according to claim 9 or 10 for the production of Alamethicin-free strain products.

12. Use according to claim 11, wherein the strain product is further defined as being a heterologous protein.

13. Use according to claim 12, wherein the heterologous protein is further defined as being a protein of human origin.

14. Use of an LCS gene product, especially LCS1 as a target for drug development in plant pathogenic fungi such as *Fusarium* spp., *Botrytis, Sclerotinia, Pyrenophora* etc and in human pathogenic fungi such as *Paracoccidioides brasiliensis, Histoplasma capsulatum* or *Aspergillus fumigatus* etc.

15. A fungal strain of the phylum Ascomycota, preferably *Trichoderma, Neurospora* or *Aspergillus,* wherein one or more of the LCS genes individually, or in combination is either
(a) overexpressed, especially in the case of LCS1 and LCS6 to improve cellulase gene expression, and LCS1, LCS2, LCS4, LCS5, LCS6, LCS8 and LCS9 to improve Trichodimerol and/or Dihydrotrichotetronin production in the dark, or
(b) *underexpressed,* especially in the case of LCS8 to improve cellulase gene expression and of LCS1, LCS3, LCS6, LCS8, and LCS9 to prevent Paracelsin and/or Alamethicine production in the dark and LCS7 to improve Trichodimerol and Dihydrotrichotetronin production in light,
in said fungal strain, compared to an unmodified wildtype fungal strain of the same genus.


SEQUENCE LISTING

<110>  AIT AUSTRIAN INSTITUTE OF TECHNOLOGY GMBH

<120>  Fungal cell of the phylum Ascomycota

<130>  R 65468

<160>  76

<170>  PatentIn version 3.5

<210>  1
<211>  49
<212>  DNA
<213>  Trichoderma reesei

<400>  1
gtaacgccag ggttttccca gtcacgacgg taaagttcgg accttgtag                    49


<210>  2
<211>  49
<212>  DNA
<213>  Trichoderma reesei

<400>  2
cgacgatatc agcttccata ttccgactaa cagacgaaag ataagaagc                    49


<210>  3
<211>  49
<212>  DNA
<213>  Trichoderma reesei

<400>  3
agaaaagcac aaagaagagg ctccaactac tgcggtttac atttcactc                    49


<210>  4
<211>  49
<212>  DNA
<213>  Trichoderma reesei

<400>  4
gcggataaca atttcacaca ggaaacagct tctttcaggg agttcaagc                    49


<210>  5
<211>  49
<212>  DNA
<213>  Trichoderma reesei

<400>  5
gtaacgccag ggttttccca gtcacgacgg tagaaggcat cgtaggtcc                    49


<210>  6
<211>  49
<212>  DNA
<213>  Trichoderma reesei

<400>  6

atccacttaa cgttactgaa atctccaacg tggttatact ccgaagtcc 49

<210> 7
<211> 49
<212> DNA
<213> Trichoderma reesei

<400> 7
ctccttcaat atcatcttct gtctccgacg ccctctctca tacatcagc 49

<210> 8
<211> 49
<212> DNA
<213> Trichoderma reesei

<400> 8
gcggataaca atttcacaca ggaaacagcg tgttcagctc ttgaagtgg 49

<210> 9
<211> 49
<212> DNA
<213> Trichoderma reesei

<400> 9
gtaacgccag ggttttccca gtcacgacgc attctacaga ggcactagg 49

<210> 10
<211> 49
<212> DNA
<213> Trichoderma reesei

<400> 10
atccacttaa cgttactgaa atctccaacg agatgtacga gtgatgagc 49

<210> 11
<211> 49
<212> DNA
<213> Trichoderma reesei

<400> 11
ctccttcaat atcatcttct gtctccgacc tatccatctg ccatagacc 49

<210> 12
<211> 49
<212> DNA
<213> Trichoderma reesei

<400> 12
gcggataaca atttcacaca ggaaacagcc tccatgccta atacctacc 49

<210> 13
<211> 49
<212> DNA
<213> Trichoderma reesei

<400> 13

gtaacgccag ggttttccca gtcacgacga cactcacctt ctcatctcc 49

<210> 14
<211> 49
<212> DNA
<213> Trichoderma reesei

<400> 14
atccacttaa cgttactgaa atctccaacg actcctccac ttacattcc 49

<210> 15
<211> 49
<212> DNA
<213> Trichoderma reesei

<400> 15
ctccttcaat atcatcttct gtctccgacc tatagtgcta gaccccagc 49

<210> 16
<211> 49
<212> DNA
<213> Trichoderma reesei

<400> 16
gcggataaca atttcacaca ggaaacagca tctagccgtg atactctgg 49

<210> 17
<211> 49
<212> DNA
<213> Trichoderma reesei

<400> 17
gtaacgccag ggttttccca gtcacgacga gtacccgact aatgactgg 49

<210> 18
<211> 49
<212> DNA
<213> Trichoderma reesei

<400> 18
atccacttaa cgttactgaa atctccaacc tctattcgtc cctgctacc 49

<210> 19
<211> 49
<212> DNA
<213> Trichoderma reesei

<400> 19
ctccttcaat atcatcttct gtctccgact cgtgagagag agagagagg 49

<210> 20
<211> 49
<212> DNA
<213> Trichoderma reesei

<400> 20

gcggataaca atttcacaca ggaaacagct agcaccaaga gtaagctcc 49

<210> 21
<211> 49
<212> DNA
<213> Trichoderma reesei

<400> 21
gtaacgccag ggttttccca gtcacgacga tctggcttgg atactcacc 49

<210> 22
<211> 49
<212> DNA
<213> Trichoderma reesei

<400> 22
atccacttaa cgttactgaa atctccaacg atcagcagat cctgtaagc 49

<210> 23
<211> 49
<212> DNA
<213> Trichoderma reesei

<400> 23
ctccttcaat atcatcttct gtctccgacc acacactcac atcttcagc 49

<210> 24
<211> 49
<212> DNA
<213> Trichoderma reesei

<400> 24
gcggataaca atttcacaca ggaaacagcg ctgtactatc cgtagaacc 49

<210> 25
<211> 49
<212> DNA
<213> Trichoderma reesei

<400> 25
gtaacgccag ggttttccca gtcacgacgg tcgaagaagt gagagatgc 49

<210> 26
<211> 49
<212> DNA
<213> Trichoderma reesei

<400> 26
atccacttaa cgttactgaa atctccaacg agttgagata ctccgatgc 49

<210> 27
<211> 49
<212> DNA
<213> Trichoderma reesei

<400> 27

ctccttcaat atcatcttct gtctccgacg tagtcatttg ctggtgtgg                49

<210>  28
<211>  49
<212>  DNA
<213>  Trichoderma reesei

<400>  28
gcggataaca atttcacaca ggaaacagcg tcttcactca ccaacatcc                49

<210>  29
<211>  49
<212>  DNA
<213>  Trichoderma reesei

<400>  29
gtaacgccag ggttttccca gtcacgacgc tcttctctat cagcagtgg                49

<210>  30
<211>  49
<212>  DNA
<213>  Trichoderma reesei

<400>  30
ctccttcaat atcatcttct gtctccgacg cgtagcatta ctccatagc                49

<210>  31
<211>  49
<212>  DNA
<213>  Trichoderma reesei

<400>  31
gcggataaca atttcacaca ggaaacagct acggtcgctc actattagg                49

<210>  32
<211>  49
<212>  DNA
<213>  Trichoderma reesei

<400>  32
gcggataaca atttcacaca ggaaacagct acggtcgctc actattagg                49

<210>  33
<211>  49
<212>  DNA
<213>  Trichoderma reesei

<400>  33
gtaacgccag ggttttccca gtcacgacga cctgaaaggt aaggtgacg                49

<210>  34
<211>  49
<212>  DNA
<213>  Trichoderma reesei

<400>  34

atccacttaa cgttactgaa atctccaaca gctgctattg aaccagagg 49

<210> 35
<211> 49
<212> DNA
<213> Trichoderma reesei

<400> 35
ctccttcaat atcatcttct gtctccgact ccgtttacac tgagacagc 49

<210> 36
<211> 49
<212> DNA
<213> Trichoderma reesei

<400> 36
gcggataaca atttcacaca ggaaacagcc atacgtccga agagtcacc 49

<210> 37
<211> 49
<212> DNA
<213> Trichoderma reesei

<400> 37
gtaacgccag ggttttccca gtcacgacgg acgcagtaga ttgtcatgg 49

<210> 38
<211> 49
<212> DNA
<213> Trichoderma reesei

<400> 38
atccacttaa cgttactgaa atctccaaca gctactcatt ggtcagagc 49

<210> 39
<211> 49
<212> DNA
<213> Trichoderma reesei

<400> 39
ctccttcaat atcatcttct gtctccgacc tggttaaggt aagcagagc 49

<210> 40
<211> 49
<212> DNA
<213> Trichoderma reesei

<400> 40
gcggataaca atttcacaca ggaaacagcg gccttaccat tgtgtctgg 49

<210> 41
<211> 22
<212> DNA
<213> Trichoderma reesei

<400> 41

gtcagcacca tttggcttcg gc                                                22

<210>  42
<211>  22
<212>  DNA
<213>  Trichoderma reesei

<400>  42
tccctgacaa ctgcgccata gc                                                22

<210>  43
<211>  22
<212>  DNA
<213>  Trichoderma reesei

<400>  43
agcgtcatca ccatgtctcc gc                                                22

<210>  44
<211>  22
<212>  DNA
<213>  Trichoderma reesei

<400>  44
cgcttcactg acgacgaggg ag                                                22

<210>  45
<211>  22
<212>  DNA
<213>  Trichoderma reesei

<400>  45
gcgcactgga gtatctgcac ga                                                22

<210>  46
<211>  22
<212>  DNA
<213>  Trichoderma reesei

<400>  46
cggtagccct gtcagcatct cg                                                22

<210>  47
<211>  22
<212>  DNA
<213>  Trichoderma reesei

<400>  47
aagacctccc agccgaccga ta                                                22

<210>  48
<211>  22
<212>  DNA
<213>  Trichoderma reesei

<400>  48

aacgtctcgt actcgcacca gc                                                22

<210>  49
<211>  22
<212>  DNA
<213>  Trichoderma reesei

<400>  49
atgacgagga tagcaaggcg gc                                                22

<210>  50
<211>  22
<212>  DNA
<213>  Trichoderma reesei

<400>  50
aatggacaac tctgctcccg cc                                                22

<210>  51
<211>  22
<212>  DNA
<213>  Trichoderma reesei

<400>  51
gcaacctcgt cgatttggct gc                                                22

<210>  52
<211>  22
<212>  DNA
<213>  Trichoderma reesei

<400>  52
aagtgtctcg agaaggacgc gc                                                22

<210>  53
<211>  22
<212>  DNA
<213>  Trichoderma reesei

<400>  53
gacgaggatg acgtgaagcg ct                                                22

<210>  54
<211>  22
<212>  DNA
<213>  Trichoderma reesei

<400>  54
gccaagacca gcgagtcttc ca                                                22

<210>  55
<211>  22
<212>  DNA
<213>  Trichoderma reesei

<400>  55

aacggctccg aaatcactgc ga                                                22

<210>  56
<211>  22
<212>  DNA
<213>  Trichoderma reesei

<400>  56
ccccggcatc agatatcgca gg                                                22

<210>  57
<211>  490
<212>  PRT
<213>  Trichoderma reesei

<400>  57

Met Ala Asn Ile Thr Gly Ile Asn Leu Cys Pro Ala Pro Phe Tyr Asp
1               5                   10                  15

Ala Ser Leu Phe Gly Asp Ser Gly Gly Tyr Ile Ser Gly Arg Leu Cys
            20                  25                  30

Gln Thr Ile Ala Gly Ser Val Gln Cys Cys Leu Pro Cys Pro Met Thr
        35                  40                  45

Asp Trp Val Tyr Pro Asp Ser Phe Thr Thr Ile Gly Thr Val Ala Asn
    50                  55                  60

Trp Ile Ala Thr Ala Ser Val Ile Cys Cys Val Phe Leu Leu Ala Ser
65                  70                  75                  80

Trp Ala Ala Leu Pro Val Asp Lys Thr Asn Arg His Tyr Leu Ser Ile
                85                  90                  95

Cys Phe Thr Ile Gly Ile Leu Ile Met Asn Leu Gly Phe Val Ile Pro
            100                 105                 110

Leu Ala Ala Glu Pro Asp Gln Cys Phe Asp Ala Ile Thr Pro Asn Asp
        115                 120                 125

Met His Thr Leu Ser Val Cys Gly Ala Ser Gly Ala Met Leu Ile Leu
    130                 135                 140

Gly Gly Trp Cys Gly Val Met Trp Ala Phe Leu Arg Ser Leu Ser Leu
145                 150                 155                 160

His Leu Gln Ile Cys Trp Gln Val Leu Val Gly Arg Asn Phe Met Ile
                165                 170                 175

Phe Ala Gln Ala Ala Gly Trp Gly Ile Pro Ala Ile Gly Ile Ile Leu
            180                 185                 190

Ala Leu Val Phe Ser Gly Val Ser Phe Arg Phe Gly Ala Thr Cys His
        195                 200                 205

Ile Asn His Lys Asn Ser Leu Ala Asp Leu Trp Ile Pro Leu Leu Ile
    210                 215                 220

Phe Ala Ser Ile Thr Val Val Thr Thr Phe Val Thr Phe Gly Tyr Cys
225                 230                 235                 240

Ile Lys Val Tyr Leu Ala Ser Leu Asn Asp Asn Ser Ala Ser Thr Glu
                245                 250                 255

Gly Ser Asn Leu Pro Thr Tyr Thr Asn Ser Val Ile Thr Met Ser Pro
            260                 265                 270

Arg Gln Ala Tyr Arg Arg Val Arg Arg Val Ile Ala Leu Gln Trp Arg
        275                 280                 285

Gly Ile Ala Ile Val Leu Ile Ile Ile Ala Asp Val Ile Phe Phe Ser
    290                 295                 300

Val Val Phe Val Phe Gln Asp Asn Ile Val Gln Ser Ile Ala Asp Asp
305                 310                 315                 320

Pro Lys Val Ala Gln Asn Trp Ile Val Cys Leu Ile Gly Ala Gln Gly
                325                 330                 335

Asp Lys Thr Lys Cys Leu Asn Glu Ala Gly Ser Leu Val Val Ser Glu
            340                 345                 350

Ala Thr Val Ser Ala Val Leu Val Leu Leu Ser Leu Asn Gly Ile Trp
        355                 360                 365

Ile Leu Phe Leu Leu Gly Arg Trp Thr Met Val Thr Gly Trp Ile Asp
    370                 375                 380

Leu Val Lys Ser Pro Phe Asn Ser Arg Lys Lys Glu Phe Val Ser Val
385                 390                 395                 400

Asp Ala Arg Tyr Gln Ser Gln Lys Asp Thr Arg Ser Tyr Glu Met Leu
                405                 410                 415

Ser Arg Asp Thr Ser Ala Ala Val Thr Pro Leu Asp Pro Val Lys Ser
            420                 425                 430

Ser Ala Gly Pro Gln Ala Pro Asp Tyr Phe Gly Asp Thr Ala Arg Tyr
        435                 440                 445

His Ala Pro Ala Arg Ser Tyr Ser Asn Pro Arg Pro Pro His Ser Ser
    450                 455                 460

Leu Pro Glu Trp Asp Ala Gln Lys Thr Tyr Ala Ala Pro Gln Gly Arg
465                 470                 475                 480

Asp Met Asn Pro Leu Gly Met Asn Arg Ile
                485                 490

<210>  58
<211>  599
<212>  PRT
<213>  Trichoderma reesei

<400>  58

Met Pro Ala Ser Gln Pro Ile Ser Lys Pro Ala Arg Gly Asp Thr Ser
1               5                   10                  15

Asp Tyr Arg Pro Val His Gln Asp Asp Arg Gln Asp Asp Ser Gln Asp
            20                  25                  30

Gly Thr Glu Glu Glu Gly Val Val Ser Pro Ala Tyr Leu Pro Pro Arg
        35                  40                  45

Arg Ser Arg Ala Asp Arg Arg Tyr Arg Thr Thr Ile Leu Val Leu Val
    50                  55                  60

Val Ala Leu Val Ile Leu Leu Ala Thr Asn Leu Tyr Leu Ser Leu Pro
65                  70                  75                  80

Tyr Leu Ile Tyr Gly Ser Gly Pro Gly Pro Gly Ser Cys Pro Pro Pro
                85                  90                  95

Ser Lys Val Pro Gln Tyr Phe Gln Thr Ser Pro Glu Leu Trp Ala Gly
            100                 105                 110

Pro Thr Ala Thr Gly Arg Ala Ala Phe Met Ala Gln Thr Arg Thr Phe
        115                 120                 125

Asp Pro Thr Ala Thr Phe Val Pro Asn Glu Pro Leu Gln Thr Ala Ile
    130                 135                 140

Pro Val Glu Gly Met Lys Pro Gly Asn Asp Thr Ile Phe Lys Met Met
145                 150                 155                 160

Gly Tyr Leu Ser Pro Tyr Phe Pro Ser Pro Gly Phe Gly Val Asp Glu
                165                 170                 175

Tyr Pro Leu Pro Ser Gly Ala Glu Ile Val Gln Val Gln Met Leu Ser
            180                 185                 190

Arg His Gly Ala Arg Tyr Pro Thr Ser Gly Val Gly Val Thr Lys Phe
        195                 200                 205

Ala Gln Arg Ile Ala Asn Ala Ser Asp Lys Phe Tyr Pro Lys Gly Pro
    210                 215                 220

Leu Ser Phe Leu Lys Asp Trp Glu Tyr Asn Leu Gly Ser Glu Ile Leu
225                 230                 235                 240

Val Pro Lys Gly Arg Glu Glu Leu Tyr Asn Ser Gly Val Leu His Ser
                245                 250                 255

Tyr Met Tyr Gly Arg Leu Tyr Asn Pro Lys Ser Lys Ile Ile Val Arg
            260                 265                 270

Thr Thr Thr Gln Asp Arg Met Leu Lys Ser Ala Glu Asn Trp Met Ala
        275                 280                 285

Gly Phe Phe Gly Leu Glu Trp Thr Asn Asn Ala Thr Ile Glu Val Ile
    290                 295                 300

Ile Glu Ser His Gly Phe Asn Asn Ser Leu Ala Gly Ala Leu Asn Cys
305                 310                 315                 320

Pro Asn Ser Asn Ser Lys His Ser Ala Asp Glu Ala Lys Glu Lys Trp
                325                 330                 335

Ile Asn Thr Tyr Leu Lys Asp Ala Val Pro Arg Phe Gln Ala Met Thr
            340                 345                 350

Glu Gly Phe Glu Trp Thr Val Asp Asp Val Tyr Asn Ala Gln Thr Leu
        355                 360                 365

Cys Pro Tyr Glu Thr Val Ala Tyr Gly Tyr Ser Met Phe Cys Asp Leu
    370                 375                 380

Phe Thr Tyr Glu Glu Trp Gln Gly Phe Gly Tyr Ser Ile Asp Val Trp
385                 390                 395                 400

Phe Ser Gly Gly Asn Ala Phe His Ser Pro Val Gly Arg Ala Thr Gly
                405                 410                 415

Ile Gly Tyr Gln Gln Glu Val Leu Ala Arg Leu Lys Asn His Thr Leu
        420                 425                 430

Gly Tyr Ser Gly Ser Gln Ile Asn Val Thr Leu Asp Asn Asn Thr Glu
        435                 440                 445

Thr Phe Pro Leu Asn Gln Ser Leu Tyr Phe Asp Phe Ser His Asp Thr
    450                 455                 460

Asn Ile Ile Ser Val Leu Thr Ala Phe Gly Leu Arg Gln Phe Ala Glu
465                 470                 475                 480

Asp Leu Pro Ala Asp Arg Tyr Pro Gly Ala His Asn Phe Thr Val Ser
                485                 490                 495

His Leu Thr Pro Phe Gly Ala Arg Leu Asp Ile Glu Ile Ile Lys Thr
            500                 505                 510

Pro Lys Pro Leu Ser Pro Asn Arg Asp Gly Tyr Leu Arg Gly Glu Glu
        515                 520                 525

Gln Lys Tyr Val His Phe Ile Leu Asn Gln Arg Thr Ile Pro Leu Gly
    530                 535                 540

Leu Ser Phe Pro Glu Cys Asp Ala Ser Arg Lys Asp Gly Trp Cys Glu
545                 550                 555                 560

Tyr Glu Thr Phe Val Lys Ile Gln Glu Gly Met Ser Ala Lys Ala Ser
                565                 570                 575

Tyr Asp Tyr Ala Cys Phe Gly Asp Tyr Pro Lys Glu Pro Tyr Gly Val
            580                 585                 590

Ile Thr Asp Gly Val Pro Lys
        595

<210>  59
<211>  449
<212>  PRT
<213>  Trichoderma reesei

<400>  59

Met Ala Lys Arg Glu Arg Ser Pro Thr Gly Tyr Gly Pro Gln Pro Glu
1               5                   10                  15

Glu Ala Ile Pro Ser Pro Ser Arg Pro Gly Ile Leu Thr Val Val Leu
            20                  25                  30

His Glu Gly Thr Gly Leu Ser Ile Pro Gly Ser Tyr Lys Glu Asp Gln
        35                  40                  45

Arg Pro Asp Gln Gln Gly Leu Ser Lys Arg Asn Thr Pro Tyr Ala Val
    50                  55                  60

Leu Asp Tyr Asp Arg Ser Gln Gln Gln Ala Asp Ser Asp Glu Gly Thr
65                  70                  75                  80

Ala Glu Asp Pro Val Trp Val Ser Asp Ile Val Pro Trp Arg Leu Asp
                85                  90                  95

Glu Val Thr Gly Glu Tyr Arg Asp Ala Asn Trp Lys Phe Asp Val Cys
            100                 105                 110

Arg Pro Ala Glu Leu Ala Ile His Leu Tyr Leu Arg Asp Pro His Ala
        115                 120                 125

Ser Pro Cys Val Arg Ser Gln Asp Ala Cys Ile Gly Val Ala Arg Ile
    130                 135                 140

Pro Ile Asp Val Ala Gln Ala Ser Ala Ser Glu Glu Thr Pro Ser Gln
145                 150                 155                 160

Trp Val Asp Val Glu Gly Gly Thr Gly Arg Leu Arg Val Ser Ile Lys
                165                 170                 175

Tyr Asp Asp Thr Lys Asp Lys Thr Leu Glu Ala Ala Asp Phe Ile Glu
            180                 185                 190

Gln Ser Lys Ile Gln Lys Gly Asn Ser Gly Tyr Thr Ala Arg Val Ile
        195                 200                 205

Lys Lys Asp Thr Arg Gln Arg Tyr Thr Thr Arg Thr Ile Pro Ala Val
    210                 215                 220

Arg Arg Pro Gln Asn Ala Asp His Pro Phe Ile Ala Pro Leu Thr Leu
225                 230                 235                 240

Val Phe Gln Ser Gln Glu Gly Leu His Leu Leu Ser Pro Thr Met Cys
                245                 250                 255

Gly Gly Tyr Leu Phe Gln His Leu Gln Asn Gln Arg Val Phe Ser Leu
            260                 265                 270

Lys Arg Ala Arg Ile Tyr Ala Ala Glu Ile Leu Cys Ala Leu Glu Tyr

275                 280                 285

Leu His Glu Ser Arg Gly Ile Tyr Ser Trp Leu Lys Pro Arg Asn Val
    290                 295                 300

Leu Leu Asp Ser Leu Gly His Val Val Leu Cys Gly Ser Gly Leu Tyr
305                 310                 315                 320

Asp Pro Gly Val Glu Asp Asp Gly Arg Gly Gly Tyr Gly Leu Pro Glu
                325                 330                 335

Tyr Pro Ala Pro Glu Ile Leu Arg Asp Gln Asn Arg Ser Gly Ala Ala
            340                 345                 350

Asp Trp Trp Thr Leu Gly Ile Phe Ile Tyr Glu Met Leu Thr Gly Leu
        355                 360                 365

Pro Pro Phe Phe His Glu Asn Ala Asp Glu Ile Arg Arg Gln Ile Leu
    370                 375                 380

Ser Ser Asp Ser Ile Gln Phe Pro Asp Asp Leu Pro Pro Asp Ala Arg
385                 390                 395                 400

Asp Val Ile Ala Lys Leu Leu Asp Arg Lys Pro Asp Phe Arg Leu Gly
                405                 410                 415

Ala Arg Gly Gly Ala Ser Glu Val Lys Glu His Pro Phe Phe Ala Asp
            420                 425                 430

Ile Asp Cys Gly Lys Leu Lys Gln Arg Glu Tyr Thr Pro Ser Phe Lys
        435                 440                 445

Pro

<210>  60
<211>  2567
<212>  PRT
<213>  Trichoderma reesei

<400>  60

Met Gly Ser Leu Gly Pro Asp Ser Gln Ser Arg Trp Ala Ser Glu Pro
1               5                   10                  15

Ile Ala Ile Ile Gly Met Ser Ser Lys Phe Ala Gly Asp Ala Thr Asn
            20                  25                  30

Thr Asp Lys Leu Trp Gln Met Leu Ala Glu Gly Arg Ser Gly Trp Thr

        35                  40                  45

Glu Phe Pro Ala Ser Arg Phe Arg Ser Glu Gly Val Tyr His Pro Asn
    50                  55                  60

Asn Glu Arg Leu Asn Thr Thr His Val Lys Gly Ala His Phe Leu Gln
65                  70                  75                  80

Glu Asp Val Gly Leu Phe Asp Ala Ala Phe Phe Ser Tyr Ser Ser Glu
                85                  90                  95

Thr Ala Ser Ser Leu Asp Pro Gln Tyr Arg Leu Gln Leu Glu Ser Ala
            100                 105                 110

Tyr Glu Ala Leu Glu Asn Ala Gly Ile Pro Leu Thr Gln Ile Ala Gly
        115                 120                 125

Ser Asn Thr Ser Val Phe Thr Gly Val Phe Val His Asp Tyr Arg Asp
    130                 135                 140

Ala Leu Leu Arg Asp Ala Asp Asn Leu Pro Arg Leu Met Ala Thr Gly
145                 150                 155                 160

Thr Gly Val Pro Met Met Ser Asn Arg Ile Ser His Phe Phe Asp Leu
                165                 170                 175

Arg Gly Ala Ser Met Thr Ile Glu Thr Ala Cys Ser Ser Gly Met Val
            180                 185                 190

Ala Thr His Gln Gly Ile Gln Ser Leu Arg Thr Gly Glu Ala Asp Met
        195                 200                 205

Ser Ile Val Gly Gly Ala Asn Leu Thr Leu Asn Pro Asp Met Phe Lys
    210                 215                 220

Ala Leu Gly Ser Ala Gly Phe Leu Ser Ala Asp Gly Lys Ser Tyr Ala
225                 230                 235                 240

Phe Asp Ser Arg Ala Ser Gly Tyr Gly Arg Gly Glu Gly Val Ala Thr
                245                 250                 255

Ile Val Met Lys Arg Leu Ser Asp Ala Leu Ala Ala Gly Asp Pro Ile
            260                 265                 270

Arg Ala Val Ile Arg Ser Ser Leu Leu Asn Gln Asp Gly Lys Thr Glu
        275                 280                 285

Thr Ile Thr Thr Pro Ser Leu Glu Ala Gln Ile Asp Leu Ile Arg Gln
    290                 295                 300

Cys Tyr Ala Arg Ala Gly Leu Asp Pro Arg Asp Thr Gln Tyr Phe Glu
305                 310                 315                 320

Ala His Gly Thr Gly Thr Gln Ala Gly Asp Thr Val Glu Ala Arg Ala
                325                 330                 335

Ile Ala Thr Val Phe Ser His Asn Gln Asp Pro Leu Leu Ile Gly Ser
            340                 345                 350

Ile Lys Thr Asn Ile Gly His Thr Glu Ala Ala Ser Gly Leu Ala Ser
        355                 360                 365

Ile Ile Lys Thr Ala Leu Ala Leu Glu Lys Gly Val Ile Pro Ala Ser
    370                 375                 380

Ile Asn Phe Glu Lys Pro Asn Pro Lys Leu Ser Leu Glu Asp Trp His
385                 390                 395                 400

Leu Lys Leu Val Arg Gln Leu Gln Glu Trp Pro Ala Ala Ser Thr Arg
                405                 410                 415

Arg Ala Ser Ile Asn Asn Phe Gly Tyr Gly Gly Ala Asn Ala His Ile
            420                 425                 430

Ile Leu Glu Asp Gly Ala Ser Trp Thr Pro Ser Pro Val Glu Gly Thr
        435                 440                 445

Ser Ser Lys Glu Pro Ile Asp Thr Gly Ser Arg Val Leu Val Leu Ser
    450                 455                 460

Gly Lys Asp Glu Gln Ala Cys Arg Thr Met Ile Ser Asn Leu Ala Glu
465                 470                 475                 480

Tyr Leu Gln Arg Val Ala Ser Ala Glu Asp Glu Pro Ser Arg Leu Leu
                485                 490                 495

Asp Ser Leu Ala Tyr Thr Leu Gly Gln Arg Arg Thr Arg Phe Pro Trp
            500                 505                 510

Val Ala Ala His Pro Val Pro Val Thr Glu Gly Ile Gly Ala Val Val
        515                 520                 525

Asn Thr Leu Gln Ser Pro Lys Phe Lys Pro Tyr Arg Ser Ser Arg Arg
    530                 535                 540

Pro Arg Ile Gly Met Val Phe Thr Gly Gln Gly Ala Gln Trp Trp Ala
545                 550                 555                 560

Met Gly Arg Glu Leu Arg Asp Ala Tyr Pro Val Tyr Lys Ala Ser Leu
                565                 570                 575

Asp Glu Ala Asp Ala Tyr Ile Arg Gln Phe Gly Ala Asp Trp Ser Leu
            580                 585                 590

Val Glu Glu Leu Ser Arg Asp Ala Ala Ser Ser Arg Ile Asn Glu Ser
        595                 600                 605

Gly Leu Ser Thr Pro Ile Cys Val Ala Val Gln Ile Ser Leu Val Arg
    610                 615                 620

Leu Leu Glu Ser Trp Gly Val Val Pro Ala Ala Val Thr Ser His Ser
625                 630                 635                 640

Ser Gly Glu Ile Ala Ala Ala Tyr Thr Val Gly Ala Leu Ser Tyr Lys
                645                 650                 655

Asp Ala Met Ala Tyr Ala Tyr His Arg Ala Val Leu Ala Ala Asp Thr
            660                 665                 670

Ser Leu Arg Gly Pro Val Lys Gly Gly Met Leu Ala Ile Gly Leu Gly
        675                 680                 685

Arg Glu Asp Thr Glu Ala Tyr Leu Lys Arg Leu Thr Thr Gly Gly Lys
    690                 695                 700

Ala Met Val Ala Cys Val Asn Ser Pro Ser Ser Thr Thr Val Ser Gly
705                 710                 715                 720

Asp Leu Ser Ala Val Gln Glu Leu Glu Glu Leu Ala Asn Ala Asp Gly
                725                 730                 735

Val Phe Ala Arg Leu Leu Lys Val Glu Thr Ala Trp His Ser His His
            740                 745                 750

Met Thr Ala Ile Ala Asn Val Tyr Val Glu Ala Leu Asp Asn Ile Lys
        755                 760                 765

Arg Lys Asn Ser Arg Asn Glu Ser Ser Ile Ala Tyr Ser Ser Pro Val
    770                 775                 780

Thr Gly Gly Arg Val Ala Asn Ile Glu Glu Val Ala Arg Pro Glu His
785                 790                 795                 800

Trp Val Lys Ser Leu Val Gln Pro Val Gln Phe Val Asp Ala Phe Thr
                805                 810                 815

Asp Met Val Leu Gly Asn Pro Asn Gly Ser Ala Asn Val Asp Val Val
            820                 825                 830

Val Glu Val Gly Pro His Thr Ala Leu Gly Gly Pro Ile Gln Gln Ile
        835                 840                 845

Leu Ala Leu Pro Ala Phe Lys Gly Leu Gln Ile Pro Tyr Tyr Gly Cys
    850                 855                 860

Leu Val Arg Lys Ala Asp Ala Lys Asp Thr Met Gln Ala Leu Ala Ala
865                 870                 875                 880

Asn Leu Leu Gln Gln Gly Tyr Pro Leu Asp Met Asp Ala Val Asn Phe
                885                 890                 895

Pro His Gly Arg Gly Ser Arg Val Arg Val Leu Thr Gly Leu Pro Ser
            900                 905                 910

Tyr Pro Trp Asn His Gln Val Lys His Trp Val Glu Pro Arg Phe Asn
        915                 920                 925

Arg Ala Leu Arg Glu Arg Ser Val Pro Pro His His Leu Leu Gly Ser
    930                 935                 940

Leu Val Glu Gly Thr Asn Leu Glu Glu Pro Thr Trp Arg His Thr Leu
945                 950                 955                 960

Arg Ile Ser Glu Ser Pro Trp Thr Arg Asp His Ala Ile Gln Ser Asn
                965                 970                 975

Val Val Tyr Pro Ala Ala Gly Tyr Ile Cys Leu Ala Ile Glu Ala Thr
            980                 985                 990

Lys Gln Leu His Ala Leu Asn His  Thr Lys Ala Gly Ala  Lys Glu Val
        995                 1000                 1005

Ser Gly  Tyr Arg Leu Arg Asp  Val Asp Phe Leu Gln  Ala Leu Met
    1010                 1015                 1020

Ile Pro  Asp Thr Ser Asp Gly  Ile Glu Ile Gln Thr  Ser Leu Arg
    1025                 1030                 1035

Pro Val  Asn Asp Lys Asp Val  Ala Ile Gln Gly Trp  Lys His Phe

1040                1045                1050

Glu Val  Trp Thr Val Thr Gly  Asp Asn Arg Trp Thr  Gln His Ala
    1055                1060                1065

Lys Gly  Leu Ile Ser Ile Glu  Phe Glu Ala Ser Ala  Gln Val Tyr
    1070                1075                1080

Glu Pro  Lys Leu Gly Glu Phe  Thr Ile Lys Gly Tyr  Lys Arg Gln
    1085                1090                1095

Ile Pro  Pro Ala Gln Leu Phe  Ala Asn Leu Lys Ala  Leu Gly Ile
    1100                1105                1110

Gly His  Gly Pro Val Phe Gln  Asn Met Ser His Ile  Val Gln Ser
    1115                1120                1125

Gly Phe  Asp Arg Arg Ser Val  Val Leu Thr Thr Val  Pro Asp Thr
    1130                1135                1140

Ser Val  Pro Asn Asp Leu Pro  Arg Glu His Val Leu  His Pro Val
    1145                1150                1155

Thr Leu  Asp Ser Phe Ile Thr  Ser Pro Tyr Ser Ala  Val Pro Gly
    1160                1165                1170

Ala Ala  Gly Arg Glu Thr Ala  Ala Lys Val Pro Arg  Ser Val Lys
    1175                1180                1185

Ser Phe  Trp Val Ser Ser Asn  Ile Ser His Ser Pro  Glu His Val
    1190                1195                1200

Phe Lys  Ala His Ser His Ile  Ile Arg Asp Asp Lys  His Gly Met
    1205                1210                1215

Glu Ala  Asp Val Ile Val Ala  Asn Asp Gly Val Asp  Asp Asn Asn
    1220                1225                1230

Val Leu  Leu Glu Met Lys Gly  Phe Ser Tyr Gln Ser  Leu Gly Arg
    1235                1240                1245

Ser Val  Ser Leu Gln His Thr  Glu Pro Trp Glu Ser  Gln Leu Cys
    1250                1255                1260

Ser Ser  Ile His Trp Arg Pro  Asp Ile Ser Ile Lys  Leu Pro Ala
    1265                1270                1275

Thr Val Ser Leu Val Lys Gln Glu Leu Ser Ser Asn Ser Asn Ser
    1280                1285                1290

Ala Glu Ala Gly Gly Val Glu Ile Ser Ser Leu Cys Leu Tyr Phe
    1295                1300                1305

Ile Gln Lys Ala Leu Ala Ser Leu Ser Asp Ser Asp Phe Thr Asn
    1310                1315                1320

Gly Ser His Tyr Ser Lys Tyr Tyr Ala Trp Met Lys Ser Ala Ala
    1325                1330                1335

Gln Gln Ala Ala Ile Thr Asp Ile Asp Glu Glu His Ile Asp Gln
    1340                1345                1350

Ile Ala Lys Ala Gln Ala Asp Gly Glu Met Ile Arg Leu Leu Gly
    1355                1360                1365

Lys Gln Leu Val Ser Ile Leu Arg Gly Gln Ser Thr Pro Thr Glu
    1370                1375                1380

Ile Met Glu Gln Asp Lys Asn Leu Leu Ser Arg Phe Tyr Ser Glu
    1385                1390                1395

Thr Pro Arg Ala Lys Arg Thr Ser Ser Gln Leu Ser Gly Leu Leu
    1400                1405                1410

Arg His Leu Val His Lys Asn Pro Arg Ala Arg Ile Leu Glu Ile
    1415                1420                1425

Gly Ala Ser Thr Gly Gly Ile Thr Gly Ser Ala Leu Gly Val Leu
    1430                1435                1440

Asp Thr Ala Thr Ser Gly Gly Pro His Ala Ser Leu Tyr His Tyr
    1445                1450                1455

Thr Asp Leu Ser Asp Arg Asn Phe Asp Ala Ala Arg Glu Gly Phe
    1460                1465                1470

Ala Ala Trp Ser Asp Ile Leu Ala Phe Asp Val Leu Asp Ile Glu
    1475                1480                1485

Arg Asp Pro Ala Asp Gln Gly Phe Thr Val Gly Ser Tyr Asp Val
    1490                1495                1500

Val Ile Ala Ser His Ala Phe Ser Ser Thr Ser Ser Ala Ile Ala
    1505                1510                1515

Gly Val Leu Glu Asn Val Arg Ser Leu Leu Lys Pro Gly Gly Thr
    1520                1525                1530

Leu Leu Phe Thr Glu Asp Phe Lys Pro Ser Ile Asp Val Gln Phe
    1535                1540                1545

Val Lys Gly Leu Phe Pro Ser Trp Trp Ser Ser Glu Arg Phe Ser
    1550                1555                1560

Glu Gln His Val Glu Pro Ser Pro Leu Leu Ser Val Pro Leu Trp
    1565                1570                1575

Asp Arg Ser Leu Arg His Ala Gly Phe Thr Gly Ile Asp Ile Glu
    1580                1585                1590

Leu Arg Asp Ser Asp Asp Val Asp Ala Ser Val Ser Ala Thr Ile
    1595                1600                1605

Met Ser Thr Leu Pro Pro His Pro Ala Gly Gln Ser Asp Ile Asp
    1610                1615                1620

Ala Gly Lys Val Val Ile Val Thr Ser Glu Gln Ala Gly Ile Pro
    1625                1630                1635

Pro Ser Glu Trp Leu Lys Ala Leu Gln His Ser Ile Ala Ser Tyr
    1640                1645                1650

Ser Lys Ala Val Asn Gly Ala Glu Gly Lys Val Leu Pro Ser Val
    1655                1660                1665

Gln Ser Ile Glu Ser Ala Ala Ala Thr Ala Ala Trp Tyr Ala Asp
    1670                1675                1680

Lys Ile Cys Ile Phe Ile Gly Glu Ile Asn Glu Pro Ile Leu Tyr
    1685                1690                1695

Asn Leu Asp Ala Ala Ser Leu Glu Gly Ile Lys Ala Met Ser Thr
    1700                1705                1710

Gly Cys Lys Gly Leu Leu Trp Val Thr Arg Gly Gly Ala Val Asp
    1715                1720                1725

Cys Glu Arg Pro Glu Val Ser Leu Ala Thr Gly Phe Val Arg Thr
    1730                1735                1740

Leu Arg Asn Glu Tyr Val Gly Arg Lys Phe Ile Thr Leu Asp Leu
    1745                1750                1755

Ser Pro Lys Gly Ser Leu Trp Gln Glu Ser Gly His Glu Ala Ile
    1760                1765                1770

Ala Gln Val Leu Gln Asn Ala Phe Gly Gln Ser Leu Pro Gly His
    1775                1780                1785

Ser Ser Gly Pro Asp Lys Gly Pro Val Glu Leu Glu Tyr Ala Glu
    1790                1795                1800

Arg Asp Gly Val Ile Leu Ile Pro Arg Val Tyr His Asp Val Ala
    1805                1810                1815

Lys Asp Asn Ala Ile Thr Pro Lys Thr Leu Glu Ser Glu Glu Asp
    1820                1825                1830

Thr Gln Gly Ile Thr Thr Val Glu Pro Phe Tyr Gln Gln His Arg
    1835                1840                1845

Pro Leu Cys Phe Leu Pro Glu Leu Leu Val Phe Gly Asp Asp Ala
    1850                1855                1860

Ser Ala Ala Val Tyr Arg Asp Thr Leu Pro Pro Arg Leu Val Glu
    1865                1870                1875

Ile Met Pro Arg Ala Tyr Gly Ala Gly Leu Asn Pro Ala Asp Arg
    1880                1885                1890

Thr Ile Thr Gly Gln Glu Cys Ser Gly Ile Ile Thr Arg Val Gly
    1895                1900                1905

Ile Glu Ala Ser Lys His Gly Tyr Ser Val Gly Asp Arg Val Val
    1910                1915                1920

Cys Leu Leu Gln Gln Ser Ser Phe Thr Ser Arg Ala Val Val Asp
    1925                1930                1935

Trp Thr Ser Val Val Gln Met Pro Ser Arg Leu Ser Phe Gln Gln
    1940                1945                1950

Ala Ala Ser Leu Pro Ala Ala Phe Leu Val Ala Tyr Phe Ser Leu
    1955                1960                1965

Val Glu Thr Ala Arg Leu Lys Thr Ser Gln Ser Val Leu Ile His
    1970                1975                1980

Asn Ala Ala Gly Ser Ile Gly Gln Ala Ala Ile Met Val Ala Lys

1985                1990                1995

His Ile Gly Ala Thr Val Phe Thr Thr Val Ala Ser Pro Lys Gln
    2000                2005                2010

Arg Asp Leu Leu Thr Arg Glu His Gly Ile Pro Ser His Gln Ile
    2015                2020                2025

Phe Asp Ser Asn Asn Ala Ser Phe Gly Thr Ala Val Thr Ala Ala
    2030                2035                2040

Thr Asn Gly Arg Gly Val Asp Val Val Leu Asn Ser Leu Thr Gly
    2045                2050                2055

Pro Leu Leu Gln Thr Ser Phe Asn Leu Val Ala Pro Leu Gly His
    2060                2065                2070

Phe Ile Glu Val Gly Lys Tyr Asp Ser Leu Ala Asn Ser Asn Leu
    2075                2080                2085

Glu Met Leu Pro Phe Thr Arg Gly Val Ser Phe Ser Ala Val Asp
    2090                2095                2100

Val Pro Ser Leu Leu Gln His Arg Gly Ser Asp Val His Arg Cys
    2105                2110                2115

Leu Glu Glu Val Met Arg Leu Phe Glu Leu Glu Ala Leu Ala Pro
    2120                2125                2130

Val Ser Pro Val Ile Glu His Asn Ile Gly Asp Ile Ala Gln Val
    2135                2140                2145

Ser Arg Leu Ile Gln Thr Glu Glu Glu Thr Gly Lys Arg Val Leu
    2150                2155                2160

Ser Val Ala Gln Asp Glu Met Val Ser Val Leu Pro His Thr Ala
    2165                2170                2175

Pro Ala Ala Thr Leu Ser Pro Asp Ala Ser Tyr Leu Ile Val Gly
    2180                2185                2190

Gly Asn Gly Gly Leu Gly Gln Ala Val Ala His Trp Met Val Ser
    2195                2200                2205

Arg Gly Ala Lys Asn Leu Val Leu Leu Ser Arg Ser Ala Gly Gln
    2210                2215                2220

Ser Pro Lys Met Ala Val Leu Ala Glu Glu Leu Arg Asp Ala Gly
    2225                2230                2235

Cys His Arg Val Leu Pro Val Ser Cys Asp Val Ala Lys Glu Asp
    2240                2245                2250

Asp Leu Ala Arg Ala Met Asp Thr Cys Ala Arg Glu Gly Leu Pro
    2255                2260                2265

Pro Ile Arg Gly Val Val His Ala Ala Phe Val Leu His Asp Ser
    2270                2275                2280

Phe Val Glu Asn Met Thr Leu Asp Asp Tyr Lys Tyr Thr Ile Gln
    2285                2290                2295

Ser Lys Val Ser Gly Ala Trp Asn Leu His Asn Gln Phe Asn Leu
    2300                2305                2310

Pro Gly Asp Leu Asp Phe Phe Val Leu Phe Ser Ser Ile Asn Gly
    2315                2320                2325

Ile Leu Gly Tyr Ala Ser Gln Ala Ala Tyr Ser Ala Ala Gly Ala
    2330                2335                2340

Tyr Glu Asp Ala Leu Ala His Trp Arg Val Lys His Lys Gly Leu
    2345                2350                2355

Pro Ala Val Ser Ile Asp Leu Ser Leu Val Asp Gly Val Gly Tyr
    2360                2365                2370

Val Ala Glu Ala Ser Ala Ala Glu Ala Met Arg Lys Ser Leu Ile
    2375                2380                2385

Lys Ala Gly Arg Arg Val Ile Asn Glu Glu Gln Val Leu Ala Ser
    2390                2395                2400

Leu Glu Leu Ala Ile Val Ser Pro Tyr Asp Pro Gln Phe Ile Leu
    2405                2410                2415

Gly Gly Ile Asn Ser Gly Pro Gly Pro His Trp Asp Val Asp Gly
    2420                2425                2430

Asp Leu Gly Arg Asp Met Arg Leu Leu Ala Leu Lys Tyr Arg Gln
    2435                2440                2445

Pro Ala Ala Ala Asp Gly His Asp Asp Glu Asp Ser Lys Ala Ala
    2450                2455                2460

Asn Gly Gly Asp Ser Leu Ser Ala Lys Ile Ala Ser Ala Ser Ser
    2465                2470                2475

Arg Asp Glu Ala Ile Ser Val Val Gly Ser Ala Val Ala Ala Met
    2480                2485                2490

Leu Ala Asp Met Phe Leu Val Ser Val Glu Glu Val Asp Leu Asn
    2495                2500                2505

Asp Ser Pro Ser Gln Gln Gly Ile Asp Ser Leu Val Ala Val Glu
    2510                2515                2520

Val Arg Asn Met Leu Phe Ser Gln Ala Gly Ala Glu Leu Ser Ile
    2525                2530                2535

Phe Asn Ile Met Gln Ser Pro Ser Leu Ala Gln Leu Val Ala Asn
    2540                2545                2550

Val Val Asp Arg Ser Thr Phe Ala Lys Phe Ala Lys Ser Ser
    2555                2560                2565

<210> 61
<211> 2633
<212> PRT
<213> Trichoderma reesei

<400> 61

Met Ala Ala Ser Ser Thr Arg Thr Leu Leu Met Phe Gly Pro Gly Ala
1               5                   10                  15

Met Ser Leu Asn Glu Thr Tyr Phe Ala Ser Ile Leu Ser Phe Ile Ser
            20                  25                  30

Thr Asp Ser Ala Ser Gln Trp Ala Leu Ser Ala Val Arg Asp Ile Glu
        35                  40                  45

Ser His Trp Pro Ser Leu Cys Glu Ala Ile Pro Lys Leu Gln His Thr
    50                  55                  60

Ser Gly Val Ser Asn Ala Gln Lys Leu Ala Glu Trp Leu Arg Thr Gly
65                  70                  75                  80

Thr Leu Ala Pro Gly Ser Thr Ile Ala Ser Leu Pro Asn Ala Ile Leu
                85                  90                  95

Gly Pro Leu Val Val Ile Ala Gln Leu Val Glu Tyr Leu Arg His Val
            100                 105                 110

Asp Ser Leu Ser Glu Ser Gly Leu Arg Gly Gly Glu Gly Phe Gln Val
        115                 120                 125

Pro Ser Ala Pro Asp Ala Glu Thr Val Gly Cys Cys Leu Gly Thr Phe
    130                 135                 140

Ser Ala Leu Val Val Ser Ser Ser Ser Ser Trp Ala Gln Phe Cys His
145                 150                 155                 160

Asn Ala Ser Ala Val Val Arg Ile Val Phe Val Leu Gly Ala Leu Ser
                165                 170                 175

Asp Ala Gln Asp Ala Thr Asp Ala Ser Gly Pro Ser Val Ser Leu Ile
            180                 185                 190

Ala Phe Trp Arg Gly Gly Gln Ser Leu Ser Asp Leu Lys Lys Ala Leu
        195                 200                 205

Glu Lys Phe Pro Glu Ala Tyr Ile Ser Val Leu Tyr Asp Glu Asn Arg
    210                 215                 220

Val Thr Val Thr Thr Ser Thr Arg Thr Val Ala Ala Leu Lys Asn His
225                 230                 235                 240

Leu Gln Thr Val Gly Ile Thr Thr Asn Glu Thr Glu Phe His Gly Arg
                245                 250                 255

Phe His Ala Ala Gln Leu Tyr Gln Thr Glu Leu Glu Ala Phe Leu Ala
            260                 265                 270

Tyr Cys Arg Arg Phe Pro Thr Phe Gln Leu Pro Asp Ser Ser Cys Ile
        275                 280                 285

Val Val Pro Thr Arg Val Asn Ser Glu Asn Val Val Thr Ser Gln Glu
    290                 295                 300

Ser Leu Leu Glu Ile Ser Cys Arg Ala Phe Leu Val Ser Gln Phe Asp
305                 310                 315                 320

Trp Ile Lys Thr Phe Arg Ala Ala Val Ser Ser Thr Leu Gln Asn Arg
                325                 330                 335

Ala Ser Arg Ile Ile Glu Phe Gly Pro Glu Arg Cys Val Pro Pro Thr
            340                 345                 350

Leu Leu Arg Arg Leu Asn Ser Gln Val Thr His Phe Asp Phe Glu Glu
        355                 360                 365

Ser Ile Lys Arg Ala Lys Ala Ser Leu Ser His Asp Gln Glu Leu Pro
    370                 375                 380

Ala Gly Val Ala Glu Asn Asp Ile Ala Val Ile Gly Met Ala Cys Lys
385                 390                 395                 400

Val Ala Gly Ala Asp Asp Val Asn Glu Tyr Trp Glu Leu Leu Leu Gln
                405                 410                 415

Gly Lys Ser Gln His Arg Glu Leu Ile Pro Asn Asp Arg Phe Val Met
            420                 425                 430

Glu Thr Pro Phe Arg Pro Phe Glu Ala Gly Asp Asp Lys Lys Lys Trp
        435                 440                 445

Phe Gly Asn Phe Ile Gly Asp His Asp Ala Phe Asp Tyr Lys Phe Phe
    450                 455                 460

Lys Lys Ser Pro Arg Glu Ala Leu His Met Asp Pro Gln Gln Arg Leu
465                 470                 475                 480

Met Leu Gln Ala Ala Tyr Gln Ala Val Ala Gln Ser Gly Tyr Tyr Asn
                485                 490                 495

Ala Asn Leu Asn Val His Gly Pro Thr Lys Val Gly Cys Tyr Ile Gly
            500                 505                 510

Val Val Ala Asn Asp Tyr Glu Asn Asn Ile Ser His Thr Thr Pro Ile
        515                 520                 525

Ala Phe Ser Ala Thr Gly Ala Leu Arg Ser Tyr Ile Ala Gly Lys Val
    530                 535                 540

Ser His Phe Phe Gly Trp Thr Gly Pro Ala Met Thr Val Asp Thr Ala
545                 550                 555                 560

Cys Ser Ala Ser Thr Val Ala Leu Asp Leu Ala Cys Lys Ala Ile Leu
                565                 570                 575

Ser Gly Glu Cys Ser Ala Ala Leu Val Gly Gly Thr Asn Phe Phe Ser
            580                 585                 590

Thr Pro Met Phe Phe Gln Asn Leu Ala Ala Gly Ser Phe Leu Ser Thr
        595                 600                 605

Thr Gly Gln Cys Lys Pro Phe Asp Ala Lys Ala Asp Gly Tyr Cys Arg

610                 615                 620

Gly Glu Ala Val Gly Thr Val Phe Leu Lys Lys Leu Ser Gln Ala Ile
625                 630                 635                 640

Ala Asp Gly Asp Gln Val Leu Gly Val Ile Ser Ala Thr Ala Ile Asn
                645                 650                 655

Gln Asn Arg Asn Glu Thr Pro Ile Phe Val Pro Asn Ser Pro Ser Leu
            660                 665                 670

Thr Asn Val Phe Arg Thr Ala Ile Glu Lys Ser Gly Leu Asp Ala Lys
        675                 680                 685

Asp Ile Ser Val Val Glu Ala His Gly Thr Gly Thr Pro Val Gly Asp
    690                 695                 700

Pro Val Glu Tyr Asp Ser Ile Arg Gln Val Phe Gly Gly Ala Val Arg
705                 710                 715                 720

Ala Gly Gln Asp Ala Leu Gln Val Gly Ser Val Lys Gly Leu Val Gly
                725                 730                 735

His Thr Glu Gly Ala Ser Gly Val Val Ala Leu Val Lys Ile Leu Leu
            740                 745                 750

Met Leu Gln Arg Gly Gln Ile Pro Pro Gln Ala Ser Phe Glu Thr Ile
        755                 760                 765

Asn Pro Ser Ile Lys Tyr Ser Pro Ser Asp Asn Leu Glu Ile Thr Lys
    770                 775                 780

Thr Pro Leu Pro Trp Asn Gln Glu Phe Lys Ala Ala Leu Ile Asn Asn
785                 790                 795                 800

Tyr Gly Ala Ala Gly Ser Asn Ala Ser Val Ile Ile Lys Gln Gly Pro
                805                 810                 815

Ala Gln Leu Leu Arg Arg Leu Pro Pro Val Val Asp Ser Glu Thr Glu
            820                 825                 830

Ala Leu Leu Ser Lys Val Gly Ala Asp Asp Ala Gln Lys Ala Pro Phe
        835                 840                 845

Phe Ile Ser Gly Leu Asp Glu Lys Ala Ile Leu Ala Tyr Ala Gln Lys
    850                 855                 860

Leu Arg Gln Phe Ile Tyr Ser His Ser Asn Leu Asp Ile Gln Asp Leu
865                 870                 875                 880

Ala Phe Ser Val Asn Arg Gln Ser Asn Trp Ser Leu Gly Arg Gly Ala
                885                 890                 895

Val Phe Ser Ala Gly Ser Ile Ala Glu Leu Asp Glu Lys Leu Ala Ser
            900                 905                 910

Ile Glu Thr Phe Pro Val Pro Ser Ser Gln Pro Pro Val Ile Leu Cys
        915                 920                 925

Phe Gly Gly Gln Val Ser Thr Phe Val Gly Leu Asp Tyr Gln Leu Phe
    930                 935                 940

Ala Lys Ser Ala Ile Leu Arg Arg His Leu Asp Gln Cys Asp Ala Ala
945                 950                 955                 960

Cys Lys Ser Ile Gly Ala Gly Ser Ile Tyr Pro Arg Ile Phe Gln Ser
                965                 970                 975

Asp Pro Ile Asp Asp Pro Ser Val Leu Gln Pro Leu Leu Phe Ser Leu
            980                 985                 990

Gln Tyr Ser Cys Ala Met Ser Trp  Ile Asp Cys Gly Ile  Val Pro Ala
        995                 1000                 1005

Lys Leu  Val Gly His Ser Phe  Gly Glu Leu Thr Ala  Leu Cys Ile
    1010                 1015                 1020

Ser Gly  Val Val Ser Leu Gln  Asp Gly Leu Lys Leu  Val Tyr Gly
    1025                 1030                 1035

Arg Ser  Lys Ile Ile Lys Glu  Ser Trp Gly Ala Glu  Arg Gly Ala
    1040                 1045                 1050

Met Leu  Ala Val Glu Ala Asp  Leu Glu Glu Leu Glu  Ser Leu Leu
    1055                 1060                 1065

Thr Thr  Val Asn Ser Ser Leu  Gln Glu Gly Arg Ala  Thr Ile Ala
    1070                 1075                 1080

Cys Phe  Asn Gly Pro Arg Ser  Phe Thr Val Ala Gly  Ser Ser Ala
    1085                 1090                 1095

Ala Ile  Asp Ala Val Gln Gln  Ala Ile Ser Asn Thr  Gln Pro Val
    1100                 1105                 1110

Leu Lys His Lys Arg Leu Asn Val Thr Asn Ala Phe His Ser Val
    1115                1120                1125

Leu Val Glu Arg Leu Lys Pro Asp Leu Glu Ala Leu Gly Arg Gln
    1130                1135                1140

Leu Thr Phe Ala Leu Pro Gln Ile Pro Leu Glu Arg Ala Thr Arg
    1145                1150                1155

Ser Arg Glu Asp His Gly Leu Ser Pro Ser Tyr Val Ala Asn His
    1160                1165                1170

Met Arg Glu Pro Val Tyr Phe His His Ala Val Glu Arg Ile Ala
    1175                1180                1185

Lys Glu Tyr Pro Glu Ala Ile Trp Leu Glu Ala Gly Ser Asn Ser
    1190                1195                1200

Thr Ile Thr Thr Met Ala Ser Lys Ala Leu Gly Leu Pro Lys Ala
    1205                1210                1215

Ser Thr Phe Gln Pro Val Asn Val Thr Asn Asn Ser Lys Ala Ser
    1220                1225                1230

Ser Gln Leu Ser Asp Val Thr Met Asn Leu Trp Lys Ala Gly Leu
    1235                1240                1245

Arg Ile Thr Phe Trp Pro His Ser Arg Ala Gln Thr Tyr Glu Tyr
    1250                1255                1260

Lys His Ile Ile Leu Pro Pro Tyr Gln Phe Glu Lys His Arg Gln
    1265                1270                1275

Trp Leu Glu Phe Lys Pro Pro Gln Ala Leu Gln Val Val Val Gln
    1280                1285                1290

Thr Asp Ser Ser Thr Asp Ala Arg Asn Gly Thr Thr Glu Pro Gln
    1295                1300                1305

Pro Val Gly Leu Tyr Thr Leu Leu Asp Arg Gly Gln Asp Lys Tyr
    1310                1315                1320

Arg Phe Arg Val Asn Thr Ala Ala Gly Gln Phe Val Asp Ala Met
    1325                1330                1335

Ser Asp His Ala Ile Gly Lys Ala Gln Thr Leu Pro Ala Met Phe
    1340                1345                1350

Gly Val Asp Leu Ala Ile Glu Ala Leu Leu Ser Ile His Pro Glu
    1355                1360                1365

Leu Gly Asp Thr Ser Arg Phe Asp Pro Gln Ile Tyr Asn Val Val
    1370                1375                1380

Asn Gln Glu Tyr Ile His Asp Ser Ser Arg Ala Leu Phe Val Leu
    1385                1390                1395

Phe Glu Arg Leu Gly Gln Asp Glu Asn Ser Trp Ala Phe Glu Leu
    1400                1405                1410

Thr Ser Lys Gly Lys Asp Gly Ala Glu Ser Leu His Met Ser Gly
    1415                1420                1425

Gln Leu His Phe Gln Ala Ser Asp Asp Ala Arg Ser Arg Leu Glu
    1430                1435                1440

Phe Ser Arg Leu Asp Arg Phe Ile Thr His Asp Arg Cys Leu Gln
    1445                1450                1455

Val Leu Glu Ser Ser Gly Arg Ser Asp Glu Val Ile Gln Gly Gln
    1460                1465                1470

Thr Ile Tyr Ser Val Leu Ser Ser Ser Asp Val Asn Tyr Gly Gln
    1475                1480                1485

Arg Leu Arg Gly Leu Gln Arg Leu Val Gly Arg Ser Asn Glu Ser
    1490                1495                1500

Ala Gly Arg Leu Val Arg Arg Arg Ser Gly Lys Leu Phe Val Asp
    1505                1510                1515

Phe Ala Leu Gly Glu Val Phe Thr Gln Val Gly Ser Ile Trp Ala
    1520                1525                1530

Asn Cys Met Ala Gln His Gln Arg Asn Thr Arg Asn Lys Gly Ile
    1535                1540                1545

Tyr Met Ala Thr Gly Leu Glu Gln Trp Ser Lys Ser Pro Lys Val
    1550                1555                1560

Leu Gln Lys Phe Asn Gln Gly Leu Tyr Asp Asn Asp Pro Glu Ile
    1565                1570                1575

Glu Trp His Val Leu Ala Gln His Lys Arg Asn Thr Ser Asp Asp

1580                1585                1590

Ser Phe Thr Thr Asp Ile Phe Val Phe Asp Ala Ala Ser Gly Asp
    1595                1600                1605

Leu Glu Glu Val Ile Leu Gly Ile Lys Tyr Ala Pro Val Ser Leu
    1610                1615                1620

Asp Gln Leu Phe Ser Gly Ser Ala Ile Ala Thr Ala Thr Thr Pro
    1625                1630                1635

Val Ala Asn Gly Tyr Val Pro Leu Thr Thr Pro Phe Val Pro Val
    1640                1645                1650

Pro Thr Thr Thr Lys Gln Ala Ala Val Pro Gln Pro Gln His Val
    1655                1660                1665

Ala Lys Lys Ala Ala Pro Arg Ala Ala Pro Lys Arg Asp Ile Lys
    1670                1675                1680

Glu Glu Leu Trp Leu Arg Leu Arg Pro Val Leu Ala Asp Ile Ser
    1685                1690                1695

Gly Leu Glu Pro Glu Glu Ile Gln Pro Thr Asp Ala Leu Ala Asp
    1700                1705                1710

Ile Gly Ile Asp Ser Leu Met Gly Met Glu Met Ala Arg Glu Val
    1715                1720                1725

Glu Thr Thr Phe Asn Cys Thr Val Glu Gln Ser Glu Leu Leu Ser
    1730                1735                1740

Ile Val Asp Val Pro Gly Ile Leu Lys Phe Leu Gln Ser Thr Leu
    1745                1750                1755

Gly Asp Glu Asp Val His Asp Ser Ser Glu Thr Met Ser Thr Val
    1760                1765                1770

Ser Ser Asp Gly Asn Val His Ser Pro Pro Thr Ser Gly Ser Glu
    1775                1780                1785

Met Ala Ser Pro Asn Leu Lys Val Ser Tyr Gly Ser Ala Tyr Gly
    1790                1795                1800

Ser Ser Asp Leu Pro Ile Ser Ala Val Leu Glu Ala Phe Gly Glu
    1805                1810                1815

Ser Ser Ala Lys Thr Asp Gln Phe Leu Lys Thr Tyr Gly Cys Ala
    1820                1825                1830

Gly Tyr Leu Asp Gly Val Ser Gln Lys Gln Thr Arg Leu Cys Leu
    1835                1840                1845

Val Leu Thr Ser Ala Ala Phe Lys Gln Leu Gly Cys Asp Leu Glu
    1850                1855                1860

Ala Ala Lys Pro Gly Glu Val Leu Gln Pro Val Pro Phe Val Glu
    1865                1870                1875

Arg His Arg Arg Phe His Gln Tyr Leu Tyr Ser Met Leu Glu Glu
    1880                1885                1890

Thr Arg Ile Ile Asn Ile Asp Gly Asp Val Ile Thr Arg Thr Ala
    1895                1900                1905

Ile Pro Leu Pro Ser Gln Ser Ala Asp Ala Ile Leu Gln Asp Leu
    1910                1915                1920

Met Arg Arg His Ala Asp Asn Gly Ser Ser His Gln Leu Ala Tyr
    1925                1930                1935

Asn Val Gly Ser Arg Met Ala Asp Val Leu Ser Gly Lys Ala Asp
    1940                1945                1950

Gly Pro Gln Leu Ile Phe Gly Asp Ala Lys Asn Arg Glu Leu Val
    1955                1960                1965

Ala Asn Phe Tyr Gly Glu Leu Pro Phe Asn Lys Leu Tyr Phe Glu
    1970                1975                1980

Leu Met Ala Asp Phe Leu Thr Arg Leu Ala Asn Lys Leu Lys Leu
    1985                1990                1995

Ser Ser Ser Arg Ser Gly Thr Pro Thr Leu Lys Ile Leu Glu Met
    2000                2005                2010

Gly Ala Gly Thr Gly Gly Thr Thr Lys Val Leu Leu Pro Ile Leu
    2015                2020                2025

Ala Lys Leu Gly Ile Pro Leu Glu Tyr Thr Phe Thr Asp Leu Ser
    2030                2035                2040

Pro Ser Leu Val Ala Gln Ala Lys Arg Arg Phe Lys Glu Tyr Pro
    2045                2050                2055

Phe Met Lys Phe Ala Val His Asp Ile Glu Gln Pro Pro Ser Asp
    2060                2065                2070

Pro Glu Leu Val Gly Ser Gln His Val Val Ile Ala Ser Asn Ala
    2075                2080                2085

Val His Ala Thr His Ser Leu Arg Asp Ser Ala Arg Asn Ile His
    2090                2095                2100

Lys Phe Leu Arg Pro Asp Gly Phe Leu Met Leu Leu Glu Met Met
    2105                2110                2115

Arg Thr Leu His Trp Val Asp Val Val Trp Gly Thr Leu Glu Gly
    2120                2125                2130

Trp Trp Leu Phe Asp Asp Gly Arg Thr His Ala Ile Val Asn Glu
    2135                2140                2145

Gln Arg Trp Glu Lys Glu Leu Leu Ala Ser Gly Phe Lys His Val
    2150                2155                2160

Arg Trp Thr Asp Gly Lys Leu Pro Glu Val His Val Gln Arg Val
    2165                2170                2175

Ile Ile Ala Leu Ala Gly Asp Gly Asp Gly Asp Val Ser Asp Ile
    2180                2185                2190

Pro Ala Leu Thr Ser Pro Ala Leu Lys Asp Glu Glu Asp His Gly
    2195                2200                2205

Ser Lys Leu Asp Gly Glu Glu Arg Lys Arg Val Ala Asn Ala Tyr
    2210                2215                2220

Val Glu Ser Thr Ile Arg Asp Phe Ala Ile Pro Ser Tyr Thr Gly
    2225                2230                2235

Pro Ile Leu Ser Thr Ala Pro Gly Ala Gly Ala Cys Val Leu Val
    2240                2245                2250

Thr Gly Ala Thr Gly Ser Leu Gly Ser His Leu Val Ala His Ile
    2255                2260                2265

Ala Gly Leu Pro Ser Val Asp Thr Ile Tyr Cys Leu Asn Arg Pro
    2270                2275                2280

Arg Pro Gly Lys Lys Gly Gln Glu Asp Gln Ser Arg Asp Pro Leu
    2285                2290                2295

Ser Arg Leu Thr Glu Val Leu Ala Ser Lys Ser Ile Gln Leu Ser
    2300                2305                2310

Glu Ala Glu Ile Ser Lys Leu Arg Val Ile Glu Thr Asp Leu Pro
    2315                2320                2325

Leu Pro Gln Phe Gly Leu Asp Glu Ile Gln Tyr Glu Gln Leu Leu
    2330                2335                2340

Asn Asn Val Thr His Ile Val His Ser Ala Phe Pro Val Asn Gly
    2345                2350                2355

Leu Arg Ser Leu Lys Gln Asn Glu Pro Gln Phe Thr Leu Met Arg
    2360                2365                2370

Asn Leu Val Asp Leu Ala Ala Gly Val Ser Ala Arg Arg Pro Thr
    2375                2380                2385

Glu Phe Lys Phe Thr Phe Gln Phe Ile Ser Ser Leu Ser Ala Val
    2390                2395                2400

Gly Met Tyr Pro Lys Val His Gly Glu Lys Arg Val Pro Glu Gln
    2405                2410                2415

Gln Trp Asp Val Asp Ser Ala Leu Pro Asn Gly Tyr Gly Glu Ala
    2420                2425                2430

Lys Val Ile Cys Glu Arg Val Leu Leu Glu Thr Leu Gly Gln His
    2435                2440                2445

Pro Asp Arg Phe Arg Ala Met Thr Val Arg Leu Gly Gln Leu Ser
    2450                2455                2460

Gly Ser Met Lys Thr Gly Tyr Trp Asn His Met Glu Met Leu Ser
    2465                2470                2475

Phe Leu Phe Lys Ser Ala Gln Thr Leu Arg Ala Leu Pro Asp Val
    2480                2485                2490

Asp Gly Asp Val Ser Trp Leu His Leu Glu Asp Ala Ser Ala Ser
    2495                2500                2505

Leu Ala Asp Leu Leu Leu Arg Asp Ala Pro Thr Cys His Ala Val
    2510                2515                2520

Tyr His Leu Asp Asn Pro Arg Pro Arg Asp Trp Lys Asp Val Ile

2525                    2530                    2535

Pro Val Leu Ala Asp Ala Leu Asp Ile Pro Ser Ser His Ile Val
    2540                    2545                    2550

Pro Phe Glu Glu Trp Leu Arg Arg Val Arg Ala Tyr Pro Gly Glu
    2555                    2560                    2565

Asp Pro Trp Asp Asn Pro Ser Ala Lys Ala Met Asp Phe Phe Glu
    2570                    2575                    2580

His Lys Phe Lys His Met Ser Cys Gly Gly Val Thr Met Ala Thr
    2585                    2590                    2595

Asp His Ala Leu Glu His Ser Glu Thr Leu Arg Ala Val Gln Pro
    2600                    2605                    2610

Val Ser Asp Glu Leu Val Arg Lys Tyr Ile Gln Ala Trp Lys Asp
    2615                    2620                    2625

Ser Gly Phe Leu Arg
    2630

<210>  62
<211>  456
<212>  PRT
<213>  Trichoderma reesei

<400>  62

Met Glu Pro Asn Asn His His Asp Gly Leu Asn Gly Gln Lys Ala Asp
1               5                   10                  15

Asn Pro Leu Glu Val Ala Ile Val Gly Gly Gly Leu Thr Gly Leu Ala
            20                  25                  30

Leu Ala Leu Gly Leu Leu Arg Arg Asn Val Asn Phe Thr Ile Tyr Glu
        35                  40                  45

Arg Ala Ala Ser Phe Gly Glu Leu Gly Val Gly Ile His Phe Thr Pro
    50                  55                  60

Asn Ala Glu Arg Ala Met Lys Ala Leu Asp Pro Arg Ile Leu Gln Ser
65                  70                  75                  80

Tyr Val Asp Val Ala Thr His Ala Glu Gly Gly Phe Leu Ser Phe Val
                85                  90                  95

Asp Gly Leu His Asp Asp Asp Leu Leu Phe Gln Leu Arg Met Gly Glu

            100                 105                 110

Gly Tyr Lys Ala Ala Arg Arg Cys Asp Ile Val Ser Gln Leu Val Lys
        115                 120                 125

His Ile Pro Gln Glu Arg Val Gln Leu Leu Lys Trp Leu Gln Ser Val
    130                 135                 140

Glu Glu Asp Ala Glu Gly Arg Ala Val Leu Thr Phe Arg Asp Gly Ser
145                 150                 155                 160

Thr Ala Lys Ala Asp Val Val Val Gly Cys Asp Gly Ile Arg Ser Gln
                165                 170                 175

Val Arg Ser Ala Met Phe Gly Ser Gly Pro Ser Ala Pro Arg Ala Gln
            180                 185                 190

Tyr Ala His Gln Leu Ala Phe Arg Gly Leu Val Pro Met Ala Lys Val
        195                 200                 205

Ala Ala Thr Leu Gly Pro Glu Lys Thr Ser Arg Ala Ile Gly Tyr Leu
    210                 215                 220

Gly Pro Gly Gly Phe Val Leu Ser Val Pro Leu Ala Gly Ile Asn Met
225                 230                 235                 240

Met His Leu Glu Val Phe Val Met Asp Pro Leu Pro Trp Ser Asp Asp
                245                 250                 255

Thr Thr Gly Ser Lys Pro Asp Lys Asp Lys Asp Glu Asp Asp Val Lys
            260                 265                 270

Arg Tyr Val Leu Pro Ser Thr Arg Ala Glu Ala Glu Lys Ala Phe Thr
        275                 280                 285

Glu Phe Asn Pro Thr Val Arg Ser Leu Ile Ser Leu Leu Pro Glu Glu
    290                 295                 300

Leu Gly Lys Trp Ala Ile Phe Asp Met Leu Asp Ser Pro Ala Pro Ser
305                 310                 315                 320

Tyr Ala Leu Gly Arg Met Cys Leu Ala Gly Asp Ala Ala His Ala Ser
                325                 330                 335

Thr Pro Asn Gln Gly Gly Gly Ala Gly Ala Gly Met Glu Asp Ser Leu
            340                 345                 350

Val Leu Ala Glu Ile Leu Ala Ala Leu Ala Asp Arg Ala Lys Ser Gly
        355                 360                 365

Ala Arg Val Gly Ser Trp Glu Ile Ser Glu Gly Leu Lys Ile Tyr Ser
    370                 375                 380

Asp Ala Arg Tyr Glu Arg Ala Gln Trp Leu Val Gln Ser Ser Arg Arg
385                 390                 395                 400

Val Ala Gln Leu Phe Thr Arg Lys Lys Gly Ala Gly Gln Gly Gly Glu
                405                 410                 415

Glu Glu Glu Pro Ile Ser Arg Glu Ile Leu Glu Arg Ser His Gln Leu
            420                 425                 430

Trp Asp Tyr Asp Val Asp Ala Ala Val Glu Glu Ala Leu Gly Lys Leu
        435                 440                 445

Arg Ala Lys Val Leu Glu Lys His
    450                 455

<210>  63
<211>  534
<212>  PRT
<213>  Trichoderma reesei

<400>  63
   Met Ser Lys Glu Ala Ser Gln Asp Ser Arg Ser Ile Thr Pro Val Glu
   1               5                   10                  15
   Ala Val Glu Pro Leu Glu Val Val Asp Ala Glu Lys Asn Val Thr Thr
               20                  25                  30
   Ser Pro Tyr Asn Gly Ser Gly Thr Val Glu Asp Pro Phe Ile Val Glu
           35                  40                  45
   Phe Gln Gln Asp Asp Lys Ser Asn Pro Met Asn Trp Gly Gln Phe Arg
       50                  55                  60
   Lys Trp Phe Leu Thr Ser Ile Val Thr Phe Ser Val Phe Ala Val Thr
   65                  70                  75                  80
   Phe Thr Ser Ser Ala Tyr Ser Val Ser Ala Glu Glu Ile Met Thr Glu
                   85                  90                  95
   Phe Asp Ile Ser Ser Thr Leu Phe Ile Thr Gly Val Ser Val Phe Val
               100                 105                 110
   Leu Gly Phe Ala Ile Gly Pro Ala Val Trp Gly Pro Leu Val Thr Pro
           115                 120                 125
   His Asp Glu Arg Ser Asn Ala Asn Arg Ala Ser Leu Gln Ser Thr Arg
       130                 135                 140
   Ser Glu Leu Tyr Gly Arg Gln Met Pro Trp Ile Ala Ser His Thr Ala
   145                 150                 155                 160
   Met Val Ala Phe Met Ala Gly Ser Ala Gly Ser Pro Asn Ile Ala Thr
                   165                 170                 175
   Leu Ile Val Leu Arg Phe Leu Ala Gly Thr Phe Gly Gly Ser Pro Leu
               180                 185                 190
   Val Asn Ser Gly Gly Ala Ile Ala Asp Leu Phe Pro Pro Ala Gln Arg
           195                 200                 205
   Gly Leu Ala Met Thr Ile Tyr Cys Val Ala Pro Phe Leu Gly Pro Ile
       210                 215                 220
   Leu Gly Pro Ile Val Gly Gly Phe Ala Thr Glu Tyr Ile Gly Trp Arg
   225                 230                 235                 240
   Trp Val Gln Gly Met Cys Thr Ile Phe Ile Gly Val Ile Gly Ile Ile
                   245                 250                 255
   Gly Val Ile Phe Val Pro Glu Thr Tyr Gly Pro Val Leu Leu Gln Arg
               260                 265                 270
   Lys Ala Asn Ala Leu Ser Lys Ala Asp Gly Lys Val Tyr Ile Ser Val
           275                 280                 285
   Leu Gln Lys Asn Gln Gly Lys Lys Gln Pro Ser Glu Val Phe Gly Arg
       290                 295                 300
   Ala Leu Ile Arg Pro Trp Val Leu Leu Phe Arg Glu Pro Ile Val Leu
   305                 310                 315                 320
   Ile Ala Ser Leu Tyr Met Ala Ile Ile Tyr Gly Thr Val Tyr Met Phe
                   325                 330                 335
   Leu Gly Ala Met Pro Ile Val Tyr Asn Glu Leu Arg Gly Trp Ser Pro
               340                 345                 350
   Gly Phe Gly Gly Leu Ala Phe Leu Gly Met Met Val Gly Ile Ile Ile
           355                 360                 365
   Gly Leu Gly Tyr Ala Ile Trp Asp Asn Asn Gly Arg Tyr Met Lys Leu
       370                 375                 380
   Asp Pro Ser Lys Arg Thr Ala Glu Ser Arg Leu Pro Pro Ala Ile Ala
   385                 390                 395                 400
   Gly Ala Val Ala Leu Pro Ile Gly Met Phe Ala Phe Ala Trp Thr Asn
                   405                 410                 415
   Tyr Pro Ser Ile His Trp Ala Val Ser Ile Val Leu Ser Ala Pro Phe
               420                 425                 430
   Gly Phe Gly Val Val Leu Val Ile Leu Pro Ile Val Asn Tyr Leu Ile
           435                 440                 445
   Asp Ser Tyr Thr Val Tyr Ala Ala Ser Val Leu Ala Ala Ala Ala Val
       450                 455                 460
   Phe Arg Ser Ile Met Gly Ala Val Phe Pro Leu Phe Thr Ser Gln Met
   465                 470                 475                 480
   Tyr His Asn Leu Gly Ile His Trp Ala Thr Ser Ile Pro Ala Phe Leu
                   485                 490                 495
   Thr Leu Val Cys Met Pro Phe Pro Phe Phe Met Tyr Arg Tyr Gly Ala
               500                 505                 510
   Val Val Arg Glu Lys Cys Lys Tyr Ala Ala Glu Ala Ala Gln Ile Met
           515                 520                 525
   Lys Lys Met Gln Gly Arg
       530

<210>  64
<211>  664
<212>  PRT
<213>  Trichoderma reesei

<400>  64

Met Ser Ala Arg Gln Asp Glu Asp Gln Arg Leu Gln Ala Gln Thr Gln
1               5                   10                  15

Ala Gln Ala Gln Val Gln Ala Gln Ala Pro Leu Ser Leu Tyr Arg Glu
            20                  25                  30

Arg Leu Lys Ile Ala Asn Ala Cys Gln Ser Cys Arg Ala Ser Lys Val
        35                  40                  45

Lys Cys Asp Gly Gly Arg Pro Val Cys Ala Arg Cys Gln Lys Arg Gly
    50                  55                  60

Arg Ala Cys Ser Tyr Ser Gln His Asp Ala Ala Ser Pro Arg Gly Arg
65                  70                  75                  80

Gly Arg Gln Arg Ala Lys Ala Pro Thr Arg Gln Pro Arg Pro Ile Arg
                85                  90                  95

Ser Arg Ala Ser Val Glu Leu Pro Val Thr Ala Pro Thr Pro Val Thr
            100                 105                 110

Ala Gln Ala Ser Pro Leu Ile Ala Gln Asp Tyr Ser Leu Gln Thr Pro
        115                 120                 125

Ser Ala Thr Gln Thr Pro Ser Thr Thr Gly Phe Ser Gly Ser Ser Asp
    130                 135                 140

Leu Glu His Val His Glu Asp Arg Asp Glu Ser Arg Ala Phe Tyr Ala
145                 150                 155                 160

Ala His Gly Arg Phe Ala Gly Glu Val Ser Ser Thr Ile Asp Lys Met
                165                 170                 175

Ala Gly Leu Ser Pro Asp Thr Thr Cys Ser Leu Val Pro Phe Val Asp
            180                 185                 190

Ala Pro Leu Phe Gly Asp Val Gly Glu Pro Pro Arg Asn Val Val Leu
        195                 200                 205

Asp Phe Ala Ser Asp Leu Pro Arg Ala Tyr Ala Asp Arg Leu Leu Ala
    210                 215                 220

Ile Tyr Trp His His Val His Pro Val Glu Pro Val Leu Asp Gln Gln
225                 230                 235                 240

Gln Phe Cys Arg Thr Tyr Asp Ala Phe Tyr Ser Gly Ser Gly Thr Pro
                245                 250                 255

Leu His Val Asp Arg Asp Ile Trp Leu Ser Thr Leu Asn Ile Val Phe
            260                 265                 270

Ala Leu Ala Val Gln Ile Gln Glu Ser Ile Pro Met Gln Lys Arg Asp
        275                 280                 285

Asp Glu Ala Asn Arg Tyr Phe Gln Arg Ala Trp Ala Leu Leu Arg Pro
    290                 295                 300

Glu Ala Ile Leu Trp Lys Pro Gly Ser Leu Glu Leu Val Gln Cys Leu
305                 310                 315                 320

Leu Leu Met Asn Arg Tyr Leu His Cys Thr Asn Asn Gln Gln Lys Thr
                325                 330                 335

Ser Met Ala Ala Thr Leu Ala Ile Arg Ile Ala Gln Asn Met Val Cys
            340                 345                 350

His Thr Ser Glu Glu Ser Pro Ser Ser Asp Ala Asp Lys Asp Leu Arg
        355                 360                 365

His Lys Val Trp Ala Ser Cys Val Ala Leu Glu Arg Pro Ala Leu Leu
    370                 375                 380

Thr Gly His Gly Ser Asp Phe His Ala Trp Glu Leu Glu Leu His Glu
385                 390                 395                 400

Ile Gly Thr His Ile Gln Leu Ala Gln Val Gln Ser Lys Asn Ser Met
                405                 410                 415

Ala Thr Lys Leu Gly Leu Pro Arg Leu Tyr Gln Gln Asp Glu Tyr His
            420                 425                 430

Ala Ile Ala Val Gln Leu Asp Gly Cys Leu Asn Lys Trp Glu Lys Ser
        435                 440                 445

Leu Pro Asp Asp Trp Arg Leu Gln Asn Met His Met Ile His Asp Arg
    450                 455                 460

Arg Ala Arg Ala Glu Arg Tyr Leu Leu His Phe Arg Leu Leu His Ser
465                 470                 475                 480

Arg Ile Tyr Leu His Arg Pro Met Leu Ala Arg Leu Tyr Ala Ile Lys
                485                 490                 495

Ser His Ala Pro Thr Ala Ala Ala Ala Ser Asp Pro Ser Thr Ile Ser
            500                 505                 510

Asp Arg Leu Leu Gln Glu Cys Ala Arg Met Cys Leu Glu Ala Ala Gln
        515                 520                 525

Lys Leu Thr Ser Leu Ile Ala Glu Ile His Asp Pro Asn Glu Pro Ile
    530                 535                 540

Gly Ile Leu Pro Trp Trp Tyr Arg Val Tyr Tyr Leu His Ile Ala Gly

545                 550                 555                 560

Ile His Phe Leu Ala Ala Met Phe Ala Ser Asp Leu Phe Thr Pro Ser
                565                 570                 575

Val Glu Arg Ala Trp Tyr Gln Val Leu Ala Ala Leu Arg Ala His Glu
            580                 585                 590

His Leu Ser Leu Tyr Val Gln Gln Cys Ala Arg Thr Phe Glu Thr Leu
        595                 600                 605

Ala Ala Arg Ile Leu Asn Ala Arg Cys Leu Ser Val Asn Gly Asn Gly
    610                 615                 620

Ile Met Ala Leu Asp Asp Gly Ala Pro Gly Leu Phe Leu Asp Asp Met
625                 630                 635                 640

Phe Gln Asp Val Asn Phe Asp Leu Asp Glu Phe Leu Phe Ser Val Asp
                645                 650                 655

Asp Thr Gly Arg Arg Thr Asn Tyr
            660

<210>  65
<211>  574
<212>  PRT
<213>  Trichoderma reesei

<400>  65

Met Tyr Ala Pro Pro Phe Val Arg Ala Phe Gly Ile Ala Val Leu Ala
1               5                   10                  15

Val Leu Pro Ser Phe Ser Ser Pro Ala Thr Ala Ala Ser Leu Lys Ser
            20                  25                  30

Ser Gly Ser Ser Ser Ser Cys Arg Cys Phe Pro Gly Asp Ala Cys Trp
        35                  40                  45

Pro Ser Pro Ala Asp Trp Lys Ala Phe Asn Gln Ser Val Gly Gly Arg
    50                  55                  60

Leu Ile Ala Thr Val Pro Leu Gly Ser Val Cys His Gly Thr Thr Tyr
65                  70                  75                  80

Asp Ala Ala Arg Cys Ala Asp Val Lys Ala Ala Trp Pro Tyr Ala Asp
                85                  90                  95

Thr His Thr Asp Ser Ser Ser Ser Val Leu Ala Pro Phe Phe Ala Asn

            100                 105                 110

Gln Ser Cys Asp Pro Phe Leu Pro Arg Glu Thr Pro Cys Val Ile Gly
        115                 120                 125

Thr Tyr Val Gln Tyr Ala Val Asn Val Ser Ser Val Ala Asp Ile Gln
    130                 135                 140

Lys Thr Leu Ala Phe Ser Gln Lys Lys Asn Leu Arg Leu Val Val Arg
145                 150                 155                 160

Asn Thr Gly His Asp Tyr Phe Gly Lys Ser Thr Gly Ala Gly Gly Leu
                165                 170                 175

Gly Leu Trp Met His Asn Leu Lys Thr Tyr Asp Ile His Asp Tyr Lys
            180                 185                 190

Ser Ala Ala Tyr Thr Gly Lys Ala Val Thr Met Gly Ala Gly Ile Gln
        195                 200                 205

Ala Gly Glu Ser Ala Ala Thr Ala Phe Lys Gln Gly Leu Thr Ile Val
    210                 215                 220

Ser Gly Ile Cys Pro Thr Val Gly Leu Ala Gly Gly Tyr Thr Gln Gly
225                 230                 235                 240

Gly Gly Leu Gly Pro Leu Thr Thr Arg Tyr Gly Leu Gly Ala Asp Gln
                245                 250                 255

Val Leu Glu Trp His Ala Val Leu Ala Asn Gly Ser Glu Ile Thr Ala
            260                 265                 270

Thr Pro Thr Lys Asn Ser Asp Leu Tyr Trp Ala Leu Thr Gly Gly Gly
        275                 280                 285

Gly Gly Thr Tyr Ala Val Val Tyr Ser Met Thr Val Lys Ala His Ala
    290                 295                 300

Asn Glu Lys Thr Thr Gly Ala Asn Leu Thr Phe Pro Asn Ala Gly Ser
305                 310                 315                 320

Glu Asp Val Phe Phe Gln Gly Val Gln Ala Phe His Asp Ile Ile Pro
                325                 330                 335

Ala Ile Ser Asp Ala Gly Gly Thr Ala Val Trp Thr Val Leu Ser Lys
            340                 345                 350

Ala Leu Ser Val Gly Pro Val Thr Gly Pro Asn Met Thr Lys Ala Thr
        355                 360                 365

Met Asp Ser Ile Phe Gln Pro Val Leu Gln Lys Leu Asp Ala Leu Asn
    370                 375                 380

Ile Thr Tyr Ser Tyr Ser Ser Gly Glu Phe Ser Ser Phe Tyr Glu Ser
385                 390                 395                 400

Asn Ala Ala Tyr Asp Pro Pro Val Val Ser Asn Gly Leu Gln Ile Gly
                405                 410                 415

Gly Arg Leu Val Lys Arg Ser Asp Phe Thr Gly Asn Pro Asp Gly Phe
            420                 425                 430

Ile Gln Ala Ile Arg Gly Ile Ala Asp Gln Gly Gly Leu Val Thr Gly
        435                 440                 445

Ala Ser Tyr Gln Leu Ser Ser Ser Leu Gln His Pro Pro Asn Ser Val
    450                 455                 460

Asn Pro Glu Leu Arg Lys Ser Leu Ile Ser Phe Gln Ile Gly Val Pro
465                 470                 475                 480

Trp Ile Asn Thr Asp Trp Ala Thr Asp Leu His Asn Gln Asp Leu Ile
                485                 490                 495

Thr Asn Ser Phe Val Pro Ala Leu Ala Ala Leu Leu Pro Ser Gly Gly
            500                 505                 510

Ser Ala Tyr Leu Asn Gln Ala Asp Phe Arg Glu Pro Gly Trp Gln Gln
        515                 520                 525

Val Phe Tyr Gly Glu Asn Tyr Glu Lys Leu Leu Glu Leu Lys Asp Val
    530                 535                 540

Tyr Asp Pro Asn Gly Val Phe Trp Gly Arg Thr Thr Val Gly Ser Glu
545                 550                 555                 560

Arg Trp Ala Glu Thr Glu Asp Lys Arg Leu Cys Arg Val Ser
                565                 570

<210>  66
<211>  685
<212>  PRT
<213>  Trichoderma reesei

<400>  66

Met Gly Ser Ser Ala Thr Ala Thr Thr Thr Gly Glu Ser Thr Arg Gln
1               5                   10                  15

Gln Pro Gly Leu Ala Cys Glu Glu Cys Arg Arg Arg Lys Ala Arg Cys
            20                  25                  30

Asp Arg Val Arg Pro Lys Cys Gly Ile Cys Ala Asp Ser Gly Arg Asn
        35                  40                  45

Cys Val Val Leu Asp Lys Arg Ser Gln Arg Gly Pro Lys Lys Gly Gln
    50                  55                  60

Leu Lys Asp Leu Arg Ser Arg Leu Met Leu Leu Glu Gln Arg Leu Val
65                  70                  75                  80

Gly Gln Asn Asp Ala Pro Ser Leu Pro Gln Glu Arg Asp Ser Leu Gly
                85                  90                  95

Cys Pro Thr Pro Ser Glu Lys Val Ser Pro Glu Gly Asp Leu Val Ser
            100                 105                 110

Ser Arg Ala Ser Thr Asp Ile Gly Leu Asp Thr Cys Met Gly Ser Thr
        115                 120                 125

Ala Gly Ala Phe Ala Leu Phe Gly Ser Thr Ser Gly Ser Asp Lys Met
    130                 135                 140

Pro Pro Leu Thr Pro Asp Leu Ser Thr Ala Ser Thr Ala Asn Ser Met
145                 150                 155                 160

Ala Ala Cys Ile Cys Lys Gly Trp Gln Thr Glu Pro Ala Met Phe Ser
                165                 170                 175

Thr Ser Pro Phe Pro Gln Ile Pro Leu Thr Pro Gln Ser Thr Thr Ala
            180                 185                 190

Pro Ser Arg Met Pro Ser Ile Ser Leu Ala Ala Ala Asp Pro Ile Met
        195                 200                 205

Pro Asp Val Arg Val His Pro Phe Ala Pro Met Val His Lys Arg Arg
    210                 215                 220

Tyr Tyr Ala Trp Ala Ser Asp Pro Asn Ala Ser Pro Ala Arg Thr Ala
225                 230                 235                 240

Leu Arg Ser Ala Met Arg Thr Ile Ala Ser Ala Met Ser Pro Gln Phe
                245                 250                 255

Cys Asp Ile Gly His Val Met Tyr Ala Ser Thr Arg Arg Met Leu Glu
            260                 265                 270

Thr Gln Asp Ala Cys Pro Glu Thr Gly Leu Pro Trp Met Thr Arg Leu
        275                 280                 285

Lys Pro Pro His Glu Gln Arg Lys Met His His Glu Arg Ile Gln Ala
    290                 295                 300

Trp Leu Leu Leu Ala Tyr Tyr Asp Val Leu Arg Lys Ser Glu His Gln
305                 310                 315                 320

Ala Phe Ile Thr Ala Arg Arg Ala Phe Arg Leu Leu Arg Leu Ser Gly
                325                 330                 335

Leu Cys Glu Met Asp Ile Asp Ala Tyr Ser Gly Arg Gln Gly Asp Val
            340                 345                 350

Ala Thr Cys Thr Pro Pro Ala Glu Thr Thr Trp Asn Met Gln Met Cys
        355                 360                 365

Ser Ser Glu Asn Gly Ala Asp Glu Ala Val Leu Gln Gln Asp Trp Ile
    370                 375                 380

Ser Val Glu Glu Arg Arg Arg Thr Val Trp Ser Ala Phe Leu Leu Asp
385                 390                 395                 400

Arg Leu Ser Thr Met Val Asn Asp Gln Pro Thr Met Leu Met Glu Glu
                405                 410                 415

Ala Val Thr Lys Pro His Ala Cys Thr Phe Ser Trp Tyr Phe Ser Ala
            420                 425                 430

Asp Leu Pro Ile Ser Asn Gln Phe Tyr Thr Arg Leu Pro Met Ser Glu
        435                 440                 445

Ala Glu Phe Gln Ser Gly Thr Gln Glu Pro Val Ser Gln Met Gly Phe
    450                 455                 460

Leu Ile Glu Ala Thr Asp Gly Ile Lys Ala Ser Asn Ser Ile Gln Pro
465                 470                 475                 480

Leu Pro Pro Phe Ala His Cys Val Val Val Ala Asn Leu Phe Ala Arg
                485                 490                 495

Cys Met Thr His Cys Lys Met Ala Met Gln Ser Pro Pro Met Ser Ala
            500                 505                 510

Pro Glu Ala His Asp Phe Trp Ile Arg His Gln Trp Leu Ala Ser Ala
        515                 520                 525

Ala Ala Asn Ala Cys Glu Ser Thr Glu Thr Arg Cys Asp Pro Met Leu
    530                 535                 540

Val Phe Thr Arg Ile Leu Ala Tyr Ser Ala Ser Leu Ser Leu Cys Ser
545                 550                 555                 560

Thr Ala Asn Ala Thr Ser Trp Gln Thr Leu Asp His His Leu Met Ala
                565                 570                 575

Met Ala Cys Lys Pro Ala Ala His Gln Ala Ala Ser Glu Val Val Arg
            580                 585                 590

Ile Ile Lys Thr Ala Pro Arg Ile Ala Phe Phe Lys Met His Pro Phe
        595                 600                 605

Phe Pro Asn Ala Ile Ala Leu Val Thr Ser Phe Leu Asn Ala Asp Val
    610                 615                 620

Pro Tyr Leu Pro Ser Thr Arg Gly Gly Asn Ala Met Asp Ala Ile Gln
625                 630                 635                 640

Glu Arg Gln Asp Ala Val Asn Glu Leu Leu Ala Ala Leu Arg Arg Ser
                645                 650                 655

Ser Gln Val Asn Asn Leu Ala Ala Glu Leu Leu Cys Lys Leu Glu Leu
            660                 665                 670

Asp Ile Gly Gln Ala Ala Ser Asp Gly Ser Ile Tyr Gly
        675                 680                 685

<210>  67
<211>  1000
<212>  DNA
<213>  Trichoderma reesei

<400>  67
acatcccaga aaagggtgga gaggacccat tggaatctcc ccttgtgcga ggccctagca      60

aaccaaacaa gccttgcttt ggcccctgtg acatctcatc accttgtgga tgccctcgta     120

ccctcagtcc agggtcgagg gctccagttg gccagagtgc aagcactcgt ctgctcgcaa     180

cctcagaagc tccggtgcgg acgacgaaga ccccccccct gtccagcttt ccgagcccat     240

ggagctccac aaaactcacg accctaccag gaacaccaga ccccggcact ctgcacctgc     300

aggggtctgc ccgtcctctt ggtcgactga acgttgtatt cgagactagt ttcctggcct     360

ggcagctgct gctcgacaga gtccctctgc ctatcgagcc aggaagtact ttatgacccg 420

catgccctct cttcatgcct ggatgccgtc gtctccgtta agatcttggg tttgggcgaa 480

actcgggttc cttccgtatt cttatcttca tacctctttg ttcttcgtgt cttccttttt 540

ccatcattgg ataataacgc tctcttctac ttcgctggat aacaccgcac acgaccgtat 600

acctttcttt gcctcactat gctgtgatac taccacgact gcctgctacc tatcaaaaca 660

cgcctgccgc aaggtctgag gattcatcac atcaatcgat tgtaatacgc cgccttcgga 720

tggctcgaac cacttgatat actgggtcat atgataacgc atctgttcga ctgtgcacga 780

gacatcacag cgctccgagt accacgattc accagtgaca ttgacggccc ctcttcttga 840

tttcgcattt cgacgatcga ttgttcagct catcactcgt acatctcggg gcgcaccggg 900

aaaagcgact atcgacgagc ctccccagga gggatactta tttctcgacg acaccctcta 960

tctttccaag gctacctttg cggaacatcc ctttcccacc 1000

<210> 68
<211> 1020
<212> DNA
<213> Trichoderma reesei

<400> 68
cccagaaaag gaagccatcg tcgtcgagct gctgcggtcc atcaaggttc atacctactt 60

aggtacctac atgcatgccg agaaatccat tctgggattg aaaccccccc ttgttagtgg 120

tacagtgtcg ctactgtgga cccgaaacgg cgggttgctt ccccgtcaga cttgcttagc 180

gggctacagc gtccactaag aactcccttt cttcagggtg cttccgctga gaaaccggag 240

gacgaggacc cgagaaatac agtgtcatat atccaagaac ccactgtacg tcccttcttc 300

cctgccaggg gcacaagtac catctccata cagctccagg ggacgagata catcgccccc 360

cttgcttatg cgtggggcct tttgtctcag ttaatgggca gcagcttgtc ttcagctcgc 420

agctccatcc ctacccttct gccatcttcc caacctcatc tctcatcagc aacatcacgg 480

gcatatcatt catcgcctcg ggggatccag gcatcagccg cttttgccct ggcagcgaat 540

cggacccggc agaggccaca cgctgcgttt ggggtgggcg cctgatagaa ggaggcgggt 600

cgcatcattg atcgcatcgc gtcgccatcg ccatcgaatc gaggcgcatc ttgcatcaca 660

gcacatcgta tcgcatcgca tcgcatctca ccgttcccgc cagactaggt agcttgaggc 720

aaggggctgg ggtagcaggg acgaatagag acgcgatacg agagcatcca attgccacta 780

ccagtaagta cacaggcccc atcatggacg ggcatcagcc gcagcgtcac gggctgagac 840

gcgcgtcttg tgatcccttt catcttgtct ccttctcctt ctgcctcttc cagcctgctg 900

gtgtcttggc ctccgtgcta attgattggc tcgccaggca gaagctgtcg aacgcacacc 960

ggccacggaa ctggccctat ccgagactcg acatccatct tagtagtagt atcaatcgcc 1020

<210> 69
<211> 1000
<212> DNA
<213> Trichoderma reesei

<400> 69
cacgctgatc tatgtagcgg tcgttgcggg gactgagacg aacggcaagt ttttgcagca 60

taatcaggtt gacccgtaag ttcatgtgat gcttattgtt gaggttctac ggatagtaca 120

gctcaactga gtttgacagg ggtgccgagt ttctcaacac tgcggaagga gaagacttta 180

ttagcaagct ctggaaagag tcggttcaag acgttgctgc tactgatccg gctcttggct 240

cttatgcttg acaggaggat gcggtgagga ggaggcatgg taacgagaat attatgcctt 300

aatcaggtac agtgtcatca agtattggtc tgagaagatg tttgatcccg agcgactctg 360

acctactatg attgcagttt gcgtctatgc cacatgtcgc tcatcatgtg ctcaacattc 420

aatgagccat gttgcaattg gtatcttgca attatgtgaa cgcttagtct cgcatcaacc 480

tatacttttg ctttcccgtg ttatatggag aggaggcctc ccgagtgctt ctcagtttca 540

gtaagtgaag ttgaagtcta tgggtatcat tcgtgtgctc actctcatgg gcattctcaa 600

aagcttgctt acaatgggac ggtatcaagt tggaacacgg aagcaaggcg caagagtcgc 660

ttctcgcttg gaatgtaagt ggaggagtcg tccagctatc atatgcgaag tatcggaata 720

acaatcgttc aggcctaacc aggccaagca atatcactta ctgcatccat cgtagtctat 780

taatctgtga gccctgtgag cgttgcattt tcatttacag acaagcattc tcagatcctt 840

ccacggcggc ggttcagcct ggcgcaacac cttcaacttg gccctcacta tttaggcagt 900

ttccccgacc ccgcatataa cagcaacgca tacctatata taatatggac tctctcacac 960

ctttcaaatt tgatcatgac accaaagcaa tctctcagca 1000

<210> 70
<211> 1000
<212> DNA
<213> Trichoderma reesei

<400> 70
cgcagccact ctgcaagctt ctgcgcgttg ctgacgcctg aagtgtgctg caacttggga 60

attgcttcac acagggatgg ccaatgactc tcgatgtctc tgacggcgct cagcgcccat 120

tggctcgcgg agtccgtgct gataaaggac aagatgctgg cgaagtaggt ctcgttcagg 180

gacatggccc caggcccgaa catgagcaga gtgcgtgtac ttgaggccgc catgactgtc 240

tgcgcaagct ggcgaacgag gttgatgcgc ttctgctgtt gaagttgccc tggaatcccc 300

cttgtgtggt aatgttgagt accacaagcc ttcaaggtga acgaaacgtg atgccttcaa 360

gatggtagat gcatgcagtc accaagacac agaatgtcgt ggactgcacg catgctttta 420

gcatctttgc gagattcgcc tctcatcctt cgtcccgcca gaagctgacg atgctgacag 480

acagattgcc cgccgccgtc attcaaacgt ttgcctgata ctgctgacat ggagtttcga 540

gtttctgccc tcagtgacga tcgccaacat tgaactccgg agttgagata ctccgatgcg 600

gatcgcactt gacgactcct tcggactctg ccaaggcgga ctttttctgc ttctttttct 660

ctctctttta attgcttact gccgaatttc gccgctctcc gaccgcgtcc gtcaggaatt 720

ttgtttcttc aaacgaatcc tgacgatagt atgtccgtat cactcgcaag ccgcttacag 780

gatctgctga tcgagttata gagaaggttc gtcatggcaa gacgcgtcta tgaagctgac 840

ggccctcaag ctgggcttcg aagcagtcgc atatgtaaaa taccttgacc ggcttcgttt 900

tcccaggcct aaacttgtct ccgtccccga acaagtattt ccagacgcaa acactcagcc 960

cccttcaacc ttctttatcg accaccctca acgacggaag 1000

<210> 71
<211> 1000
<212> DNA
<213> Trichoderma reesei

<400> 71
gatttccagc aggctctacc tacagtattc agccgctcgt tgttcggatg gtagactcct 60

tccgatctga atctagaggc tgggaactct gtccacccgc ttctcccctc agccagcatc 120

tgccagagct tgtccgtgtt ggttgcatct ccagcaaact tggagctcat gccaatgatg 180

gcaataggct cactcgccca gcgagactgg ctatcggggc cgagacttcc catcttccgt 240

cgttgagggt ggtcgataaa gaaggttgaa gggggctgag tgtttgcgtc tggaaatact 300

tgttcgggga cggagacaag tttaggcctg ggaaaacgaa gccggtcaag gtattttaca 360

tatgcgactg cttcgaagcc cagcttgagg gccgtcagct tcatagacgc gtcttgccat 420

gacgaacctt ctctataact cgatcagcag atcctgtaag cggcttgcga gtgatacgga 480

catactatcg tcaggattcg tttgaagaaa caaaattcct gacggacgcg gtcggagagc 540

ggcgaaattc ggcagtaagc aattaaaaga gagagaaaaa gaagcagaaa aagtccgcct 600

tggcagagtc cgaaggagtc gtcaagtgcg atccgcatcg gagtatctca actccggagt 660

tcaatgttgg cgatcgtcac tgagggcaga aactcgaaac tccatgtcag cagtatcagg 720

caaacgtttg aatgacggcg gcgggcaatc tgtctgtcag catcgtcagc ttctggcggg 780

acgaaggatg agaggcgaat ctcgcaaaga tgctaaaagc atgcgtgcag tccacgacat 840

tctgtgtctt ggtgactgca tgcatctacc atcttgaagg catcacgttt cgttcacctt 900

gaaggcttgt ggtactcaac attaccacac aagggggatt ccagggcaac ttcaacagca 960

gaagcgcatc aacctcgttc gccagcttgc gcagacagtc 1000

<210> 72

<211>  1044
<212>  DNA
<213>  Trichoderma reesei

<400>  72
cgggtgcgag cctaccctgg ggaggacccg tgggataacc cctcggccaa ggcaatggac      60

tttttcgaac acaagttcaa gcacatgtct tgcggcggag tgacaatggc gacggatcat     120

gcgctggagc attcggagac tttgagggct gtacagcctg tgagtgatga gctggtgcgc     180

aagtatatcc aggcgtggaa ggatagcggc tttttgcgat gagtctgtat tgaagggtta     240

tgtctactat attgttcaaa ttgaatatct tatcttctat actctacttc ttcaggtgaa     300

attgacaact attcaatcat gaatatgagt taccaagtgc tttgtacata cgacatgagc     360

tatcaggaaa gcgtcaagta cctaggtatg cttcaaactt gtatacctac ctaggtaata     420

tgagcttgca ataattcaaa agtttctttt ttaattgcat ttggaaggta gatttacgca     480

agtcttgtta ttccaagaac tttagggtag tcatttgctg gtgtggtatt ctttatcgag     540

ctctttcttt tacgtcttcc ctcatgaatt taggattaac cgaagagtct catgcttgtt     600

gcgcttacaa tgataatagt ctgagcaaat cgttgcatta caacccttac tgtgaaatct     660

ctgaggagcc atggcgtttg tttcatagtt gaagtcagca gtcgtacgac aacgtatgac     720

aagttcgtga aggcaaatca tttagttgtc aagtcctcga gaaactccgt actccggctt     780

acagcgtaat ggccaccgat aattactccg acaaacagga tgacacacag catacagaat     840

cctacagttg ccaaactcat ggcaggcaac aacaaacacg aaagtctcaa agcgtattat     900

gcctcattta taagctgtaa atgcatgtat gtatactaga cgtagcacaa gcctgatgcc     960

agatcctttc tctcaccttt gtcagatcaa cctgcacgtt tctcacccac tctctcgggc    1020

aatagtccta cagccagtaa caag                                           1044

<210>  73
<211>  1028
<212>  DNA
<213>  Trichoderma reesei

<400>  73
tgcaatgagt agtcttgcgc gatcaggggc gaagcctggg ccgtgacggg ggttggagcg      60

gtgactggga gttcgacaga ggcacgactg cggataggtc taggctggcg ggttggtgct     120

tttgcccttt gtctgccgcg acctcttggt gacgctgcat cgtgttggct gtagctgcac     180

gcccggcctc ttttctggca tctcgcacag actgtacgca gaacatgtgt aagtgacttt     240

ctattgtagg acttggatgg atgggagatg gctatagaca acaagagcga caacaaggat     300

tatagacagt tccattacct gggcgaccac catcgcactt caccttgctc gcccggcagg     360

actggcaggc gttggcaatc ttgagtcgct ccctgtacag cgacaagggc gcctgagcct     420

gcacctgagc ctgggcctga gtctgagcct gaagccgttg atcctcgtct tgacgggcgg     480

acatgacgct ttacgtgagc tgagtccagg gctgtcgtcg gcaagtgatt cggcagccgg 540

caggcacaga cgaaagataa gaagcggctc gttcaccaga tccgaaggcg gaatgcaaga 600

atgtccgatg atgtcgggga agagaaggcg atgcctcgtc tatctgggag gcgagggaag 660

aagggaaagc aactcccgcc tcggcacagc tactcattgg tcagagcgga cttcggagta 720

taaccacacg gctgacaaac ctgcatcgac cgtgatggcg ttcttttggt ccaatcatgg 780

ctgaatgcga cgttggttgt gtcggcccct gcagccaatg agaagctggt ccgatcctgg 840

ggacgaggga gaggcaagca tatcgtataa gtccaagctc aagcttggcg gcaggtaccg 900

gttgatgtgg tcgctgtata taagtaaaag aactcgattc cgcttcggag ttttctctgt 960

cacccttcat ggacgaagag ctattgagcc gcatctttgc ttcgttgtcc agcgtgaatt 1020

ctcctaca 1028

<210> 74
<211> 1000
<212> DNA
<213> Trichoderma reesei

<400> 74
aaatatcgga acttactata tgggaaagat tcattagctt tgtgcatcct tggagacatt 60

ggagtactat agacaactta ccagaggacc ccaaacagca gggccaattg caaagcccaa 120

cacaaagaca gagacaccgg tgatgaagag agtgctgctg atgtcaaact cggtcatgat 180

ctcttcagcc gacacggaat acgccgaaga cgtaaacgtg acagcaaaca cggaaaaggt 240

gacgatggat gtgaggaacc atttcctgaa ctggccccag ttcatggggt tgctcttgtc 300

gtcctgctgg aactcgacga taaagggatc ttccaccgtg ccggagccat tgtatgggga 360

ggtcgtaaca ttcttctcgg cgtcgacaac ctcgaggggc tcgacagcct cgaccggcgt 420

tatcgatcgg gagtcttggg aggcttcttt cgacattgta ggagaattca cgctggacaa 480

cgaagcaaag atgcggctca atagctcttc gtccatgaag ggtgacagag aaaactccga 540

agcggaatcg agttctttta cttatataca gcgaccacat caaccggtac ctgccgccaa 600

gcttgagctt ggacttatac gatatgcttg cctctccctc gtccccagga tcggaccagc 660

ttctcattgg ctgcaggggc cgacacaacc aacgtcgcat tcagccatga ttggaccaaa 720

agaacgccat cacggtcgat gcaggtttgt cagccgtgtg gttatactcc gaagtccgct 780

ctgaccaatg agtagctgtg ccgaggcggg agttgctttc ccttcttccc tcgcctccca 840

gatagacgag gcatcgcctt ctcttccccg acatcatcgg acattcttgc attccgcctt 900

cggatctggt gaacgagccg cttcttatct ttcgtctgtg cctgccggct gccgaatcac 960

ttgccgacga cagccctgga ctcagctcac gtaaagcgtc 1000

<210>  75
<211>  1000
<212>  DNA
<213>  Trichoderma reesei

<400>  75
ctcaatcagt tagtcgactt ggcaagcaaa tgcttggtgc aaccctttcg gctcccgctg      60

ggagaactaa cccccatgta cttggactat caaaagggac cggctgcagg aatctcttgc     120

cgcgcctcgt aagatcggac cagtggctga aacttccaat ggcagagaac gacctatcgg     180

aggggtcaaa tcgttttggc tcccttagta gacgaaggaa tgcataacaa tggccatggc     240

gtcaaacggt gagatcgggg gttgtcggac taaacatgcc tcgggcaccc tggtcggctc     300

ggtccgatgc ttgtagcaga tcccttcccg ctttatcctc cgtatccctt ccctacctat     360

ctcacagtgg aacatagtct aataagataa agcccgggac agagctcatg tgatactctt     420

gactcgcagt tccacgtggt ttatgcaact ctatttagac ctttataaag ctgcgtgctg     480

cccgaatcaa tattgaacca ggcagcactt gagactcgat atcacgccgc tgttgggggt     540

acagtagcat tgacaatacc tgggtaggct tcggttggca aagggtcaac cctggagctg     600

agcaatcctt cgatccttcg actagaaatc tgcccatagc tgaagacatc cggttcccct     660

attgggcaat ccgacagccg agactccttc aatcaagatg gcctcattgt tcgctagtcg     720

ctctttattg acagccgtaa gatggctcgt cattacgccg acggaacttt tcccgaagct     780

gacaccacat accagaatct acacagcaaa aaactccgta ctccgacaga acctcatcac     840

cccacgcctc attcgacctc tggttcaata gcagcttttc tttttcttta tcgatgtatg     900

atagtttata taaaaatgtg gaaaaaacac acttgcgtag cgataatgca aagaccagcg     960

ataaccttga aacgacgggc catttgacgt tctagacgcc                          1000

<210>  76
<211>  3000
<212>  DNA
<213>  Trichoderma reesei

<400>  76
ctcaatcagt tagtcgactt ggcaagcaaa tgcttggtgc aaccctttcg gctcccgctg      60

ggagaactaa cccccatgta cttggactat caaaagggac cggctgcagg aatctcttgc     120

cgcgcctcgt aagatcggac cagtggctga aagtacggct taccccaagc gaatgaagga     180

cgagcgacgg caagggccag gctcacagaa gcagcaaacg gtcggcgtac gtccgacaga     240

gcggctgtgg tgtagttttg tttcacgacg ccgctgatcc gccctgacga catacgtaca     300

tcaatagtgt ccaagaacag gagtgaaggt gcatccacag tgcatgtaga gtgcatacca     360

agttgactac agacatgttg gatggtggca cagctctagg ctgcttaggt agccggccga     420

tgtgtaaggt agcgagagtg aactcgcgcc tcataaagag catctccagg gggggggggg     480

gctaatgacg gatagctcag tttgcaacaa gtttgataca aggcgaaaga gaaggaccag 540
agcgtcgatt ggacagtagt gatatgcagg atcgtggact cgataagggc cacccaccca 600
tgtactcacg caagcaatat gcaagagtcg tcctattcag ccgggcttat tgctcggcac 660
acacacctgc aggatcggca cgcgaggctc ataaaggctc aacggcgtta agatggttga 720
aagacgcctg caactggtcc aagggtctga aggctccatg gccgcatgtg attatgcaag 780
ccagagccct ccgaagtctg ttgcccaggt acgctgtata ggaatagcag gtatctgtgc 840
gggtttggag cctcgtatgt tacgagtata ctccccacaa cataaggatt ctcacatctg 900
gaccttgtct atgcatggct tgagtggtcc cgctagcaga aaagcctgga agtaggatga 960
gactgcttgc taggctacaa ggggggaggc aattcggatg ttatcatata acaggtcttc 1020
cgctaccaaa ccatcaagcc atctgtgttg gcgagacttc catctgatcc tgatagacat 1080
cgccatacat cttgcctcat tatcctaccg tttcaacagg ctaggtgtgg tatcgtaagg 1140
tagcctgtca tccttccaat ggcagagaac gacctatcgg aggggtcaaa tcgttttggc 1200
tcccttagta gacgaaggaa tgcataacaa tggccatggc gtcaaacggt gagatcgggg 1260
gttgtcggac taaacatgcc tcgggcaccc tggtcggctc ggtccgatgc ttgtagcaga 1320
tcccttcccg ctttatcctc cgtatccctt ccctacctat ctcacagtgg aacatagtct 1380
aataagataa agcccgggac agagctcatg tgatactctt gactcgcagt tccacgtggt 1440
ttatgcaact ctatttagac ctttataaag ctgcgtgctg cccgaatcaa tattgaacca 1500
ggcagcactt gagactcgat atcacgccgc tgttgggggt acagtagcat tgacaatacc 1560
tgggtaggct tcggttggca aagggtcaac cctggagctg agcaatcctt cgatccttcg 1620
actagaaatc tgcccatagc tgaagacatc cggttcccct attgggcaat ccgacagccg 1680
agactccttc aatcaagatg gcctcattgt tcgctagtcg ctctttattg acagccgtaa 1740
gatggctcgt cattacgccg acggaacttt tcccgaagct gacaccacat accagaatct 1800
acacagcaaa aaactccgta ctccgacaga acctcatcac cccacgcctc attcgacctc 1860
tggttcaata gcagcttttc tttttcttta tcgatgtatg atagtttata taaaaatgtg 1920
gaaaaaacac acttgcgtag cgataatgca aagaccagcg ataaccttga aacgacgggc 1980
catttgacgt tctagacgcc ctcaatcagt tagtcgactt ggcaagcaaa tgcttggtgc 2040
aaccctttcg gctcccgctg ggagaactaa cccccatgta cttggactat caaaagggac 2100
cggctgcagg aatctcttgc cgcgcctcgt aagatcggac cagtggctga aacttccaat 2160
ggcagagaac gacctatcgg aggggtcaaa tcgttttggc tcccttagta gacgaaggaa 2220
tgcataacaa tggccatggc gtcaaacggt gagatcgggg gttgtcggac taaacatgcc 2280
tcgggcaccc tggtcggctc ggtccgatgc ttgtagcaga tcccttcccg ctttatcctc 2340
cgtatccctt ccctacctat ctcacagtgg aacatagtct aataagataa agcccgggac 2400

agagctcatg tgatactctt gactcgcagt tccacgtggt ttatgcaact ctatttagac 2460

ctttataaag ctgcgtgctg cccgaatcaa tattgaacca ggcagcactt gagactcgat 2520

atcacgccgc tgttgggggt acagtagcat tgacaatacc tgggtaggct tcggttggca 2580

aagggtcaac cctggagctg agcaatcctt cgatccttcg actagaaatc tgcccatagc 2640

tgaagacatc cggttcccct attgggcaat ccgacagccg agactccttc aatcaagatg 2700

gcctcattgt tcgctagtcg ctctttattg acagccgtaa gatggctcgt cattacgccg 2760

acggaacttt tcccgaagct gacaccacat accagaatct acacagcaaa aaactccgta 2820

ctccgacaga acctcatcac cccacgcctc attcgacctc tggttcaata gcagcttttc 2880

tttttttctta tcgatgtatg atagtttata taaaaatgtg gaaaaacac acttgcgtag 2940

cgataatgca aagaccagcg ataaccttga aacgacgggc catttgacgt tctagacgcc 3000